# EUROPEAN PATENT APPLICATION

(11) **EP 1 854 473 A1**
(43) Date of publication of application: **14.11.2007**
(21) Application number: 06728801.9
(22) Date of filing: 02.03.2006
(51) Int. Cl.: A61K 38/00, A61K 31/7088, A61K 39/395, A61K 48/00, A61P 35/00

(54) **PREVENTIVE/THERAPEUTIC AGENT FOR CANCER**

(30) Priority: 03.03.2005 JP 2005059277
(71) Applicant: Takeda Pharmaceutical Company Limited, Osaka-shi, Osaka 541-0045 (JP)
(72) Inventor: KATSUYAMA, Ryosuke, TAKEDA PHARMACEUTICAL COMP LTD, Osaka-shi, Osaka 5328686 (JP); KONDO, Shinichi, TAKEDA PHARMACEUTICAL COMPANY LTD, Osaka-shi, Osaka 5328686 (JP)
(74) Representative: Kalhammer, Georg
(86) International application number: PCT/JP2006/304532
(87) International publication number: WO 2006/093337

(57) **Abstract**

Identification of a novel target molecule for preventing/treating cancer and a preventive/remedy agent for cancer which targets the molecule, specifically, an agent for promoting the apoptosis in cancer cells and/or inhibiting the growth of cancer cells and a preventive/remedy agent for cancer, which comprise an antibody against a protein including the same or substantially the same amino acid sequence as the amino acid sequence represented by SEQ ID NO: 2, SEQ ID NO: 4, SEQ ID NO: 6, SEQ ID NO: 8, or SEQ ID NO: 10; an antisense polynucleotide of the protein (preferably, siRNA against mRNA of the protein); and a substance controlling the expression and/or activity of the protein, are provided by the present invention. A method of screening for a substance that promotes apoptosis in cancer cells and/or inhibits the growth of cancer cells and a preventive/remedy substance for cancer, in which the method comprises using a protein (preferably a cell producing the protein), an antibody against the protein, or a polynucleotide encoding the protein, is also provided by the present invention.

## Description

### Technical Field

The present invention relates to novel preventive/remedy agents for cancer, agents for promoting the apoptosis in cancer cells, and agents for inhibiting cancer cell growth. Specifically, the present invention relates to preventive/remedy agents for cancer comprising substances that control the expression and/or activities of novel target proteins, agents for promoting the apoptosis in cancer cells, and agents for inhibiting cancer cell growth. The present invention also relates to diagnostic agents for cancer comprising substances that can detect the expression of the target proteins or genes encoding the proteins. Further, the present invention relates to preventive/remedy for cancer using the target proteins or the nucleic acids encoding the proteins, and to a method of screening for the substance that promotes the apoptosis in cancer cells and/or inhibits cell growth.

### Background Art

Rapid advance in molecular biology study has enabled the elucidation of the molecular mechanism of cancer cell growth or malignant alteration. By clarifying the molecular target involved in the mechanism and the function thereof, there has initiated an investigation with a new concept of molecular target-based remedy which leads to a remedy by controlling the function, and developed new molecular target-based medicines such as Herceptin, Gleevec, Iressa, and the like, and thus accomplished certain achievement. However, since the medicines are consistently limited on the effectiveness, it is still an important task to search a novel drug development target molecule for treating cancer.

It is predicted that a cancer could be assessed for its pathological conditions by microarray profiling data for the gene. Actually in leukemia, it is reportedly possible to classify leukemia by gene expression profiles. By clarifying the gene expression profile of each cancerous tissue and accumulating its classification, it is considered possible to predict responsiveness to a particular cancer therapy or discover a novel drug development target protein for a particular cancer. Specifically, where enhanced expression of a certain protein is observed in a certain cancer, it becomes possible to induce an anti-tumor activity in patients newly diagnosed to be antigen positive, by means of (i) reducing its expression level, (ii) suppressing its function, (iii) eliciting immune response of host to the protein, etc. At the same time, patients diagnosed to be antigen negative can immediately switch over to another cancer therapy, assuming to eliminate any concern of imposing a superfluous burden on patients. As such, it is expected that the expression profile analysis would greatly contribute to molecular diagnosis of a cancer and development of molecular target-based drugs.

Desmocollin-3 gene is a gene isolated from human bladder cancer cell line HT-1376 cDNA library in 1994. The Desmocollin-3 gene comprises a Desmocollin-3a gene (Refseq Accession No. NM_001941) and a Desmocollin-3b gene (Refseq Accession No. NM_024423) which is a splicing variant thereof, and encodes proteins comprising 896 amino acids (Refseq Accession No. NP_001932) and 839 amino acids (Refseq Accession No. NP_077741), respectively (hereinafter, Desmocollin-3a gene and Desmocollin-3b gene may be generally referred to as Desmocollin-3). Further, a mouse gene (RefSeq Accession No. NM_007882) which is homologous to the Desmocollin-3a gene has been cloned, and encodes a protein comprising 895 amino acids (RefSeq Accession No. NP_031908) . The mouse gene has about 78% homology in a base sequence and about 78% homology in an amino acid sequence, to the Desmocollin-3a gene. The proteins encoded by Desmocollin-3a gene and the Desmocollin-3b gene belong to a Desmocollin family and amino acid sequences from the first amino acid to the 831 amino acid are the same but amino acid sequences from the 832 amino acid to C terminal which is an intracellular region are different from each other. The Desmocollin family is a molecular group belonging to a cadherin superfamily, which is comprised of three subfamily of Desmocollin-1, Desmocollin-2 and Desmocollin-3 (hereinafter, they may be generally referred to as Desmocollin). Desmocollin is one of main constituent molecules of desmosome, and serves as an intercellular adhesion molecule outside the cell. Desmocollin is a single transmembrane molecule and has four extracellular subdomains EC1-EC4 highly-conserved in its extracellular region. Functional difference between Desmocollin-3a and Desmocollin-3b is not apparent. However, Desmocollin-3a binds with many proteins belonging to Plakoglobin and Plakophilin families, but Desmocollin-3b binds with only Plakophilin-3, as evidenced at present.

Desmocollin forms a trans-interaction outside the cell. Counter molecules of the trans-interaction includes various bonds such as heterophilic interaction, homophilic interaction, etc., but generally it is considered that heterophilic interaction between Desmoglein which is also a main constituent molecule and Desmocollin is dominant. Furthermore, Desmocollin molecular binds to molecules belonging to armadillo family, Plakoglobin and Plakophilin, and then interacts with intermediate filaments via the binding within the cell.

Recently, it is reported that Desmocollin has not only a function as a molecule getting involved in an intercellular mechanical bond, but also a function of molecules for the morphogenesis of a tissue or determining positional relationship between individual cells within the tissue. For example, in mammary gland epithelial tissue comprising two layers of gland ductal epithelial cells and myoepithelial cells, Desmocollin-3 is expressed only in myoepithelial cells at a basal side (Desmocollin-2 is expressed in both layers, and Desmocollin-1 does not expressed in any layer), and it is disclosed that the inhibition of its function breaks the positional relationship of both cells (Nat. Cell Biol. (2001), 3: 823-830).

Regarding the relationship between Desmocollin-3 and a cancer, it is reported that the expression level of Desmocollin-3 in breast cancer cell line is decreased compared with in normal cell (Int J Oncol. (2001), 19(1): 169-174). Furthermore, Desmocollin-3 is reported as useful for the diagnosis of lung cancer (Publication No. WO 2002/86443) and useful for discovering breast cancer, ovary cancer or the like (Publication No. WO 2003/000012). However, these usefulness is only analogized from a gene expression analysis, and a particular case proving the assumption has not yet been clarified.

TM4SF13 gene (RefSeq Accession No. NM_014399) is a gene found from an EST library by applying a homology to sequence of ten genes (CD9, CD37, CD53, CD63, CD81, CD82, CD151, Co-029, NAG-2 and Talla-1) belonging to tetraspanin superfamily and amino acid sequence of proteins encoded by these genes an indicator, and encodes a protein comprising 204 amino acids (RefSeq Accession No. NP_055214). Furthermore, a mouse gene (RefSeq Accession No. NM_025359) which is homologous to TM4SF13 gene has been cloned from cDNA of mouse tissue origin, and encodes a protein comprising 204 amino acids (RefSeq Accession No. NP_079635) . The mouse gene has about 88% homology in a base sequence and about 96% homology in an amino acid sequence, to the TM4SF13 gene. TM4SF13 also known as NET-6 belongs to a tetraspanin superfamily which is tetra-transmembrane molecule family and is expected to be localized on a cell membrane.

TM4SF13 is reported as one of proteins useful for the detection of cancer (Publication No. WO 2000/53756), and one of genes useful for the detection of breast cancer or the like (Publication No. US 2002/081609). However, these usefulness is only analogized from a gene expression analysis, and a particular case proving the assumption has not yet been clarified. Since TM4SF13 level is decreased in an estrogen receptor-negative high grade breast cancer and the ectopic expression thereof has an inhibition effect against growth and infiltration of a cancer, TM4SF13 is also reported as a breast cancer inhibition gene, thus the position of this gene in cancers is still unknown.

TM4SF6 gene (RefSeq Accession No. NM_003270) is a gene isolated from human fetal lung cDNA library, and encodes a protein comprising 245 amino acids (RefSeq Accession No. NP_003261). Furthermore, a mouse gene (RefSeq Accession No. NM_019656) which is homologous to TM4SF6 gene has been cloned from cDNA of mouse tissue origin, and encodes a protein comprising 245 amino acids (RefSeq Accession No. NP_062630). The mouse gene has about 87% homology in a base sequence and about 93% homology in an amino acid sequence, to the TM4SF6 gene. TM4SF6 belongs to a tetraspanin superfamily which is tetra-transmembrane molecule family and is expected to be localized on a cell membrane.

The TM4SF6 gene is reported as one of the useful proteins for the diagnosis of cancers (Publication No. WO 2003/057160), and one of useful genes for the diagnosis of a colon cancer or the like (Publication No. WO 2001/22920). However, the usefulness thereof is only analogized from a gene expression analysis, a particular case proving the assumption has not yet been clarified.

LY-6K gene (RefSeq Accession No. NM_017527) is a gene isolated from head and neck mucosal cancer cell line UM-SCC-22A cDNA and encodes a protein comprising 223 amino acids (RefSeq Accession No. NP_059997). LY-6K is also known as HSJ001348 and is a molecule belonging to LY-6 family.

LY-6K gene is reported as a useful gene for the diagnosis of prostate cancer (Publication No. WO 2002/30268) and lung cancer (Publication No. WO 2002/86443). However, the usefulness thereof is only analogized from a gene expression analysis, a particular case proving the assumption has not yet been clarified.

### Disclosure of the Invention

As described above, a safe novel medicine which leads to a growth inhibition of cancer cells by targeting the molecule specifically expressed in a cancer cell is desired. In a cancer cell, many genes of which the expression significantly varies is known, but there are still many to be elucidated to find among them, which gene and expression product thereof can be the drug development target for treating cancer. Accordingly, the object of the invention is to identify a molecule which can be the novel target of a cancer molecular target-based treatment, to provide a preventive/remedy approach for cancer by controlling the expression or function of the target molecule, and to provide a method of screening for the substance having cancer preventive/remedy activity with the use of the target molecule.

In order to solve the foregoing problems, the present inventors made extensive investigations and as a result, discovered a gene showing a significantly increased expression in cancer tissues. Further, they found that siRNA for the gene inhibits a cancer cell growth, thus proved that the gene can be the target for a cancer preventive/remedy. In addition, the inventors have found the TM4SF13 influences on the relationship with an extracellular matrix by interacting with integrin, and accomplished the clarification of a part of working mechanism of the gene to cancers. Further investigation was carried out based on these findings, thereby the inventors completed the invention.

That is, the invention provides:
(1) An agent for promoting the apoptosis in cancer cells and/or inhibiting the growth of cancer cells, which comprises an antibody against a protein comprising the same or substantially the same amino acid sequence as the amino acid sequence represented by SEQ. ID NO: 2 or SEQ ID NO: 4, or against partial peptide thereof,
(2) The agent according to (1) above, which is for preventive/remedy for cancer,
(3) An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 2 or SEQ ID NO: 4,
(4) The agent according to (3) above, which is for preventive/remedy for cancer,
(5) An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 2 or SEQ ID NO: 4,
(6) The agent according to (5) above, which is for preventive/remedy for cancer,
(7) A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 2 or SEQ ID NO: 4,
(8) The method according to (7) above, which is for preventive/remedy for cancer,
(9) Use of a substance that inhibits the expression and/or activity of a protein comprising the same or substantially the same amino acid sequence as the amino acid sequence represented by SEQ ID NO: 2 or SEQ ID NO: 4, to manufacture an agent for promoting the apoptosis in cancer cells and/or inhibiting the growth of cancer cells,
(10) The use according to (9) above, wherein the agent for promoting the apoptosis in cancer cells and/or inhibiting the growth of cancer cells is for preventive/remedy for cancer,
(11) A method of screening for a substance that promotes the apoptosis in cancer cells and/or inhibits the growth of cancer cells, the method includes using a protein comprising the same or substantially the same amino acid sequence as the amino acid sequence represented by SEQ ID NO: 2 or SEQ ID NO: 4, or partial peptide thereof,
(12) The method according to (11) above, wherein the protein comprising the same or substantially the same amino acid sequence as the amino acid sequence represented by SEQ ID NO: 2 or SEQ ID NO: 4, or partial peptide thereof, is provided in the form of a cell capable of producing the protein or the partial peptide,
(13) The method according to (12) above, comprises further using any one selected from a group consisting of antibodies against a protein comprising the same or substantially the same amino acid sequence as the amino acid sequence represented by SEQ ID NO: 2 or SEQ ID NO: 4, or against partial peptide thereof, and polynucleotide encoding the protein or polynucleotide including a part of the base sequence,
(14) The method according to (11) above, which is for the screening for a preventive/remedy substance for cancer,
(15) An agent for promoting the apoptosis in cancer cells and/or inhibiting the growth of cancer cells, which comprises a substance that controls the expression of a protein comprising the same or substantially the same amino acid sequence as the amino acid sequence represented by SEQ ID NO: 6 and/or the interaction between the protein and integrin,
(16) The agent according to (15) above, wherein the substance that controls the interaction between the protein comprising the same or substantially the same amino acid sequence as the amino acid sequence represented by SEQ ID NO: 6 and integrin is a neutralizing antibody against the protein,
(17) The agent according to (15) above, wherein the substance that controls the expression of the protein comprising the same or substantially the same amino acid sequence as the amino acid sequence represented by SEQ ID NO: 6 is an antisense polynucleotide including a base sequence or a part of the base sequence, the base sequence being complementary or substantially complementary to a base sequence of polynucleotide encoding the protein,
(18) The agent according to (15) above, wherein the substance that controls the interaction between the protein comprising the same or substantially the same amino acid sequence as the amino acid sequence represented by SEQ ID NO: 6 and integrin is a non-neutralizing antibody against the protein,
(19) The agent according to (18) above, wherein the antibody is an agonist antibody,
(20) The agent according to (15) above, wherein the substance that controls the expression of the protein comprising the same or substantially the same amino acid sequence as the amino acid sequence represented by SEQ ID NO: 6 is the protein or partial peptide thereof, or a polynucleotide that encodes the protein or the partial peptide,
(21) The agent according to (15) above, which is for preventive/remedy for cancer,
(22) A method of promoting the apoptosis in cancer cells and/or inhibiting the growth of cancer cells, the method comprises controlling the expression of a protein comprising the same or substantially the same amino acid sequence as the amino acid sequence represented by SEQ ID NO: 6 and/or the interaction between the protein and integrin,
(23) The method according to (22) above, which is for preventive/remedy for cancer,
(24) Use of a substance that controls the expression of a protein comprising the same or substantially the same amino acid sequence as the amino acid sequence represented by SEQ ID NO: 6 and/or the interaction between the protein and integrin, to manufacture an agent for promoting the apoptosis in cancer cells and/or inhibiting the growth of cancer cells,
(25) The use according to (24) above, wherein the agent for promoting the apoptosis in cancer cells and/or inhibiting the growth of cancer cells is for preventive/remedy for cancer,
(26) A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 6, or partial peptide thereof,
(27) A method according to (26) above, wherein the protein comprising the same or substantially the same amino acid sequence as the amino acid sequence represented by SEQ ID NO: 6, or partial peptide thereof, is provided in the form of a cell capable of producing the protein or the partial peptide,
(28) The method according to (27) above, comprises further using any one selected from a group consisting of antibodies against a protein comprising the same or substantially the same amino acid sequence as the amino acid sequence represented by SEQ ID NO: 6, or against partial peptide thereof, and polynucleotide encoding the protein or polynucleotide including a part of the base sequence thereof,
(29) The method according to (26) above, which is for the screening for a preventive/remedy substance for cancer,
(30) A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 6 or partial peptide thereof, and integrin,
(31) The method according to (30) above, wherein the protein comprising the same or substantially the same amino acid sequence as the amino acid sequence represented by SEQ ID NO: 6 or partial peptide thereof, and/or integrin is provided in the form of a cell capable of producing the protein or the partial peptide and/or integrin,
(32) The method according to (30) above, which is for the screening for a preventive/remedy substance for cancer,
(33) An agent for promoting the apoptosis in cancer cells and/or inhibiting the growth of cancer cells, which comprises an antibody against a protein comprising the same or substantially the same amino acid sequence as the amino acid sequence represented by SEQ ID NO: 8 or SEQ ID NO: 10, or against partial peptide thereof,
(34) The agent according to (33) above, which is for preventive/remedy for cancer,
(35) An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 8 or SEQ ID NO: 10,
(36) The agent according to (35) above, which is for preventive/remedy for cancer,
(37) An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 8 or SEQ ID NO: 10,
(38) The agent according to (37) above, which is for preventive/remedy for cancer,
(39) A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 8 or SEQ ID NO: 10,
(40) The method according to (39) above, which is for preventive/remedy for cancer,
(41) Use of a substance that inhibits the expression and/or activity of a protein comprising the same or substantially the same amino acid sequence as the amino acid sequence represented by SEQ ID NO: 8 or SEQ ID NO: 10, to manufacture an agent for promoting the apoptosis in cancer cells and/or inhibiting the growth of cancer cells,
(42) The use according to (41) above, wherein the agent for promoting the apoptosis in cancer cells and/or inhibiting the growth of cancer cells is for a preventive/remedy for cancer,
(43) A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 8 or SEQ ID NO: 10, or partial peptide thereof,
(44) The method according to (43) above, wherein the protein comprising the same or substantially the same amino acid sequence as the amino acid sequence represented by SEQ ID NO: 8 or SEQ ID NO: 10, or partial peptide thereof, is provided in the form of a cell capable of producing the protein or the partial peptide,
(45) The method according to (44) above, comprises further using any one selected from a group consisting of antibodies against a protein comprising the same or substantially the same amino acid sequence as the amino acid sequence represented by SEQ ID NO: 8 or SEQ ID NO: 10, or against partial peptide thereof, and polynucleotide encoding the protein or polynucleotide including a part of the base sequence,
(46) The method according to (43) above, which is for the screening for a preventive/remedy substance for cancer, and the like.

The protein comprising the same or substantially the same amino acid sequence as the amino acid sequence represented by SEQ ID NO: 2, SEQ ID NO: 4, SEQ ID NO: 6, SEQ ID NO: 8 or SEQ ID NO: 10, and the polynucleotide encoding the protein are specifically expressed in cancer tissue, thus can be a diagnosis marker for cancer. Further, an antibody against the protein, an antisense polynucleotide to the polynucleotide, and a substance that controls the expression and/or activity of the protein, can be safely used, for example, as preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably as preventive/remedy agents for breast cancer, lung cancer, colon cancer, prostate cancer, ovary cancer, pancreatic cancer, etc.), agents for promoting apoptosis in cancer cells, agents for inhibiting cancer cell growth, or the like. Further, the above-mentioned protein, polynucleotide, antibody and the like are useful for screening for a preventive/remedy substance for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably for a preventive/remedy substance for breast cancer, lung cancer, colon cancer, prostate cancer, ovary cancer, pancreatic cancer, etc.), a substance promoting the apoptosis in cancer cells, a substance inhibiting the growth of cancer cells, or the like.

### Brief Description of the Drawings

Fig. 1 shows a gene expression profile of Democollin-3 prepared by using various cancer tissue origin mRNAs and neighboring normal tissue origin mRNAs.
FIG. 2 shows a result of administering siRNA for a Democollin-3 gene. Fig 2A shows that the cell growth of human lung cancer cell line is inhibited by administering the siRNA; Fig 2B shows that the level of mRNA expression of the Democollin-3 gene is reduced by administering the siRNA; and Fig. 2C shows that the expression level of a Democollin-3 protein is reduced by administering the siRNA.
Fig. 3 shows a gene expression profile of TM4SF13 prepared by using various cancer tissue origin mRNAs and neighboring normal tissue origin mRNAs.
Fig. 4 shows a result of administering siRNA for a TM4SF13 gene. Fig 4A shows that the cell growth of human breast cancer cell line is inhibited by administering the siRNA; and Fig 4B shows that the level of mRNA expression of the TM4SF13 gene is reduced by administering the siRNA.
Fig. 5 shows that a TM4SF13 protein is localized on a cytoplasmic membrane. From left, DAPI staining, immunofluorescence staining and a chromatic figure combining them are shown, respectively.

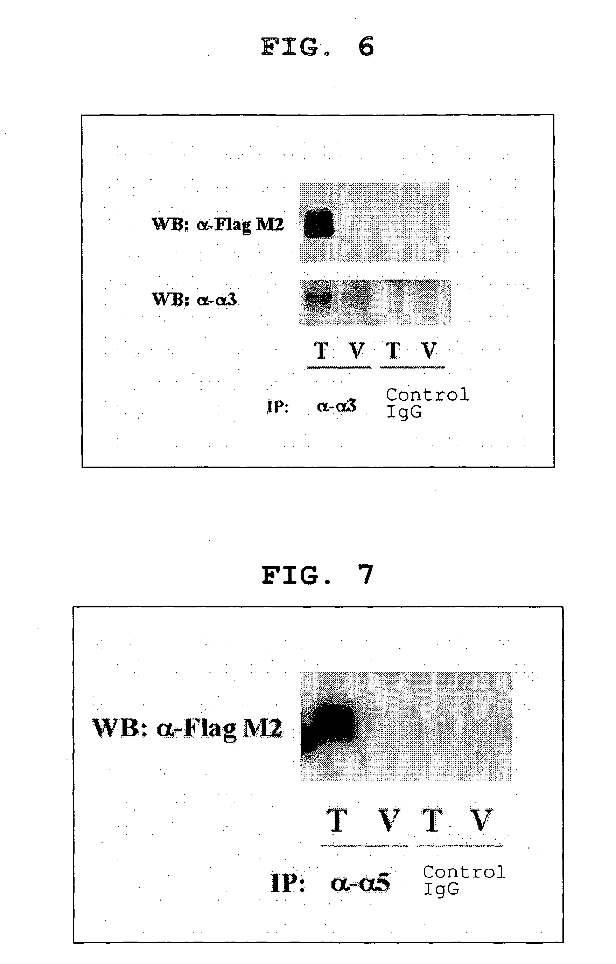

FIG. 6 shows that the TM4SF13 protein interacts with an integrin-α3 (T: TM4SF13-3xFLAG, V: empty vector (control)).
FIG. 7 shows that the TM4SF13 protein interacts with an integrin-α5 (T: TM4SF13-3xFLAG, V: empty vector (control)).
Fig. 8 shows the morphology change of a cell by the expression of a TM4SF13 protein. The upper panels show a result of culturing the cell on a plate coated with fibronectin, and the lower panels show a result of culturing the cell on a plate coated with laminin. From left, a TM4SF13 expressing cell, a LacZ expressing cell and a nontransfectant (NT) are shown.

### Best Mode for Carrying Out the Invention

A protein used in the present invention is a protein comprising the same or substantially the same amino acid sequence as the amino acid sequence represented by SEQ ID NO: 2, SEQ ID NO: 4, SEQ ID NO: 6, SEQ ID NO: 8 or SEQ ID NO: 10 (hereinafter these proteins are sometimes referred to as the protein of the present invention). The protein of the present invention may be any protein isolated and purified from cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells, etc.] of human and other warm-blooded animals (e.g., guinea pig, rat, mouse, chicken, rabbit, swine, sheep, bovine, simian, etc.); or any tissue where such cells are present [for example, brain or each part of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle (e.g., smooth muscle, skeletal muscle), lung, gastrointestinal tract (e.g., large intestine and small intestine), blood vessel, heart, thymus, spleen, submandibular gland, peripheral blood, prostate, testis, ovary, placenta, uterus, bone, joint, adipose tissue (e.g., white adipose tissue, brown adipose tissue), etc.] . The protein may also be a protein biochemically synthesized by a chemical synthesis or cell-free translation system, or a recombinant protein produced from a transfectant in which the nucleic acid comprising a base sequence encoding the above-mentioned amino acid sequence is transfected.

The amino acid sequence whish is substantially the same amino acid sequence as that represented by SEQ ID NO: 2, SEQ ID NO: 4, SEQ ID NO: 6, SEQ ID NO: 8 or SEQ ID NO: 10, includes amino acid sequences having about 50% or more homology, preferably about 60% homology or more, more preferably about 70% or more homology, even more preferably about 80% or more homology, particularly preferably about 90% or more homology and most preferably about 95% or more homology, to the amino acid sequence shown by SEQ ID NO: 2, SEQ ID NO: 4, SEQ ID NO: 6, SEQ ID NO: 8 or SEQ ID NO: 10, and the like. Herein, the 'Homology' means a ratio (%) of the same amino acid and similar amino acid residue to the total overlapped amino acid residue, in the best alignment when two amino acid sequences are aligned with the use of a mathematical algorithm commonly known in the technical field (preferably, the algorithm considers introduction of gaps on one or both side of the sequence for the best alignment). The term 'similar amino acid' refers to an amino acid similar in its physiochemical properties, and the examples include amino acids classified in a same group such as aromatic amino acid (Phe, Trp, Tyr), aliphatic amino acid (Ala, Leu, Ile, Val), polar amino acid (Gln, Asn), basic amino acid (Lys, Arg, His), acidic amino acid (Glu, Asp), amino acid including a hydroxyl group (Ser, Thr), amino acid having a short side chain(Gly, Ala, Ser, Thr, Met), and the like. A substitution by such similar amino acid is expected to give no change in the phenotype of protein (thus is a conservative amino acid substitution). A specific example of the conservative amino acid substitution is well-known in the technical field, and is disclosed in various documents (for example, refer Bowie et al, Science, 247: 1306-1310 (1990)).

Homology of the amino acid sequences in the present specification can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool). Other algorithm for determining homology of the amino acid sequence is exemplified by an algorithm disclosed in Karlin et al, Proc. Natl. Acad. Sci. USA, 90: 5873-5877 (1993) [this algorithm is incorporated in NBLAST and XBLAST program (version 2. 0) (Altschul et al, Nucleic Acids Res., 25:3389-3402(1997))]; an algorithm disclosed in Needleman et al, J. Mol. Biol., 48: 444-453 (1970) [This algorithm is incorporated in a GAP program in a GCG software package]; an algorithm disclosed in Myers and Miller, CABIOS, 4: 11-17 (1988) [This algorithm is incorporated in ALIGN program (version 2.0) which is a part of a CGC sequence alignment software package] ; an algorithm disclosed in Pearson et al. Proc. Natl. Acad. Sci. USA, 85: 2444-2448 (1998) [This algorithm is incorporated in an FASTA program in a GCG software package], etc., and these may be also preferably used.

More preferably, the amino acid sequence which is substantially the same amino acid sequence as that represented by SEQ ID NO: 2, SEQ ID NO: 4, SEQ ID NO: 6, SEQ ID NO: 8 or SEQ ID NO: 10, includes amino acid sequences having about 50% or more identity, preferably about 60% or more identity, more preferably about 70% or more identity, even more preferably about 80% or more identity, particularly preferably about 90% or more identity and most preferably about 95% or more identity, to the amino acid sequence shown by SEQ ID NO: 2, SEQ ID NO: 4, SEQ ID NO: 6, SEQ ID NO: 8 or SEQ ID NO: 10.

The protein used in the present invention is a protein comprising substantially the same amino acid sequence as the amino acid sequence represented by SEQ ID NO: 2, SEQ ID NO: 4, SEQ ID NO: 6, SEQ ID NO: 8 or SEQ ID NO: 10, and having an activity substantially equivalent to the protein comprising the amino acid sequence represented by SEQ ID NO: 2, SEQ ID NO: 4, SEQ ID NO: 6, SEQ ID NO: 8 or SEQ ID NO: 10.

As the substantially equivalent activity described above, there are, for example, the ligand binding activity, and signal transduction, and the like. The substantially equivalent is used to mean that the nature of the activities is equivalent in terms of quality (e.g., physiologically or pharmacologically). Thus, the activities of the protein of the present invention are preferably equivalent, but differences in quantitative factors such as a level of these activities (e.g., about 0.01 to 100 times, preferably about 0.1 to 10 times, more preferably 0.5 to 2 times), a molecular weight of the protein, and the like may be present and allowable.

The activity such as the ligand binding activity and signal transduction can be assayed by publicly known methods, for example, it can be assayed by the method to be described later, comprises screening for a compound that inhibits the activity of the protein used in the invention, or salts thereof.

Examples of the protein used in the present invention include so-called muteins such as proteins having (i) the amino acid sequence represented by SEQ ID NO: 2, SEQ ID NO: 4, SEQ ID NO: 6, SEQ ID NO: 8 or SEQ ID NO: 10, of which 1 or 2 or more (e.g. , about 1 to about 50, preferably about 1 to about 30, more preferably about 1 to about 10 and much more preferably several (1 to 5, 4, 3 or 2)) amino acids are deleted; (ii) the amino acid sequence represented by SEQ ID NO: 2, SEQ ID NO: 4, SEQ ID NO: 6, SEQ ID NO: 8 or SEQ ID NO: 10, to which 1 or 2 or more (e.g., about 1 to about 50, preferably about 1 to about 30, more preferably about 1 to about 10 and much more preferably several (1 to 5, 4, 3 or 2)) amino acids are added; (iii) the amino acid sequence represented by SEQ ID NO: 2, SEQ ID NO: 4, SEQ ID NO: 6, SEQ ID NO: 8 or SEQ ID NO: 10, in which 1 or 2 or more (e.g., about 1 to about 50, preferably about 1 to about 30, more preferably about 1 to about 10 and much more preferably several (1 to 5, 4, 3 or 2)) amino acids are inserted; (iv) the amino acid sequence represented by SEQ ID NO: 2, SEQ ID NO: 4, SEQ ID NO: 6, SEQ ID NO: 8 or SEQ ID NO: 10, in which 1 or 2 or more (e.g. , about 1 to about 50, preferably about 1 to about 30, more preferably about 1 to about 10 and much more preferably several (1 to 5, 4, 3 or 2)) amino acids are substituted by other amino acids; or (v) a combination of these amino acid sequences; and the like.

Where the amino acid sequence is inserted, deleted or substituted as described above, the position of its insertion, deletion, or substitution is not particularly limited.

Preferable examples of the protein used in the present invention include human Desmocollin-3a comprising the amino acid sequence represented by SEQ ID NO: 2 (Refseq Accession No. NP 001932) or the homolog thereof (for example, mouse homolog registered as RefSeq Accession No. NP_031908 in GenBank) in other mammals, human Desmocollin-3b comprising the amino acid sequence represented by SEQ ID NO: 4 (Refseq Accession No. NP_077741) or the homolog thereof in other mammals, human TM4SF13 comprising the amino acid sequence represented by SEQ ID NO: 6 (RefSeq Accession No. NP_055214) or the homolog thereof (for example, mouse homolog registered as RefSeq Accession No. NP_079635 in GenBank) in other mammals, human TM4SF6 comprising the amino acid sequence represented by SEQ ID NO: 8 (RefSeq Accession No. NP_003261) or the homolog thereof in other mammals (for example, mouse homolog registered as RefSeq Accession No. NP_062630 in GenBank), and human LY-6K comprising the amino acid sequence represented by SEQ ID NO: 10 (RefSeq Accession No. NP_059997) or the homolog thereof in other mammals.

In the present specification, the proteins and peptides are represented in accordance with the conventional way of describing peptides, that is, the N-terminus (amino terminus) at the left hand and the C-terminus (carboxyl terminus) at the right hand. In the protein used in the present invention which includes the protein comprising the amino acid sequence represented by SEQ ID NO: 2, SEQ ID NO: 4, SEQ ID NO: 6, SEQ ID NO: 8 or SEQ ID NO: 10, the C-terminus may be in any form of a carboxyl group (-COOH), carboxylate (-COO⁻), an amide (-CONH₂) or an ester (-COOR) .

Herein, examples of R in the ester include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc. or α-naphthyl-C₁₋₂ alkyl group such as α-naphthylmethyl, etc.; pivaloyloxymethyl group and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester in this case may be the C-terminal esters described above, etc.

Furthermore, examples of the protein used in the present invention also include those wherein the amino group at the N-terminal amino acid residues (e.g., methionine residue) is protected with a protecting group (e.g., a C₁₋₆ acyl group such as a C₁₋₆ alkanoyl, e.g., formyl group, acetyl group, etc.); those wherein the N-terminal region is cleaved in vivo and the glutamine residue thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as so-called glycoproteins having sugar chains; etc.

The partial peptide of the protein used in the present invention may be any peptide as long as it is a peptide having a partial amino acid sequence of the protein used in the present invention described above and has the activity substantially equivalent to that of the protein used in the present invention described above. Herein, the 'activity substantially equivalent' means the same as mentioned above. The 'activity substantially equivalent' can also be assayed in a same manner as in the case of protein used in the present invention as above mentioned.

For example, there are used peptides containing, e.g., at least 20 or more, preferably 50 or more, more preferably 70 or more, much more preferably 100 or more and most preferably 150 or more amino acids, in the constituent amino acid sequence of the protein used in the present invention, etc.

The partial peptide used in the present invention may be peptides containing the amino acid sequence, of which 1 or 2 or more (preferably about 1 to about 20, more preferably about 1 to about 10 and much more preferably several (1 to 5, 4, 3 or 2)) amino acids may be deleted; to which 1 or 2 or more (preferably about 1 to about 20, more preferably about 1 to about 10 and much more preferably several (1 to 5, 4, 3 or 2)) amino acids may be added; in which 1 or 2 or more (preferably about 1 to about 20, more preferably about 1 to about 10 and much more preferably several (1 to 5, 4, 3 or 2)) amino acids may be inserted; or in which 1 or 2 or more (preferably about 1 to about 20, more preferably about 1 to about 10, and mush more preferably several (1 to 5, 4, 3 or 2)) amino acids may be substituted by other amino acids.

In the partial peptide used in the present invention, the C-terminus may be in any form of a carboxyl group (-COOH), a carboxylate (-COO⁻), an amide (-CONH₂) or an ester (-COOR). Herein, as the 'R' in ester, same ones as in the protein used in the present invention can be exemplified. Where the partial peptide of the present invention contains a carboxyl group (or a carboxylate) at a position other than the C-terminus, the carboxyl group may be amidated or esterified and such an amide or ester is also included within the partial peptide of the present invention. Examples of the ester in this case may be the same as C-terminal esters described above, etc.

Furthermore, the partial peptide used in the present invention also includes those wherein the amino group at the N-terminal amino acid residues (e.g., methionine residue) is protected with a protecting group; those wherein the N-terminal region is cleaved in vivo and the glutamine residue thus formed is pyroglutaminated; those wherein a substituent on the side chain of an amino acid in the molecule is protected with a suitable protecting group, or conjugated peptides such as so-called glycopeptides having sugar chains; etc., as in the protein used in the present invention described above.

The partial peptide used in the present invention may also be used as an antigen for producing antibodies.

The protein, or its partial peptide used in the present invention, may be a free form, or salt form (it is true throughout the present specification unless otherwise specified). As the salts, salts with physiologically acceptable acids (e.g., inorganic acids, organic acids, etc.) or bases (e.g., alkali metal salts, etc.), preferably physiologically acceptable acid addition salts can be used. Examples of such salts include salts with inorganic acids (e.g., hydrochloric acid, phosphoric acid, hydrobromic acid, sulfuric acid), salts with organic acids (e.g., acetic acid; formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein used in the present invention may be manufactured by publicly known methods used to purify a protein from human or warm-blooded animal cells or tissues described above. Specifically, mammalian tissues or cells are homogenized in the presence of surfactant, and then crude tissue extract fraction obtained is subjected to chromatography techniques such as reverse phase chromatography, ion exchange chromatography, affinity chromatography and the like, thereby the proteins used in the present invention can be prepared.

The protein used in the present invention or its partial peptide can be manufactured by publicly known methods for peptide synthesis.

For the methods for peptide synthesis, for example, either solid phase synthesis or liquid phase synthesis may be used. That is, the partial peptide or amino acids that can constitute the protein or its partial peptide used in the present invention are condensed with the remaining part. Where the product contains protecting groups, these protecting groups are removed to give the desired protein (peptide). Publicly known methods for condensation and elimination of the protecting groups are, for example, described in (i) to (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

The protein (peptide) obtained in the above manner may be purified and isolated by a known purification methods such as solvent extraction, distillation, column chromatography, liquid chromatography and recrystallization.

When the protein (peptide) obtained by the above methods is in a free form, it can be converted into an appropriate salt by a publicly known method or its modification; conversely when the protein (peptide) is obtained in a salt form, it can be converted into a free form or other different salt form by a publicly known method or its modification.

To synthesize the protein or its partial peptide used in the present invention, or amides thereof, commercially available resins that are used for protein synthesis may be usually used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents available for protein synthesis may be used, and carbodiimides are particularly preferable. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) may be added directly to the resin, or the protected amino acids may be previously activated in the form of symmetric acid anhydrides, HOBt esters or HOOBt esters, followed by adding the thus activated protected amino acids to the resin.

Solvents for use to activate the protected amino acids or condense them with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; or appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Protection of the functional groups that should not be involved in the reaction of the starting materials, protecting groups, elimination of the protecting groups, activation of the functional groups involved in the reaction, and the like may be appropriately chosen from publicly known groups and publicly known means.

Examples of the protecting groups used to protect the starting amino groups include Z, Boc, t-pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl, e.g., methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethyl-benzenesulfonyl, DNP, benzyloxymethyl, Bum, Boc, Trt, Fmoc, etc.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1, 2-ethanedithiol, 1,4-butanedithiol, etc. as well as by a treatment with an alkali such as a dilute sodium hydroxide solution, dilute ammonia, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As example of the activated amino groups in the starting material, the corresponding phosphoric amides are employed.

In another method for obtaining the amides of the desired protein or partial peptide, for example, the α-carboxyl group of the carboxy terminal amino acid is first protected by amidation; the peptide (protein) chain is then extended from the amino group side to a desired length. Subsequently, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide of the protein used in the present invention can be manufactured by cleaving the protein used in the present invention with an appropriate peptidase.

Further, the protein used in the present invention or partial peptides thereof can also be manufactured by culturing a transformant containing polynucleotide encoding the same and then by separating and purifying the protein or partial peptide from the obtained culture. The polynucleotide encoding the protein used in the present invention or its partial peptide may be DNA or RNA, or DNA/RNA chimera, and preferably is DNA. In addition, the polynucleotide may be a double-strand, or single-strand. The double-strand may include a double-stranded DNA, a double-stranded RNA, and DNA: RNA hybrid. The single-strand may include a sense strand (that is, coding strand) and an antisense strand (that is, non-coding strand).

The polynucleotide encoding the protein used in the present invention or its partial peptide can be exemplified by genomic DNA, genomic DNA library, cDNA derived from any mammalian (e.g., human, bovine, simian, horse, swine, sheep, goat, canine, feline, guinea pig, rat, mouse, rabbit, hamster, etc.) cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g. , macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells, etc.]; or any tissues where such cells are present [for example, brain or each part of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, adipose tissue (e.g., white adipose tissue, brown adipose tissue), skeletal muscle, etc.], synthetic DNA, etc. The genomic DNA and cDNA encoding the protein used in the present invention or its partial peptide can be directly amplified by Polymerase Chain Reaction (hereinafter, abbreviate to "PCR") and Reverse Transcriptase-PCR (hereinafter, abbreviate to "RT-PCR") with the use of each genomic DNA fraction, and total RNA or mRNA fraction prepared from the above-described cells or tissues as a template. Further, the genomic DNA and cDNA encoding the protein used in the present invention or its partial peptide can be respectively cloned from genomic DNA library and cDNA library which are prepared by inserting the fragment of genomic DNA, and total RNA or mRNA prepared from the above-described cells or tissues into an appropriate vector, in accordance with a colony or plaque hybridization assay or PCR method. The vector used for the library may be any of bacteriophage, plasmid, cosmid, phagemid and the like.

Examples of the DNA encoding the protein used in the present invention may be any of DNA comprising a base sequence hybridizable to DNA comprising the base sequence represented by SEQ ID NO: 1, SEQ ID NO: 3, SEQ ID NO: 5, SEQ ID NO: 7 or SEQ ID NO: 9 under high stringent conditions and encoding a protein which has the activity substantially equivalent to the protein comprising the amino acid sequence represented by SEQ ID NO: 2, SEQ ID NO: 4, SEQ ID NO: 6, SEQ ID NO: 8 or SEQ ID NO: 10.

As the DNA that is hybridizable to the base sequence represented by SEQ ID NO: 1, SEQ ID NO: 3, SEQ ID NO: 5, SEQ ID NO: 7 or SEQ ID NO: 9 under high stringent conditions, there are employed, for example, DNAs comprising base sequences having about 50% or more homology, preferably about 60% or more homology, more preferably about 70% or more homology, even more preferably about 80% or more homology, particularly preferably about 90% or more homology, and most preferably about 95% or more homology, to the base sequence represented by SEQ ID NO: 1, SEQ ID NO: 3, SEQ ID NO: 5, SEQ ID NO: 7 or SEQ ID NO: 9.

Homology of the base sequences in the present specification, for example, can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool) . As the other algorithm for determining homology of the base sequence, same ones as the above-described homology scoring algorithms for the amino acid sequence are preferably exemplified.

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out more preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium salt concentration at about 19 mM at a temperature of about 65°C are most preferred. Those skilled in the art can simply regulate the condition to a desired stringency by appropriately changing a concentration of hybridization solution, temperature of hybridization reaction, probe concentration, length of probe, number of mismatch, time for hybridization reaction, salt concentration of washing solution, temperature for washing, etc.

Preferable examples of the DNA encoding the protein used in the present invention include DNA comprising the base sequence represented by SEQ ID NO: 1 (Refseq Accession No. NM_001941) or the homolog thereof (for example, mouse homolog registered as RefSeq Accession No. NM_007882 in GenBank) in other mammals, DNA comprising the base sequence represented by SEQ ID NO: 3 (Refseq Accession No. NM_024423) or the homolog thereof in other mammals, DNA comprising the based sequence represented by SEQ ID NO: 5 (RefSeq Accession No. NM_014399) or the homolog thereof (for example, mouse homolog registered as RefSeq Accession No. NM_025359 in GenBank) in other mammals, DNA comprising the base sequence represented by SEQ ID NO: 7 (RefSeq Accession No. NM_003270) or the homolog thereof (for example, mouse homolog registered as RefSeq Accession No. NM_019656 in GenBank) in other mammals, DNA comprising the base sequence represented by SEQ ID NO: 9 (RefSeq Accession No. NM_017527) or the homolog thereof in other mammals.

The polynucleotide (e.g., DNA) encoding the partial peptide of the protein used in the present invention may be any polynucleotide so long as it contains the base sequence encoding the partial peptide of the protein used in the present invention described above. The polynucleotide may also be any of genomic DNA, genomic DNA library, cDNA derived from the cells and tissues described above, cDNA library derived from the cells and tissues described above, and synthetic DNA.

As the DNA encoding the partial peptide of the protein used in the present invention, there is employed, for example, DNA containing a partial base sequence of the base sequence represented by SEQ ID NO: 1, SEQ ID NO: 3, SEQ ID NO: 5, SEQ ID NO: 7 or SEQ ID NO: 9; or DNA containing a base sequence hybridizable to polynucleotide which comprises the base sequence represented by SEQ ID NO: 1, SEQ ID NO: 3, SEQ ID NO: 5, SEQ ID NO: 7 or SEQ ID NO: 9 under high stringent conditions, and also encoding a peptide which has the activity substantially equivalent to the protein used in the present invention. The DNA hybridizable to the base sequence represented by SEQ ID NO: 1, SEQ ID NO: 3, SEQ ID NO: 5, SEQ ID NO: 7 or SEQ ID NO: 9 represent the same meaning as described above. Further, same hybridization method and high stringent conditions as described above can be used.

For cloning of DNAs that encode the protein used in the present invention and its partial peptide (hereinafter sometimes merely referred to as the protein of the present invention in the description of cloning of DNAs encoding the same and their expression), the DNA can be either amplified by PCR using synthetic DNA primers containing a part of the base sequence encoding the protein of the present invention, or the DNA inserted into an appropriate vector can be selected by hybridization with those labeled with DNA fragment or synthetic DNA that encodes a part or entire region of the protein of the present invention. The hybridization can be carried out, for example, according to the method described in Molecular Cloning, 2nd ed. (J. Sambrook et al., Cold Spring Harbor Lab. Press, 1989). Where the hybridization is carried out using commercially available library, the procedures may be conducted in accordance with the protocol described in the attached instructions.

Conversion of the base sequence of DNA can be effected by publicly known methods such as the ODA-LA PCR method, the Gapped duplex method, the Kunkel method, etc., or modification thereof, using PCR, a publicly known kit available as Mutan^{™}-super Express Km (Takara Bio) or Mutan^{™}-K (Takara Bio) , etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by excising the desired DNA fragment from the DNA encoding the protein of the present invention, and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pAl-11, pXT1, pRc/CMV, pRc/RSV, pcDNA I/Neo, etc.

The promoter used in the present invention may be any promoter if it matches well with a host to be used for gene expression. For example, when animal cells are used as the host, examples of the promoter are SRα promoter, SV40 promoter, LTR promoter, CMV promoter, HSV-TK promoter, etc..

Among them, it is preferable to use CMV (cytomegalovirus) promoter, SRα promoter, etc. When bacteria of the genus Escherichia is used as a host, preferred examples of the promoter are trp promoter, lac promoter, recA promoter, λP_{L} promoter, lpp promoter, T7 promoter, etc. When bacteria of the genus Bacillus is used as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter are polyhedrin promoter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, the objective gene can also be selected on a thymidine free medium.

If necessary, a signal sequence that matches with a host is added to the N-terminus of the protein of the present invention. The signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured. Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cerevisiae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five^{™} cell derived from egg of Trichoplusia ni, cells derived from Mamestra brassicae, cells derived from Estigmena acrea, etc.; and for the virus BmNPV, Bombyx mori N cell (BmN cell), etc. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include simian cell COS-1, COS-3, COS-7, Vero, Chinese hamster ovary cell (hereinafter simply referred to as CHO cell), dhfr gene-deficient CHO cell (hereinafter simply referred to as CHO (dhfr⁻) cell), mouse L cell, mouse AtT-20, mouse myeloma cell, mouse ATDC5 cell, rat GH3, human FL cell, human 293 cell, human HeLa cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors bearing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Proc. Natl. Acad. Sci. U.S.A., 77, 4505 (1980)] or in SD medium supplemented with 0.5% Casamino acids [Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Nature, 195, 788 (1962)) to which an appropriate additive such as inactivated 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside the transformant.

The protein of the present invention can be separated and purified from the culture described above, for example, by the following procedures.

When the protein of the present invention is extracted from the bacteria or cells of the culture, the bacteria or cell is collected after culturing by a publicly known method and suspended in an appropriate buffer. The bacteria or cell is then disrupted by methods such as ultrasonication, a treatment with lysozyme and/or freeze-thaw cycling, followed by centrifugation, filtration, etc. to produce crude extract of the protein. The buffer used for the procedures may contain a protein denaturant such as urea or guanidine hydrochloride, or a surfactant such as Triton X-100^{™}, etc. When the protein is secreted in the culture broth, the supernatant can be separated, after completion of the cultivation, from the bacteria or cell to collect the supernatant by a publicly known method.

The protein of the present invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein of the present invention thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The protein used in the present invention or its partial peptide can be synthesized by the in vitro translation with the use of a cell-free protein translation system comprising rabbit reticulocyte lysate, wheat germ lysate, E. coli lysate, etc., and RNA which corresponds to DNA encoding the same as a template. The protein or its partial peptide can also be synthesized by a cell-free transcription/translation system further comprising RNA polymerase with the use of DNA encoding calmodulin or its partial peptide as a template. For the cell-free protein (transcription /) translation system, commercially available ones and a method known per se can be employed. In particular, the Escherichia coli extract solution can be prepared according to a method disclosed in Pratt J.M. et al, "Transcription and Tranlation", Hames B.D. and Higgins S.J. edition, IRL Press, Oxford 179-209 (1984). As the commercially available cell lysate, Escherichia coli derived lysates such as E. coli S30 extract system (manufactured by Promega) and RTS 500 Rapid Tranlation System (manufactured by Roche), etc.; rabbit reticulocyte derived lysates such as Rabbit Reticulocyte Lysate System (manufactured by Promega), etc.; and wheat germ derived lysates such as PROTEIOS^{™}(manufactured by TOYOBO) , etc., can be exemplified. Among them, the wheat germ lysate is preferably used. As the method of manufacturing wheat germ lysate, for example, methods disclosed in Johnston F.B. et al, Nature, 179, 160-161 (1957), Erickson A.H. et al, Meth. Enzymol., 96, 38-50 (1996), etc., can be employed.

As a system or device for the protein synthesis, a batch method (Pratt, J.M. et al, (1984) mentions above); continuous cell-free protein synthesis system (SpirinA.S. et al, Science, 242, 1162-1164 (1988)) in which an amino acid, energy source, etc. is continuously supplied in a reaction system; dialysis (Kikawa, et al, 21st Annual meeting of the Molecular Biology Society of Japan, WID6)); a double layer method (PROTEIOS^{™} Wheat germ cell-free protein synthesis core kit instruction manual, manufactured by TOYOBO), and the like can be exemplified. In addition, a method which comprises supplying RNA template, amino acid, energy source, etc., into a synthesis reaction system according to necessity, and eliminating a synthesized or degraded material according to necessity (Japanese Patent Laid-Open No. 2000-333673) can be used.

The antibody against the protein used in the present invention or against its partial peptide (hereinafter, sometimes abbreviated as "antibody of the present invention") can be any of polyclonal or monoclonal antibody, as long as it recognizes the protein of the present invention or its partial peptide. The isotype of the antibody is not particularly limited, but it is preferably IgG, IgM or IgA, particularly preferably IgG.

The antibody of the present invention is not subject to limitation, as long as it has at least a complementality determining region (CDR) for specifically recognizing and binding to the target antigen; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like, and the like.

The antibody against the protein used in the present invention or against its partial peptide (hereinafter sometimes merely referred to as the protein of the present invention in the description of antibody) can be manufactured according to a method of producing an antibody or antisera known per se.

Hereinafter, a preparation method of immunogen of an antibody of the present invention and a production method of the antibody will be described.

### (1) Preparation of antigen

As an antigen used to prepare the antibody of the present invention, any antigen such as the above-mentioned protein of the present invention or its partial peptide, or a (synthetic) peptide having 1 or 2 or more antigenic determinants, which are the same as in the above-mentioned protein of the present invention or its partial peptide, etc. may be used (hereinafter these antigens are sometimes merely referred to as the antigen of the present invention).

The aforesaid protein of the present invention or its partial peptide can be, for example, (a) prepared from warm-blooded animal tissues or cells of human, simian, rat, mouse, chicken, etc., by publicly known methods or with modifications, (b) chemically synthesized by publicly known peptide synthesis methods using a peptide synthesizer, etc., (c) produced by culturing a transformant bearing DNA encoding protein of the present invention or its partial peptide, or (d) biochemically synthesized from nucleic acid encoding protein of the present invention or its partial peptide as a template by using a cell-free transcription/ translation system.
(a) Where the protein of the present invention is prepared from the warm-blooded animal tissues or cells, the tissues or cells are homogenized, and then the crude extract (e.g., membrane fraction, soluble fraction) can be also used per se as an antigen. Alternatively, the tissues or cells are extracted with an acid, a surfactant, an alcohol, etc., and the extract solution can be purified and isolated by a combination of salting-out, dialysis, gel filtration, chromatography techniques such as reverse phase chromatography, ion exchange chromatography, affinity chromatography and the like. Thus obtained protein of the present invention can be used as per se as an immunogen, and can also be used as the immunogen in the form of a partial peptide prepared by limited degradation using a peptidase, etc.
(b) Where the antigen of the present invention is prepared chemically, the synthetic peptides used are, for example, a peptide having the same structure as the protein of the present invention purified from natural material by the above method (a), specifically, a peptide containing 1 or 2 or more amino acid sequences, which are the same amino acid sequences consisting of 3 or more, preferably 6 or more amino acids in an optional region of the amino acid sequence of the protein.
(c) Where the antigen of the present invention is produced using the DNA-bearing transformants, the DNA can be produced in accordance with publicly known cloning techniques [e.g., the method described in Molecular Cloning 2nd ed., (J. Sambrook et al., Cold Spring Harbor Lab. Press, 1989), etc.]. The cloning techniques include (1) a method in which DNAs encoding the antigen are isolated from cDNA library by a hybridization method using DNA probes designed on the basis of the gene sequence encoding the protein of the present invention, and (2) a method in which DNAs encoding the antigen are produced from cDNA as a template by PCR using DNA primers designed based on the gene sequence encoding the protein of the present invention, and the DNA are inserted into an expression vector suitable for a host. Incubating the transformants which is obtained from host by being transformed with the expression vector, in appropriate medium, the desired antigen can be obtained.
(d) When a cell-free transcription/translation system is utilized, a method for synthesizing an mRNA by using an expression vector incorporating DNA that encodes the antigen (for example, an expression vector wherein the DNA is placed under the control of the T7 or SP6 promoter and the like, and the like) as the template, that was prepared by the same method as (c) above, and a transcription reaction mixture comprising an RNA polymerase matching the promoter and its substrates (NTPs); and thereafter performing a translation reaction with the mRNA as the template using a known cell-free translation system (e.g., extract from E. *coli,* rabbit reticulocytes, wheat germ, etc.), and the like can be mentioned. By adjusting the salt concentration and the like appropriately, the transcription reaction and the translation reaction can also be carried out in the same reaction mixture at one time.

As the immunogen, a whole protein molecule of the present invention or a peptide having a partial amino acid sequence of the protein molecule can be used. As examples of the partial amino acid sequence, those comprising 3 or more continuous amino acid residues, preferably those comprising 4 or more, more preferably 5 or more, still more preferably 6 or more continuous amino acid residues, can be mentioned. Alternatively, as examples of the amino acid sequence, those comprising 20 or less continuous amino acid residues, preferably those comprising 18 or less, more preferably 15 or less, still more preferably 12 or less continuous amino acid residues, can be mentioned. A portion of these amino acid residues (e.g., 1 to several residues) may be substituted with a substituent group (e.g., Cys, hydroxyl group, etc.). The peptide used as the immunogen has an amino acid sequence comprising one to several such partial amino acid sequences.

Warm-blooded animal cells itself which express the protein of the present invention can also be used directly as the antigen of the present invention. As the Warm-blooded animal cells, there can be used the naturally occurring cells as described in (a) above, cells transformed by the methods as described in (c) above, etc. Hosts used for the transformation may be any cells as long as they are the cells collected from human, simian, rat, mouse, hamster, chicken etc. and preferably used are HEK293, COS7, CHO-K1, NIH3T3, Balb3T3, FM3A, L929, SP2/0, P3U1, B16, P388, or the like. Naturally occurring warm-blooded animal cells or transformed warm-blooded animal cells, which express the protein of the present invention, can be injected to immune animal as a suspension of the cells in a medium used for tissue culture (e.g., RPMI 1640) or buffer (e.g., Hanks' Balanced Salt Solution). Immunization may be done by any method, as long as it can stimulate antibody production, and preferably used are intravenous injection, intraperitoneal injection, intramuscular injection, subcutaneous injection, etc.

The antigen of the present invention permit direct use for immunization in an insolubilized form, as long as it has immunogenicity; when an antigen of low molecular weight (i.e. , molecular weight about 3,000 or less) having only one to several antigenic determinants in the molecule thereof (for example, a partial peptide of the protein of the present invention) is used, it can be used for immunization in the form of a complex bound or adsorbed to a suitable carrier because these antigens are normally hapten molecules of low immunogenicity. As the carrier, a naturally occurring or synthetic polymer can be used. As examples of the naturally occurring polymer, serum albumin of a mammal such as bovine, rabbit, or human, thyroglobulin of a mammal such as bovine or rabbit, ovalbumin of chicken etc., hemoglobin of a mammal such as bovine, rabbit, human, or sheep, keyhole limpet hemocyanin (KLH) and the like can be used. As examples of the synthetic polymer, various latexes of polymers or copolymers of polyamino acids, polystyrenes, polyacryls, polyvinyls, polypropylenes and the like, and the like can be mentioned.

A mixing ratio of the carrier to the hapten may be in any ratio of any type, as long as the antibody can be efficiently produced to the antigen bound or adsorbed to the carrier. The above described naturally occurring or synthetic high molecular carrier conventionally used to produce an antibody against a hapten may be used in a weight ratio of 0.1 to 100 based on 1 of hapten.

For coupling of the hapten and the carrier, a variety of condensing agents can be used. Examples of the condensing agents, which are advantageously employed, are diazonium compounds such as bis-diazotized benzidine capable of crosslinking tyrosines, histidines or tryptophans; dialdehyde compounds such as glutaraldehyde, etc. capable of crosslinking amino groups with each other; diisocyanate compounds such as toluene-2,4-diisocyanate, etc.; dimaleimide compounds such as N,N'-o-phenylenedimaleimide, etc. capable of crosslinking thiol groups with each other; maleimide activated ester compounds capable of crosslinking an amino group with a thiol group; carbodiimide compounds capable of crosslinking an amino group with a carboxyl group; etc. In the crosslinking of amino groups with each other, one amino group is reacted with an activated ester reagent having dithiopyridyl group (e.g., N-succinimidyl 3-(2-pyridyldithio)propionate (SPDP), etc.) and then reduced to introduce the thiol group, whereas another amino group is introduced with a maleimide group using a maleimide activated ester reagent, and the two groups may be reacted with each other.

### (2) Preparation of monoclonal antibody

### (a) Preparation of monoclonal antibody producing-cell

An antigen of the present invention is administered as is, or along with a carrier or a diluent, to a warm-blooded animal at a site enabling antibody production by the methods such as intraperitoneal injection, intravenous injection, subcutaneous injection, intradermal injection and the like. In order to increase antibody productivity upon the administration, Freund's complete adjuvant or Freund's incomplete adjuvant may be administered. Dosing is normally performed about two to 10 times in total, with one time every 1 to 6 weeks. As examples of the warm-blooded animal used, simians, rabbits, canine, guinea pigs, mice, rats, hamsters, sheep, goats, donkeys and chickens can be mentioned. Although it is preferable to use a mammal of the same species as the recipient in order to avoid the problem of anti-Ig antibody production, mice and rats are generally preferably used for generating a monoclonal antibody.

Since artificial immunization to humans is ethically difficult, it is preferable, when the antibody of the present invention targets a human, (i) to obtain a human antibody by immunizing a human antibody-producing animal (e.g., mouse) produced according to a method described below, (ii) to produce a chimeric antibody, humanized antibody or fully human antibody according to a method described below, or (iii) to obtain a human antibody using in combination the *in vitro* immunization method and cell immortalization with virus, human-human (or -mouse) hybridoma production technique, phage display method and the like. Note that the in vitro immunization method can also be used preferably as a method for obtaining an antigen against an antigen that is unstable and difficult to prepare in large amounts for the purpose of preparing a non-human animal-derived antibody, because there is the possibility of obtaining an antibody against an antigen for which antibody production is suppressed by ordinary immunization, because it is possible to obtain an antibody with an amount of antigen on the nanogram to microgram order, because immunization completes in several days, and for other reasons.

As the animal cells used in the in vitro immunization method, lymphocytes, preferably B-lymphocytes and the like, isolated from peripheral blood, spleen, lymph node and the like of a human and the above-described warm-blooded animals (preferably mouse or rat) can be mentioned. For example, in the case of mouse or rat cells, the spleen is extirpated from an about 4- to 12-week-old animal, and splenocytes are separated and rinsed with a appropriate medium [e.g., Dulbecco' s modified Eagle medium (DMEM), RPMI1640 medium, Ham's F12 medium and the like], after which the splenocytes are suspended in an antigen-containing medium supplemented with fetal calf serum (FCS; about 5 to 20%) and cultured using a CO₂ incubator and the like for about 4 to 10 days. Examples of the antigen concentration include, but are not limited to, 0.05 to 5 µg. It is preferable to prepare a culture supernatant of thymocytes of an animal of the same strain (preferably at about 1 to 2 weeks of age) according to a conventional method, and to add the supernatant to the medium.

Since it is difficult to obtain a thymocyte culture supernatant in *in vitro* immunization of human cells, it is preferable to perform immunization by adding, to the medium, several kinds of cytokines such as IL-2, IL-4, IL-5, and IL-6 and the like, and if necessary, an adjuvant substance (e.g., muramyldipeptide and the like) along with the antigen.

In preparing a monoclonal antibody, it is possible to establish an antibody-producing hybridoma by selecting an individual or cell population showing an elevated antibody titer from among antigen-immunized warm-blooded animals (e. g. , mice, rats) or animal cells (e.g., human, mouse, rat), respectively; collecting spleens or lymph nodes at 2 to 5 days after the final immunization or collecting the cells after 4 to 10 days of cultivation after in vitro immunization to isolate antibody-producing cells; and fusing the isolated cells with myeloma cells. A measurement of serum antibody titer can be performed by, for example, reacting a labeled antigen and an antiserum, and thereafter determining the activity of the label bound to the antibody.

Although the myeloma cells are not subject to limitation, as long as they are capable of producing a hybridoma that secretes a large amount of antibody, those that do not produce or secrete the antibody per se are preferable, with greater preference given to those of high cell fusion efficiency. To facilitate hybridoma selection, it is preferable to use a cell line that is sensitive to HAT (hypoxanthine, aminopterin, thymidine). As examples of the mouse myeloma cells, NS-1, P3U1, SP2/0, AP-1 and the like can be mentioned; as examples of the rat myeloma cells, R210.RCY3, Y3-Ag 1.2.3 and the like can be mentioned; as examples of the human myeloma cells, SKO-007, GM 1500-6TG-2, LICR-LON-HMy2, UC729-6 and the like can be mentioned.

Fusion operation can be performed according to a known method, for example, the method of Koehler and Milstein [Nature, 256, 495 (1975)]. As a fusion promoter, polyethylene glycol (PEG), Sendai virus and the like can be mentioned, and PEG and the like are preferably used. Although the molecular weight of PEG is not subject to limitation, PEG1000 to PEG6000, which are of low toxicity and relatively low viscosity, are preferable. As examples of the PEG concentration, about 10 to 80%, preferably about 30 to 50%, can be mentioned. As the solution for diluting PEG, various buffers such as serum-free medium (e.g., RPMI1640), complete medium comprising about 5 to 20% serum, phosphate buffered saline (PBS), and Tris buffer can be used. DMSO (e.g. , about 10 to 20%) can also be added as desired. As examples of the pH of the fusion solution, about 4 to 10, preferably about 6 to 8 can be mentioned.

The ratio by number of antibody-producing cells (splenocytes) and myeloma cells is preferably normally about 1:1 to 20:1, and the cell fusion can be efficiently performed by incubation normally at 20 to 40°C, preferably at 30 to 37°C, normally for 1 to 10 minutes.

An antibody-producing cell line can also be obtained by infecting antibody-producing cells with a virus capable of transforming lymphocytes to immortalize the cells. As such viruses, for example, Epstein-Barr (EB) virus and the like can be mentioned. Although the majority of persons have immunity because they have ever been infected with this virus in an asymptomatic infection of infectious mononucleosis, virion is also produced when the ordinary EB virus is used; therefore, appropriate purification must be performed. As an EB system free from the possibility of viral contamination, it is also preferable to use a recombinant EB virus that retains the capability of immortalizing B lymphocytes but lacks the capability of replicating virion (for example, deficiency of the switch gene for transition from latent infection state to lytic infection state and the like).

Since marmoset-derived B95-8 cells secrete EB virus, B lymphocytes can be easily transformed by using a culture supernatant thereof. An antibody-producing B cell line can be obtained by, for example, culturing these cells using a medium supplemented with serum and penicillin/streptomycin (P/S) (e. g. , RPMI1640) or a serum-free medium supplemented with a cell growth factor, thereafter separating the culture supernatant by filtration or centrifugation and the like, suspending therein antibody-producing B lymphocytes at a suitable concentration (e.g., about 10⁷ cells/mL), and incubating the suspension normally at 20 to 40°C, preferably at 30 to 37°C, normally for about 0.5 to 2 hours. When human antibody-producing cells are provided as mixed lymphocytes, it is preferable to previously remove T lymphocytes by allowing them to form an E rosette with, for example, sheep erythrocytes and the like, to increase transformation frequency of EB virus, because the majority of persons have T lymphocytes which exhibit cytotoxicity to cells infected with EB virus. It is also possible to select lymphocytes specific for the target antigen by mixing sheep erythrocytes, previously bound with a soluble antigen, with antibody-producing B lymphocytes, and separating the rosette using a density gradient of Percoll and the like. Furthermore, because antigen-specific B lymphocytes are capped by adding the antigen in large excess so that they no longer present IgG on the surface, mixing with sheep erythrocytes bound with anti-IgG antibody results in the formation of rosette only by antigen-nonspecific B lymphocytes. Therefore, by collecting a layer of cells that don't form rosette from this mixture using a density gradient of Percoll and the like, it is possible to select antigen-specific B lymphocytes.

Human antibody-secreting cells having acquired the capability of proliferating infinitely by the transformation can be back fused with mouse or human myeloma cells in order to stably sustain the antibody-secreting ability. As the myeloma cells, the same as those described above can be used.

Hybridoma screening and breeding are normally performed using a medium for animal cells (e.g., RPMI1640) containing 5 to 20% FCS or a serum-free medium supplemented with cell growth factors, with the addition of HAT (hypoxanthine, aminopterin, thymidine). As examples of the concentrations of hypoxanthine, aminopterin and thymidine, about 0.1 mM, about 0.4 µM and about 0.016 mM and the like, respectively, can be mentioned. For selecting a human-mouse hybridoma, ouabain resistance can be used. As human cell lines are more sensitive to ouabain than mouse cell lines, it is possible to eliminate unfused human cells by adding ouabain at about 10⁻⁷ to 10⁻³ M to the medium.

In selecting a hybridoma, it is preferable to use feeder cells or culture supernatants of certain cells. As the feeder cells, an allogenic cell species having a lifetime limited so that it dies after helping the emergence of hybridoma, cells capable of producing large amounts of a growth factor useful for the emergence of hybridoma with their proliferation potency reduced by irradiation and the like, and the like are used. For example, as the mouse feeder cells, splenocytes, macrophage, blood, thymocytes and the like can be mentioned; as the human feeder cells, peripheral blood mononuclear cells and the like can be mentioned. As examples of the cell culture supernatant, primary culture supernatants of the above-described various cells and culture supernatants of various established cell lines can be mentioned.

Moreover, a hybridoma can also be selected by reacting a fluorescein-labeled antigen with fusion cells, and thereafter separating the cells that bind to the antigen using a fluorescence-activated cell sorter (FACS). In this case, efforts for cloning can be lessened significantly because a hybridoma that produces an antibody against the target antigen can be directly selected.

For cloning a hybridoma that produces a monoclonal antibody against the target antigen, various methods can be used.

It is preferable to remove aminopterin as soon as possible because it inhibits many cell functions. In the case of mice and rats, aminopterin can be removed 2 weeks after fusion and beyond because most myeloma cells die within 10 to 14 days. However, a human hybridoma is normally maintained in a medium supplemented with aminopterin for about 4 to 6 weeks after fusion. It is desirable that hypoxanthine and thymidine be removed more than one week after the removal of aminopterin. That is, in the case of mouse cells, for example, a complete medium (e.g., RPMI1640 supplemented with 10% FCS) supplemented with hypoxanthine and thymidine (HT) is added or exchanged 7 to 10 days after fusion. About 8 to 14 days after fusion, visible clones emerge. Provided that the diameter of clone has reached about 1 mm, the amount of antibody in the culture supernatant can be measured.

A measurement of the amount of antibody can be performed by, for example, a method comprising adding the hybridoma culture supernatant to a solid phase (e. g. , microplate) to which the target antigen or a derivative thereof or its partial peptide (including the partial amino acid sequence used as the epitope) is adsorbed directly or with a carrier, subsequently adding an anti-immunoglobulin (IgG) antibody (an antibody against IgG derived from an animal of the same species as the animal from which the original antibody-producing cells are derived is used) or protein A, which had been labeled with a radioactive substance (e.g., ¹²⁵I, ¹³¹I, ³H, ¹⁴C), enzyme (e.g., β-galactosidase, β-glucosidase, alkaline phosphatase, peroxidase, malate dehydrogenase), fluorescent substance (e.g., fluorescamine, fluorescein isothiocyanate), luminescent substance (e.g., luminol, luminol derivative, luciferin, lucigenin) and the like, and detecting the antibody against the target antigen (epitope) bound to the solid phase, a method comprising adding the hybridoma culture supernatant to a solid phase to which an anti-IgG antibody or protein A is adsorbed, adding the target antigen, or a derivative thereof, or its partial peptide labeled with the same labeling agent as described above, and detecting the antibody against the target antigen (epitope) bound to the solid phase, and the like.

Although limiting dilution is normally used as the cloning method, cloning using soft agar and cloning using FACS (described above) are also possible. Cloning by limiting dilution can be performed by, for example, the following procedures, which, however, are not to be construed as limiting.

The amount of antibody is measured as described above, and positive wells are selected. Selected suitable feeder cells are previously added to a 96-well plate. Cells are collected from the antibody-positive wells and suspended in complete medium (e.g., RMPI1640 supplemented with 10% FCS and P/S) to obtain a density of 30 cells/mL; 0.1 mL (3 cells/well) of this suspension is added to the well plate with feeder cells added thereto; the remaining cell suspension is diluted to 10 cells/mL and sown to other wells (1 cell/well) in the same way; the still remaining cell suspension is diluted to 3 cells/mL and sown to other wells (0.3 cells/well). The cells are cultured for about 2 to 3 weeks until a visible clone appears, when the amount of antibody is measured to select positive wells, and the selected cells are recloned in the same way. In the case of human cells, cloning is relatively difficult, so that a plate in which cells are seeded at 10 cells/well is also prepared. Although a monoclonal antibody-producing hybridoma can be obtained normally by two times of subcloning, it is desirable to repeat recloning regularly for several more months to confirm the stability thereof.

Hybridomas can be cultured *in vitro* or *in vivo.*

As a method of *in vitro* culture, a method comprising gradually scaling up a monoclonal antibody-producing hybridoma obtained as described above, from a well plate, while keeping the cell density at, for example, about 10⁵ to 10⁶ cells/mL, and gradually lowering the FCS concentration, can be mentioned.

As a method of *in vivo* culture, for example, a method comprising intraperitoneally injecting a mineral oil to a mouse (a mouse that is histocompatible with the parent strain of the hybridoma) to induce plasmacytoma (MOPC), intraperitoneally injecting about 10⁶ to 10⁷ cells of hybridoma to the mouse5 to 10 days later, and collecting ascites fluid under anesthesia 2 to 5 weeks later, can be mentioned.

### (b)Purification of the monoclonal antibody

Separation and purification of the monoclonal antibody can be performed according to a publicly known method, for example, separation and purification method of immunoglobulin [e.g., salting-out, alcohol precipitation, isoelectric point precipitation, electrophoresis, adsorption-desorption with an ion exchanger (e.g., DEAE, QEAE), ultracentrifugation, gel filtration, specific purification comprising selectively collecting the antibody by means of an antigen-coupled solid phase or an active adsorbent such as protein A or protein G, and dissociating the linkage to obtain the antibody, and the like].

As described above, a monoclonal antibody can be produced by culturing a hybridoma in or outside the living body of a warm-blooded animal, and harvesting an antibody from the body fluid or culture thereof.

When using the antibody of the present invention for cancer preventive / remedy, since the antibody is required to have antitumor activity, it is necessary to examine the level of antitumor activity of provided monoclonal antibody. The antitumor activity can be assayed by comparing the cancer cell growth or induction of apoptosis, in the presence and absence of antibody.

In a preferred mode of embodiment, since the antibody of the present invention is used as a pharmaceutical product having humans as the subject of administration thereof, the antibody of the present invention (preferably a monoclonal antibody) is an antibody whose risk of showing antigenicity when administered to a human has been reduced; to be specific, the antibody is a fully human antibody, a humanized antibody, a mouse-human chimeric antibody and the like, particularly preferably a fully human antibody. A humanized antibody and a chimeric antibody can be prepared by genetic engineering technology according to the method described below. Although a fully human antibody can also be produced from the above-described human-human (or -mouse) hybridoma, it is desirable to produce it using a human antibody-producing animal (e.g., mouse) or the phage display method described below in order to stably supply the antibody in large amounts at low costs.

### (i) Preparation of chimeric antibody

As used herein, "a chimeric antibody" means an antibody wherein the sequences of the variable regions of the H chain and L chain (V_{H} and V_{L}) thereof are derived from a mammalian species, and wherein the sequences of the constant regions (C_{H} and C_{L}) are derived from another mammalian species. The sequences of the variable regions are preferably derived from, for example, an animal species permitting easy preparation of a hybridoma, such as mouse, and the sequences of the constant regions are preferably derived from the recipient mammalian species.

As examples of the method of preparing a chimeric antibody, the method described in US Patent No. 6, 331, 415 or a partially modified method thereof and the like can be mentioned. To be specific, first, mRNA or total RNA is prepared from a monoclonal antibody-producing hybridoma (for example, mouse-mouse hybridoma) obtained as described above, according to a conventional method, to synthesize cDNA. DNAs that encode V_{H} and V_{L} are amplified and purified by PCR according to a conventional method with the cDNA as the template, using appropriate primers [for example, oligo DNAs comprising the base sequences that encode the N-terminal sequences of V_{H} and V_{L}, respectively, as the sense primers, and oligo DNAs that hybridize to the base sequences that encode the N-terminal sequences of C_{H} and C_{L}, respectively, as the antisense primer (see, for example, Bio/Technology, 9: 88-89, 1991)]. In the same manner, DNAs that encode C_{H} and C_{L} are amplified and purified from an RNA prepared from lymphocytes and the like of other mammal (e.g., human) by RT-PCR. V_{H} and C_{H}, and V_{L} and C_{L}, are ligated together, respectively, using a conventional method, and the chimeric H chain DNA and chimeric L chain DNA obtained are inserted into respective appropriate expression vectors [for example, vectors comprising promoters that have transcription activity in CHO cells, COS cells, mouse myeloma cells and the like (e.g., CMV promoter, SV40 promoter and the like)]. The DNAs that encode the two chains may be inserted into separate vectors, and may be inserted into a single vector in tandem. Host cells are transformed with the chimeric H chain and chimeric L chain expression vector (s) obtained. As the host cells, animal cells, for example, Chinese hamster ovary (CHO) cells, simian-derived COS-7 cells, Vero cells, rat-derived GHS cells and the like, in addition to the above-described mouse myeloma cells, can be mentioned. For the transformation, any method applicable to animal cells can be used, with preference given to electroporation method and the like. It is possible to isolate a chimeric monoclonal antibody by culturing the host cells in a medium suitable thereto for a given period, and thereafter collecting the culture supernatant and purifying it in the same manner as described above. Alternatively, it is also possible to obtain a chimeric monoclonal antibody easily and in large amounts from milk or eggs of transgenic animals which are produced by a conventional method using germ line cells of an animal such as bovine, goat, or chicken as the host cells, for which a transgenic technique has been established and a know-how of mass propagation as a domestic animal (domestic fowl) has been accumulated. Furthermore, it is also possible to obtain a chimeric monoclonal antibody in large amounts from the seeds, leaves and the like of a transgenic plant, produced by using microinjection and electroporation into protoplast, the particle gun method and Ti-vector method for intact cells and the like, with cells of a plant such as corn, rice, wheat, soybean, or tobacco as the host cells, for which a transgenic technique has been established, and which is cultured in large amounts as a major crop.

When the chimeric monoclonal antibody obtained is digested with papain, Fab is obtained; when the same is digested with pepsin, F(ab')₂ is obtained.

It is also possible to make scFv by ligating DNAs that encode mouse V_{H} and V_{L} via a suitable linker, for example, DNA that encodes a peptide consisting of 1 to 40 amino acids, preferably 3 to 30 amino acids, more preferably 5 to 20 amino acids [e.g., [Ser-(Gly) m] n or [(Gly) m-Ser] n (m is an integer from 0 to 10, n is an integer from 1 to 5) and the like]. Furthermore, it is possible to make a minibody monomer by ligating DNA that encodes C_{H3} via a suitable linker thereto, or make a scFv-Fc by ligating DNA that encodes C_{H} full length via a suitable linker thereto. The DNA encoding such an antibody molecule modified (coupled) by genetic engineering can be expressed in a microorganism such as E. *coli* or yeast under the control of a suitable promoter, to produce the antibody molecule in large amounts.

When DNAs encoding mouse V_{H} and V_{L} are inserted into the downstream of one promoter in tandem and introduced into E. *coli,* a dimer named as Fv is formed by monocistronic gene expression. When an appropriate amino acid in the FRs of V_{H} and V_{L} is substituted with Cys using molecule modeling, a dimer named as dsFv is formed via the intermolecular disulfide bond between the two chains.

### (ii) Humanized antibody

As used herein, "a humanized antibody" means an antibody wherein the sequences of all regions present in the variable region, other than the complementality determining region (CDR), [i.e., framework region (FR) in constant region and variable region] are derived from a human, and wherein only the sequence of CDR is derived from another mammalian species. The other mammalian species is preferably an animal species, for example, mouse and the like, with which production of hybridomas can be easily performed.

As examples of the method of preparing a humanized antibody, the methods described in US Patent Nos. 5,225,539, 5,585,089, 5,693,761 and 5,693,762 or partially modified methods therefrom and the like can be mentioned. To be specific, DNAs that encode V_{H} and V_{L} derived from a non-human mammalian species (e.g., mouse) are isolated in the same manner as with the above-described chimeric antibody, after which sequencing is performed by a conventional method using an automated DNA sequencer (e.g., manufactured by Applied Biosystems and the like), and the base sequences obtained or deduced amino acid sequences therefrom are analyzed using a known antibody sequence database [for example, Kabat database (see Kabat et al., "Sequences of Proteins of Immunological Interest", edited by US Department of Health and Human Services, Public Health Service, NIH, 5th edition, 1991) and the like] to determine the CDR and FR of the two chains. A base sequence wherein the CDR encoding region of a base sequence that encodes the L chain and H chain of a human antibody having an FR sequence similar to the determined FR sequence [e.g., human κ type L chain subgroup I and human H chain subgroup II or III (see Kabat et al., 1991 (supra))] is substituted with the determined base sequence that encodes the CDR of another animal species, is designed, and the base sequence is divided into fragments of about 20 to 40 bases, and a sequence complementary to the base sequence is divided into fragments of about 20 to 40 bases so that they alternately overlap with the aforementioned fragments. It is possible to construct DNAs that encode V_{H} and V_{L} having human-derived FR and a CDR derived from another mammalian species by synthesizing individual fragments using a DNA synthesizer, and hybridizing and ligating them in accordance with conventional methods. In order to transfer a CDR derived from another mammalian species into human-derived V_{H} and V_{L} more quickly and more efficiently, it is preferable to use PCR-based site directed mutagenesis. As examples of such a method, the sequential CDR grafting method described in Japanese Patent Unexamined Publication No. HEI-5-227970 and the like can be mentioned. It is possible to obtain cells or transgenic animal/plant that produces a humanized antibody by ligating the thus-obtained DNAs that encode V_{H} and V_{L} to DNAs that encode human-derived C_{H} and C_{L}, respectively, in the same manner as with the above-described chimeric antibody, and introducing the ligated product into suitable host cells.

A humanized antibody, like a chimeric antibody, can be modified to scFv, scFv-Fc, minibody, dsFv, Fv and the like by using genetic engineering techniques; and they can be produced in a microorganism such as E. *coli* or yeast by using a suitable promoter.

The technology for preparing a humanized antibody can also be applied to, for example, preparing a monoclonal antibody that can be preferably administered to another animal species for which no hybridoma production technology has been established. For example, animals widely propagated as domestic animals (domestic fowls) such as bovine, swine, sheep, goat, and chicken, and pet animals such as canine and felines, and the like can be mentioned as the subject animal species.

### (iii) Preparation of fully human antibody using human antibody-producing animal

Provided that a functional human Ig gene is introduced into a non-human warm-blooded animal having the endogenous immunoglobulin (Ig) gene knocked out (KO) therein, and that this animal is immunized with an antigen, a human antibody is produced in place of the antibody derived from the animal. Therefore, provided that an animal such as mice, for which a technique for producing a hybridoma has been established, is used, it is possible to acquire a fully human monoclonal antibody by the same method as the conventional method used to prepare a mouse monoclonal antibody. First, some of the human monoclonal antibodies, that were generated by using a human antibody-producing mouse (see Immunol. Today, 17: 391-397, 1996) obtained by crossing a mouse transfected with minigenes of the human Ig H chain and L chain using an ordinary transgenic (Tg) technique with a mouse wherein the endogenous mouse Ig gene has been inactivated using an ordinary KO technique, are already in clinical stage, and to date production of anti-human Ig human antibody (HAHA) has not been reported.

Later, Abgenix Inc. [trade name: XenoMouse (see Nat. Genet., 15: 146-156, 1997; US Patent No. 5,939,598 and the like)] and Medarex Inc. [trade name: Hu-Mab Mouse (see Nat. Biotechnol. , 14: 845-851, 1996; US Patent No. 5, 545, 806 and the like) ] established Tg mice transfected with even a larger human Ig gene using a yeast artificial chromosome (YAC) vector, thus enabling the production of human antibodies of richer repertoire. However, because the human Ig gene, for example, in the case of the H chain, exhibits its diversity as the VDJ exon, which is a variable combination of about 80 kinds of V fragments, about 30 kinds of D fragments and 6 kinds of J fragments, encodes the antigen binding site, the full length thereof is as large as about 1.5 Mb (14th chromosome) for the H chain, about 2 Mb (2nd chromosome) for the κL chain, and about 1 Mb (22nd chromosome) for the λL chain. To reproduce the diverse antibody repertoire in another animal species as in human, it is desirable to introduce the full length of each Ig gene. However, DNA that is insertable into a conventional transfection vector (plasmid, cosmid, BAC, YAC and the like) is normally several kb to several hundred kb in length, and it has been difficult to introduce the full length of Ig genes by the conventional technique for establishing a transgenic animal, which comprises inserting a cloned DNA into a fertilized egg.

Tomizuka et al. (Nat. Genet. , 16: 133-143, 1997) prepared a mouse having the full-length human Ig gene by introducing a natural fragment of a human chromosome harboring the Ig gene (hCF) into a mouse [transchromosomic (TC) mouse]. That is, first, a human-mouse hybrid cell having human chromosomes in which the 14th chromosome comprising the H chain gene and the 2nd chromosome comprising the κL chain gene, both labeled with, for example, a drug-resistance marker and the like, is treated with a spindle formation inhibitor (e.g., colcemid) for about 48 hours to prepare a microcell wherein one to several chromosomes or fragments thereof are enveloped in nuclear membrane, and the chromosomes are introduced into a mouse ES cell by the micronuclear fusion method. A hybrid ES cell retaining the chromosomes having the human Ig gene or fragments thereof is selected using a medium containing a drug, and the cell is microinjected into a mouse embryo in the same manner as with the preparation of an ordinary KO mouse. A germ line chimera is selected among the chimeric mice obtained, with coat color as the index, and the like, to establish a TC mouse strain carrying the human 14th chromosome fragment (TC (hCF14)) and a TC mouse strain carrying the human 2nd chromosome fragment (TC(hCF2)). After establishing mouse strains wherein the endogenous H chain gene and κL chain gene are knocked out, respectively [KO (IgH) and KO (Igκ)] by a conventional method, it is possible to establish a mouse strain having all the four kinds of gene modifications (double TC/KO) by repeating the crossing of these four strains.

Provided that the same method as that for producing an ordinary mouse monoclonal antibody is applied to a double TC/KO mouse established as described above, it is possible to obtain an antigen-specific human monoclonal antibody-producing hybridoma. However, there is the drawback of a lower efficiency to obtain hybridomas than that with the ordinary mouse, because hCF2 containing the κL chain gene is unstable in the mouse cells.

On the other hand, because the aforementioned Hu-Mab mouse has a structure wherein the variable region cluster are doubled although it has about 50% of the κL chain gene, it exhibits a K chain diversity equivalent to that with full length (on the other hand, HuMab mouse exhibits a low H chain diversity and inadequate response to antigen because it carries only about 10% of the H chain gene). And the κ chain is stably retained in the mouse cells because it is inserted in mouse chromosome via a YAC vector (Igκ-YAC). Making use of this advantage, it is possible to get the efficiency for obtaining hybridomas and affinity of antibody to antigen that are equivalent to those with the ordinary mouse, by crossing a TC(hCF14) mouse with a Hu-Mab mouse to establish a mouse that stably retains both hCF14 and Igκ-YAC (trade name: KM mouse).

Furthermore, it is also possible to establish a human antibody-producing animal in which the λL chain gene is further transfected to reconstruct the diverse human antibody repertoire more completely. Such an animal can also be obtained by producing a TC mouse in which the human 22nd chromosome or a fragment thereof harboring the λL chain gene is introduced in the same manner as described above [TC (hCF22)], and crossing the mouse with the above-described double TC/KO mouse or KM mouse, or can also be obtained by, for example, constructing a human artificial chromosome (HAC) comprising both the H chain locus and the λL chain locus, and introducing it into a mouse cell *(*Nat. Biotechnol., 18: 1086-1090, 2000).

When an antibody of the present invention is used as a medicine, an antibody of the present invention is desirably a monoclonal antibody, but it may be a polyclonal antibody. When the antibody of the present invention is a polyclonal antibody, it is not necessary to use hybridoma; therefore, provided that a human antibody-producing animal is produced in the same manner as described above using an animal species for which no technique for preparing a hybridoma has been established but a transgenic technique has been established, preferably an ungulate such as bovine, it is also possible to produce a human antibody in larger amounts at low costs (see, for example, Nat. Biotechnol., 20: 889-894, 2002). The human polyclonal antibody thus obtained can be purified by collecting blood, ascites fluid, milk, egg and the like, preferably milk or egg, of the human antibody-producing animal, in combination with the same purification techniques as described above.

### (iv) Preparation of fully human antibody using phage display human antibody library

Another approach to produce a fully human antibody is a method using phage display. This method sometimes encounters cases in which a mutation due to PCR is introduced into a site other than CDRs; for this reason, a few reports of cases of HAHA production in clinical stage are available. On the other hand, however, the method has advantages such as no risk of cross-species viral infection derived from the host animal and the indefinite specificity of the antibody (antibodies against forbidden clone, sugar chain and the like can also be easily prepared).

The method of preparing a phage display human antibody library include, but are not limited to, for example, the methods described below.

Although a phage used is not subject to limitation, filamentous phage (Ff bacteriophage) is normally preferably used. As the method of presenting a foreign protein on the phage surface, a method comprising expressing and presenting the foreign protein as a fusion protein with any of the coat proteins g3p, and g6p to g9p on the coat protein can be mentioned; and a method comprising fusing the foreign protein to the N-terminal side of g3p or g8p is often used. As the phage display vector, 1) one in which the foreign gene is introduced in the form of fusion gene with the coat protein gene of the phage genome, to allow all the coat proteins presented on the phage surface to be presented as a fusion protein with the foreign protein, 2) one in which the gene encoding the fusion protein is inserted separately from the wild-type coat protein gene to allow the fusion protein and the wild-type coat protein to be expressed simultaneously, and 3) one in which an E. coli having a phagemid vector harboring the gene that encodes the fusion protein is infected with a helper phage having the wild-type coat protein gene to produce phage particles that express the fusion protein and the wild-type coat protein simultaneously, and the like can be mentioned. However, a phage display vector of the type 2) or 3) is used for the preparation of an antibody library, because in the case of 1), the capability of infection is lost when a large foreign protein is fused.

As a specific vector, those described by Holt et al. (Curr. Opin. Biotechnol., 11: 445-449, 2000) can be mentioned as examples. For example, pCES1 (see J. Biol. Chem., 274: 18218-18230, 1999) is an Fab-expressing phagemid vector wherein DNA encoding the κL chain constant region allocated to downstream of the g3p signal peptide, and DNA encoding CH3, His-tag, c-myc tag, and the amber stop codon (TAG) followed by the g3p coding sequence, allocated to downstream of the g3p signal peptide, are arranged under the control of one lactose promoter. When this is introduced to an *E. coli* having an amber mutation, Fab is presented onto the g3p coat protein, but when it is expressed in the HB2151 strain and the like, which do not have an amber mutation, a soluble Fab antibody is produced. And as the scFv-expressing phagemid vector, for example, pHEN1 (J. Mol. Biol., 222: 581-597, 1991) and the like are used.

Meanwhile as examples of the helper phage, M13-KO7, VCSM13 and the like can be mentioned.

And as another phage display vector, a vector that is designed so that a sequence comprising the cysteine-encoding codon is linked to each of the 3' end of the antibody gene and the 5' end of the coat protein gene to express the two genes simultaneously and separately (not in the form of a fusion protein), and to present the antibody onto the coat protein on the phage surface via S-S bonds between the introduced cysteine residues (CysDisplay^{™} technology of Morphosys AG) and the like, can be mentioned.

As the kind of human antibody library, a naive/non-immunized library, a synthetic library, an immunized library and the like can be mentioned.

The naive/non-immunized library is a library obtained by acquiring the V_{H} and V_{L} genes retained by a normal human by RT-PCR, and randomly cloning them into the above-described phage display vector. Normally, mRNA derived from lymphocytes of peripheral blood, bone marrow, tonsil and the like of a normal human, and the like are used as the template. A library prepared by selectively amplifying IgM-derived mRNA in which a class switch due to antigen sensitization is not undergoing, to avoid V gene biases such as clinical history, is particularly called a naive library. Representatively, the library of Cambridge Antibody Technology (see J. Mol. Biol., 222: 581-597, 1991; Nat. Biotechnol., 14: 309-314, 1996), the library of Medical Research Council *(see* Annu. Rev. Immunol. , 12: 433-455, 1994) , the library of Dyax Corp. (see J. Biol. Chem., 1999 *(supra);* Proc. Natl. Acad. Sci. USA, 14: 7969-7974, 2000) and the like can be mentioned.

A synthetic library is obtained by selecting a functional particular antibody gene in human B cells, and substituting a portion of antigen-binding region in a V gene fragment, for example, CDR3 and the like, with DNAs encoding a random amino acid sequence of appropriate length, to construct a library. It is recognized to be excellent in antibody expression efficiency and stability because the library can be constructed with the combination of the V_{H} and V_{L} genes, which produce functional scFv and Fab, since the beginning. Representatively, the HuCAL library of Morphosys AG (see J. Mol. Biol., 296: 57-86, 2000), the library of BioInvent (see Nat. Biotechnol. , 18: 852, 2000), the library of Crucell (see Proc. Natl. Acad. Sci. USA, 92: 3938, 1995; J. Immunol. Methods, 272: 219-233, 2003) and the like can be mentioned.

An immunized library is a library obtained by preparing an mRNA from lymphocytes collected from a human such as a patient with cancer, autoimmune disease, infectious disease and the like or a recipient of vaccination, having an elevated blood antibody titer against the target antigen, or from human lymphocytes and the like which are artificially immunized with the target antigen by the above-described *in vitro* immunization method, in the same manner as with the above-described naive/non-immunized library, and amplifying the V_{H} and V_{L} genes by RT-PCR, to construct a library. It is possible to obtain the desired antibody even from such libraries of relatively small size because the desired antibody gene is contained in the library already at the beginning.

The wider the diversity of the library is, the better; actually, however, an appropriate library size is about 10⁸ to 10¹¹ clones, taking into consideration of the number of phages handlable in the following panning operation (10¹¹ to 10¹³ phages) and the number of phages necessary to isolate and amplify clones in ordinary panning (100 to 1,000 phages/clone); it is possible to screen for an antibody normally having a Kd value on the order of 10⁻⁹ with a library of about 10⁸ clones.

The process for selecting an antibody against the target antigen by the phage display method is referred to as panning. To be specific, for example, a phage presenting an antigen-specific antibody is concentrated by repeating a series of operations of bringing an antigen-immobilized carrier and a phage library into contact with each other, washing out the unbound phage, thereafter eluting the bound phage from the carrier, and infecting the phage to E. *coli* to propagate it, about 3 to 5 times. As the carrier for immobilizing the antigen, various carriers used in ordinary antigen-antibody reactions or affinity chromatography, for example, insoluble polysaccharides such as agarose, dextran, and cellulose, synthetic resins such as polystyrene, polyacrylamide, and silicon, or microplates, tubes, membranes, columns, beads and the like comprising glass, metal and the like, and surface plasmon resonance (SPR) sensor chips, and the like can be mentioned. For the antigen immobilization, physical adsorption may be used, and a method using a chemical bond used to insolubilize and immobilize a protein or enzyme and the like is also acceptable. For example, a biotin-(strept)avidin system and the like are preferably used. When the endogenous ligand, that is a target antigen, is a small molecule such as a peptide, it is necessary to pay special attention to prevent masking of the portion used as the epitope by conjugating with the carrier. For washing the unbound phage, a blocking solution such as BSA solution (1 to 2 times), a PBS containing a surfactant such as Tween (3 to 5 times) and the like can be used sequentially. There is also a report mentioning that the use of citrate buffer (pH 5) and the like is preferable for the washing. For elution of the specific phage, an acid (e.g., 0.1 M hydrochloric acid and the like) is normally used; cleavage with a specific protease (for example, a gene sequence that encodes the trypsin cleavage site can be introduced into the linkage site between the antibody gene and the coat protein gene. In this case, E. coli infection and propagation are possible even if all the coat protein is expressed in the form of a fusion protein because the wild-type coat protein is presented on the surface of the eluted phage), competitive elution with a soluble antigen, or elution by reduction of S-S bond (for example, in the aforementioned CysDisplay^{™}, the antigen-specific phage can be recovered by dissociating the antibody and the coat protein by using a suitable reducing agent after performing panning.) is also possible. When elution has been performed with an acid, the eluate is neutralized with Tris and the like, and the eluted phage is then infected to E. coli, which is cultured; after which the phage is recovered by a conventional method.

After the phage presenting the antigen-specific antibody is concentrated by panning, the phage is infected to E. *coli* and the cells are sown onto a plate to perform cloning. The phage is again collected, and the antigen binding activity is confirmed by the above-described antibody titer assay (e.g., ELISA, RIA, FIA and the like) or a measurement utilizing FACS or SPR.

Isolation and purification of the antibody from the selected phage clone that presents the antigen-specific antibody can be performed by, for example, when using a vector incorporating an amber stop codon at the linker site of the antibody gene and the coat protein gene as the phage display vector, infecting the phage to an E. *coli* that does not have amber mutation (e.g., HB2151 strain) to produce and secrete soluble antibody molecules in periplasm or the medium, lysing the cell wall with lysozyme and the like, collecting the extracellular fraction, and purifying using the same purification technique as described above. Provided that the His-tag or c-myc tag has been introduced in advance, the antibody can easily be purified by using IMAC, an anti-c-myc antibody column and the like. When cleavage with a specific protease is utilized in panning, the antibody molecule is separated from the phage surface by an action of the protease, so that the desired antibody can be purified by performing the same purification operation as above mentioned.

The technology for producing a fully human antibody using a human antibody-producing animal and a phage display human antibody library can also be applied to the production of a monoclonal antibody derived from another animal species. For example, animals widely propagated as domestic animals (domestic fowls) such as bovine, swine, sheep, goat, and chicken, and pet animals such as canine and feline, and the like can be mentioned as the subject animal species. In non-human animals, the utilization of an immunized library is more effective because there are fewer ethical problems concerning artificial immunization with the target antigen.

### (3) Preparation of polyclonal antibody

The polyclonal antibody of the present invention can be manufactured by publicly known methods or modifications thereof. For example, a warm-blooded animal is immunized with an immunogen (protein or peptide antigen) per se, or with a complex of immunogen and a carrier protein formed in a manner similar to the method described above for the manufacture of monoclonal antibodies. The product containing the antibody against the protein of the present invention is collected from the immunized animal followed by separation and purification of the antibody.

In the complex of immunogen and carrier protein used to immunize a warm-blooded animal, the type of carrier protein and the mixing ratio of carrier protein to hapten may be any type and in any ratio, as long as the antibody is efficiently produced to the hapten immunized by crosslinking to the carrier protein. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier protein to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 1 to 6 weeks and about 2 to 10 times in total.

The polyclonal antibody can be collected from the blood, ascites, etc., preferably from the blood of warm-blooded animal immunized by the method described above.

The polyclonal antibody titer in antiserum can be assayed, by the same procedure as the assay of antibody titer of the antiserum described above. The separation and purification of the polyclonal antibody can be carried out, following the method for the separation and purification of immunoglobulins performed as in the separation and purification of monoclonal antibodies described hereinabove.

The polynucleotide having the base sequence complementary to the target region of target polynucleotides, that is, the polynucleotides hybridizable to target polynucleotides, can be denoted to be 'antisense' to the target polynucleotides.

The antisense polynucleotide having a complementary or substantially complementary base sequence or a part thereof to a base sequence of the polynucleotide (e.g., DNA (hereinafter these DNAs that encode the protein of the present invention are sometimes collectively referred to as the DNA of the present invention in the description of antisense polynucleotide)) that encodes the protein of the present invention, can be any antisense polynucleotide, as long as it has a complementary or substantially complementary base sequence or a part thereof to a base sequence of the polynucleotide (e.g., DNA) that encodes the protein of the present invention, and is capable of suppressing the expression of the polynucleotide.

The base sequence substantially complementary to the DNA of the present invention may include, for example, a base sequence having about 70% or more homology, preferably about 80% or more homology, more preferably about 90% or more homology and most preferably about 95% or more homology, for the region overlapping to the base sequence complementary to the DNA of the present invention (i.e., complementary strand to the DNA of the present invention), and the like. The term 'homology' has the same meaning as the case of the DNA of the present invention mentioned above. Especially in the entire base sequence of the complementary strand to the DNA of the present invention, preferred are (a) an antisense polynucleotide having about 70% or more homology, preferably about 80% or more homology, more preferably about 90% or more homology and most preferably about 95% or more homology, to the complementary strand of the base sequence which encodes the N-terminal region of the protein of the present invention (e.g. , the base sequence around the initiation codon) in the case of antisense polynucleotide directed to translation inhibition and (b) an antisense polynucleotide having about 70% or more homology, preferably about 80% or more homology, more preferably about 90% or more homology and most preferably about 95% or more homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to the base sequence represented by SEQ ID NO: 1, SEQ ID NO: 3, SEQ ID NO: 5, SEQ ID NO: 7 or SEQ ID NO: 9, preferably an antisense polynucleotide containing the entire or part of a base sequence complementary to the base sequence represented by SEQ ID NO: 1, SEQ ID NO: 3, SEQ ID NO: 5, SEQ ID NO: 7 or SEQ ID NO: 9.

The antisense polynucleotide having a complementary or substantially complementary base sequence or a part thereof to a base sequence of the DNA of the present invention (hereinafter it will also be called as the antisense polynucleotide of the invention) can be designed and synthesized on the basis of the base sequence information of cloned or identified protein-encoding DNA of the present invention. Such an antisense polynucleotide can inhibit replication or expression of the genes encoding the protein of the invention (hereinafter will also simply called as the "gene of the present invention') . Accordingly, an antisense polynucleotide of the present invention is hybridizable to RNA transcribed from a gene of the present invention (mRNA or primary transcript) to inhibit the synthesis (processing) or function (translation to a protein) of said mRNA or is capable of modulating and/or controlling the expression of a gene of the present invention via interaction with RNA associated with the gene of the present invention. Polynucleotides complementary to the selected sequences of RNA associated with the gene of the present invention and polynucleotides specifically hybridizable to RNA associated with the gene of the present invention are useful in modulating/controlling the in vivo and in vitro expression of the gene of the present invention, and are useful for the treatment or diagnosis of diseases, etc.

Target region of an antisense polynucleotide of the present invention is not particularly limited in its length as long as the translation into a protein of the present invention is inhibited as a result of hybridization of an antisense polynucleotide, and it may be a whole sequence or a partial sequence of mRNA encoding the protein which can be exemplified by a short strand of about 10 bases and a long strand of mRNA or whole sequence of early transcription product. In consideration of a simple synthesis and an antigenic problem, oligonucleotide comprising about 10 to 40 bases, particularly about 15 to 30 bases is preferable, but it is not limited thereto. In specific, in the genes of the present invention, the 5' end hairpin loop, 5' end 6-base-pair repeats, 5' end untranslated region, translation initiation codon, protein coding region, ORF translation termination codon, 3' end untranslated region, 3' end palindrome region, 3' end hairpin loop, and the like, may be selected as preferred target regions of an antisense polynucleotide, though any other region may be selected as a target in the genes of the present invention. For example, an intron-part of the gene can be exemplified as the target region.

Further, the antisense polynucleotide of the present invention may be a polynucleotide in which the translation into a protein is inhibited by hybridizing with mRNA of a protein of the present invention or with early transcription product, and it may as well as be the polynucleotide capable of forming a triplex by binding with the double-stranded DNA which is the gene of the present invention and inhibiting the transcription of RNA. Moreover, it may be a polynucleotide forming a DNA: RNA hybrid and directing the degradation by RNaseH.

To prevent digestion with a hydrolase such as nuclease, etc., the phosphoric acid residue (phosphate) of each nucleotide that constitutes the antisense polynucleotide may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to DNA containing the base sequence represented by SEQ ID NO: 1, SEQ ID NO: 3, SEQ ID NO: 5, SEQ ID NO: 7 or SEQ ID NO: 9.

Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e. g. , phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms 'nucleoside', 'nucleotide' and 'nucleic acid' are used to refer to moieties that contain not only the purine and pyrimidine bases, but also other heterocyclic bases, which have been modified. Such modifications may include methylated purines and pyrimidines, acylated purines and pyrimidines or other heterocyclic rings. Modified nucleotides and modified nucleotides also include modifications on its sugar moiety, wherein, for example, one or more hydroxyl groups may optionally be substituted with a halogen atom(s), an aliphatic group(s), etc., or may be converted into the functional groups such as ethers, amines, or the like.

The antisense polynucleotide of the present invention is RNA, DNA or a modified nucleic acid (RNA, DNA). Specific examples of the modified nucleic acid are, but not limited to, sulfur and thiophosphate derivatives of nucleic acids and those resistant to degradation such as polynucleoside amides or oligonucleoside amides. The antisense polynucleotide of the present invention can be modified, for example, based on the following design, that is, by increasing the intracellular stability of the antisense polynucleotide, enhancing the cell permeability of the antisense polynucleotide, increasing the affinity of the nucleic acid to the targeted sense strand to a higher level, or minimizing the toxicity, if any, of the antisense polynucleotide. Many of such modifications are known in the art, as disclosed in J. Kawakami, et al., Pharm. Tech. Japan, Vol. 8, pp. 247 or pp. 395, 1992; Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

In addition, the antisense polynucleotide of the present invention includes a ribozyme capable of specifically cleaving the internal (intron is included in early transcription products) coding region of mRNA which encodes the protein of the present invention or an early transcription product. 'ribozyme' is RNA having enzymatic activity for cleaving nucleic acid, but since recently it is discovered that the oligoDNA having a base sequence of the enzymatic activity site also has the activity for cleaving nucleic acid, the present specification also includes DNA as long as it has a sequence specific enzymatic activity for cleaving nucleic acid. Riobzyme with mostly high-generality includes self-splicing RNA which can be found in infectious RNA such as viroid, a virusoid, etc. , and hammer-head type or hairpin type are known. The hammer-head type exhibits enzymatic activity at about 40 bases, and it is possible to specifically cleave only the target mRNA by complementary arranging several bases (about 10 bases in total) of both ends adjacent to the part having a hammer-head structure. This type of ribozyme has a further advantage that genomic DNA is never targeted as its substrate is only RNA. When mRNA of protein of the present invention forms itself a double-stranded structure, the target sequence can be formed into a single-strand by using hybrid ribozyme coupled with RNA motif derived from viral nucleic acid which specifically binds to RNA helicase [Proc. Natl. Acad. Sci. USA, 98 (10) : 5572-5577 (2001)]. Also, in a case where ribozyme is used in the form of an expression vector having DNA which encodes the ribozyme, the ribozyme can be hybrid ribozyme further coupled with the sequence of modified tRNA so as to promote transport to cytoplasm of a transcriptional product [Nucleic Acids Res., 29 (13): 2780-2788 (2001)].

In the present specification, the antisense polynucleotide of the present invention is also defined to include so-called siRNA which is double-stranded RNA comprising oligoRNA complementary to a partial sequence of coding region (intron is included in early transcription products) in mRNA of the protein of the present invention or early transcription products and a strand complementary to the oligoRNA. The phenomenon of so-called RNA interference (RNAi) in which mRNA complementary to the RNA introduced is degraded by introducing short-stranded mRNA into a cell, is known to occur in nematode, an insect, plant, etc., but after it is confirmed that the phenomenon also occurs in animal cells [Nature, 411 (6836): 494-498 (2001)] , it is widely used as an alternative technology of ribozyme. siRNA can be appropriately designed on the basis of base sequence information of target mRNA by using a commercially available software (e.g., RNAi Designer; Invitrogen).

The antisense oligonucleotide of the present invention and ribozyme can be prepared by determining a target sequence of mRNA or early transcription product on the basis of a cDNA sequence or genomic DNA sequence of the gene of the present invention, and synthesizing its complementary sequence with the use of a commercially available DNA/RNA automatic synthesizer (Applied Biosystems, Beckman, etc.). siRNA can be prepared according a process comprising synthesizing a sense strand and an antisense strand respectively with the DNA/RNA automatic synthesizer, denaturing in a suitable annealing-buffer solution at about 90 to 95 °C for about 1 minute, and annealing at about 30 to 70 °C for about 1 to 8 hours. In addition, longer double-stranded polynucleotide can be prepared according to a process comprising synthesizing complementary oligonucleotides in an alternately overlapping manner, annealing the oligonucleotides, and ligating with ligase. Alternatively, siRNA can be designed such that it is synthesized as RNA (shRNA) in which a sense strand and an antisense strand are linked via a linker of suitable length (for example, about 3 to 10 bases), and that it can be subjected for processing by an enzyme dicer or the like in an animal cell to which the RNA is introduced. Further, siRNA may be formed by preparing an expression vector in which DNAs encoding a sense strand and an antisense strand are under control of respective Pol III promoter such as U6 or H1, or an expression vector in which DNA encoding an RNA strand, which comprises the sense strand and the antisense strand linked via a linker, is under control of Pol III promoter, and expressing the vector in an animal cell.

Inhibitory activity of the antisense polynucleotide of the present invention can be examined by transformants to which gene of the present invention is introduced, in vivo or in vitro gene expression system of the present invention, or in vivo or in vitro protein translation system of the present invention.

Hereinafter, the use of protein of the present invention or its partial peptide (hereinafter, sometimes merely abbreviated as 'protein of the present invention'), DNA encoding the protein of the present invention or its partial peptide (hereinafter, sometimes merely abbreviated as 'DNA of the present invention'), antibody of the present invention described above, and antisense polynucleotide of the present invention described above, will be explained.

The protein of the present invention can be used as a disease marker as its expression is increased in cancer tissue. In other words, the protein is useful as a marker for early diagnosis in cancer tissue, judgment of symptomatic disease severity, prediction of disease progression.

In specific, by the inhibition of expression and/or activity of Desmocollin-3, TM4SF6, and LY-6K of the protein of the present invention, an apoptosis in cancer cells is promoted, growth of cancer cells is inhibited. Therefore, a medicine comprising the antisense polynucleotide of the present invention, antibody of the present invention, or substance inhibiting the expression and/or activity of protein described above (e.g., low molecular compound) can be used as an agent for promoting the apoptosis in cancer cells and/or inhibiting the growth of cancer cells, that is, it can be used as preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably, preventive/remedy agents for breast cancer, lung cancer, colon cancer, prostate cancer, ovary cancer, pancreatic cancer, etc.). In the present specification, term 'cancer cell' is used to include cells directing to turn cancerous in the future.

TM4SF13 can interact with integrin and change relations between the integrin and an extracellular matrix component (for example, fibronectin, laminin, etc.). There is reported that expression of the integrin is closely related to a malignant grade such as a growth rate and metastasis in in vivo of cancer cells, and it has been known that the integrin binds with its ligand such as fibronectin, laminin, thereby plays an important role in metastasis process of cancer cells such as basal membrane invasion or the like. There is reported that HER-2 signal is suppressed with high expression of the integrin, and thus growth, tumorigenicity of colon cancer are reduced (Mol Biol Cell. 1995 ;6(6):725-740., J Biol Chem. 2005 ;280(19):19027-19035).

By controlling (inhibition, promotion, or the like) the expression of TM4SF13 and/or an interaction of the protein and integrin, the apotosis in cancer cells is promoted and growth of cancer cells is suppressed, therefore, a medicine comprising the antisense polynucleotide of the present invention, antibody of the present invention, or substance controlling the expression of the above protein and/or interaction of the protein and integrin (e.g., antibody, polynucleotide, etc.) can be used as an agent for promoting the apoptosis in cancer cells and/or inhibiting the growth of cancer cells, that is, it can be used as preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably, preventive/remedy agents for breast cancer, lung cancer, colon cancer, prostate cancer, ovary cancer, pancreatic cancer, etc.).

### (1)Pharmaceutical comprising antibody of the present invention

The antibody (neutralizing antibody) of the present invention is capable of neutralizing activity of the protein of the present invention, therefore can be used as an agent for promoting apoptosis in cancer cells, agent for inhibiting cancer cell growth, and preventive/remedy agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably, preventive/remedy agents for breast cancer, lung cancer, colon cancer, prostate cancer, ovary cancer, pancreatic cancer, etc.). The term 'neutralizing' in the present invention is defined to cover antibody-dependent cell-mediated cytotoxicity activity (ADCC activity), complement-dependent cytotoxicity activity (CDC activity), inhibition of growth signal in cancer cells (neutralization activity in the narrow sense), induction of an apoptosis, etc. (not limited thereto).

Since there is a possibility that TM4SF13 act to promote or inhibit the cell growth depending on the environment of cell periphery (e. g. , organs, cancer types, etc.) as described above, an antibody for TM4SF13 can be any one of neutralizing antibody or non-neutralizing antibody (preferably, agonist antibody) depending on the environment of cell periphery. In the present invention, the term 'non-neutralizing antibody' means that the antibody does not inhibit the interaction of TM4SF13 and integrin, or inhibit TM4SF13 to such an extent of maintaining the interaction of TM4SF13 and integrin necessary for suppressing cancers, as a whole. Namely, due to the stabilization and increased level of TM4SF13 by the antibody, TM4SF13 molecules may exert the desired activity as a whole, even if the activity of each TM4SF13 molecule is partially inhibited. As a result, the antibody does not appear to neutralize TM4SF13. In the present invention, the agonist antibody means an antibody increasing the interaction of TM4SF13 and integrin.

The preventive/remedy agents for the above diseases and the promoting agent comprising the antibody of the present invention are low-toxic and can be administered as they are in the form of liquid preparations, or as pharmaceutical compositions of suitable preparations to human or mammals (e. g. , rats, rabbits, sheep, swine, bovine, feline, canine, simian, etc.), orally or parenterally (e.g., intravascularly, subcutaneously, etc.).

The antibody of the present invention may be administered in itself, or may be administered as an appropriate pharmaceutical composition. The pharmaceutical composition used for the administration may contain a pharmacologically acceptable carrier, diluent or excipient with the antibody of the present invention and salt thereof. Such a pharmaceutical composition is provided in the form of pharmaceutical preparations suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody of the present invention described above or the salts thereof in a sterile aqueous medium or oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the antibody described above or the salts thereof with conventional bases for suppositories.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and may contain a carrier, a diluent or excipient conventionally used in the field of pharmaceutical preparations. Examples of the carrier or excipient for tablets are lactose, starch, sucrose, magnesium stearate, etc.

Advantageously, the pharmaceutical compositions for parenteral or oral use described above are prepared into pharmaceutical preparations with a unit dose suited to fit a dose of the active ingredients. Such unit dose preparations include, for example, tablets, pills, capsules, injections (ampoules), suppositories, etc. The amount of the antibody contained is generally 5 to 500 mg per dosage unit form; it is preferred that the antibody described above is contained in 5 to 100 mg especially in the form of injection, and in 10 to 250 mg for the other forms.

The dose of the agent described above containing the antibody of the present invention may vary depending upon subject to be administered, target disease, conditions, route of administration, etc. For example, when used for the purpose of treating/preventing breast cancer in adult, it is advantageous to administer the antibody of the present invention intravenously in a dose of about 0.01 to about 20 mg/kg body weight, preferably about 0.1 to about 10 mg/kg body weight and more preferably about 0.1 to about 5 mg/kg body weight, about 1 to 5 times/day, preferably about 1 to 3 times/day. In other parenteral and oral administration, the agent can be administered in a dose corresponding to the dose given above. When the condition is especially severe, the dose may be increased according to the condition.

The antibody of the present invention may be administered in itself, or may be administered as an appropriate pharmaceutical composition. The pharmaceutical composition used for the administration may contain a pharmacologically acceptable carrier, diluent, or excipient with the antibody or the salts thereof. Such a composition is provided in the form of pharmaceutical preparations suitable for oral or parenteral administration (e.g., intravascular injection, subcutaneous injection, etc.).

Each composition described above may further contain other active components unless they cause any adverse interaction with the antibody described above due to blending.

Furthermore, the antibody of the present invention may be used in combination with other drugs, for example, alkylating agents (e.g., cyclophosphamide, ifosfamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, Taxol, etc.), cisplatin, carboplatin, etoposide, irinotecan, etc. The antibody of the present invention and the substance described above may be administered simultaneously or at staggered times to the patient.

### (2) Pharmaceutical comprising antisense polynucleotide

The antisense polynucleotide of the present invention which is capable of binding complementarily to a transcription product of the gene of the present invention and suppressing the expression of the gene is low toxic and can suppress the in vivo functions or actions of the protein or gene of the present invention to induce apoptosis in cancer cells. Thus, the antisense polynucleotide can also be used as an agent for promoting apoptosis in cancer cells, agent for inhibiting cancer cell growth, and preventive/remedy agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably, preventive/remedy agents for e.g., breast cancer, lung cancer, colon cancer, prostate cancer, ovary cancer, pancreatic cancer, etc.).

For the use of the antisense polynucleotide of the present invention as a preventive / remedy agent, an agent for promoting apoptosis, an agent for inhibiting growth as above described and the like, it can be prepared into pharmaceutical preparations by publicly known methods, which are provided for administration.

In addition, for example, the antisense polynucleotide can be administered alone, or the antisense polynucleotide can be inserted into an appropriate vector such as retrovirus vector, adenovirus vector, adenovirus-associated virus vector, etc., and then administered. The antisense polynucleotide may be administered orally or parenterally to human or a mammal (e. g. , rat, rabbit, sheep, swine, bovine, feline, canine, simian etc.) in a conventional manner. The antisense polynucleotide may also be administered as it stands, or may be prepared in pharmaceutical preparations together with a physiologically acceptable carrier such as auxiliary substance to promote its uptake, which are then administered by gene gun or through a catheter such as a hydrogel catheter. Alternatively, the antisense polynucleotide may be prepared into an aerosol, which is topically administered into the trachea as an inhaler.

Further for the purposes of improving pharmacokinetics in the body, prolonging a half-life, and improving intracellular uptake efficiency, the antisense polynucleotide described above may be administered alone, or may be prepared into pharmaceutical preparations (injectable preparations) together with a carrier such as liposome, etc. and the preparations may be administered intravenously, subcutaneously, etc.

A dose of the antisense polynucleotide may vary depending on target disease, subject to be administered, route for administration, etc. For example, where the antisense polynucleotide of the present invention is administered for the purpose of treating breast cancer, the antisense polynucleotide is generally administered to an adult (60 kg body weight) in a daily dose of about 0.1 to 100 mg.

In addition, the polynucleotide having a base sequence that encodes the protein of the present invention or a part of the base sequence (also referred to as 'sense polynucleotide of the present invention' ) and the antisense polynucleotide of the present invention may also be used as a nucleotide probe (or primer) for diagnosis to examine the states of expression of the gene of the present invention in tissues or cells.

### (3) Screening for drug candidates for disease

The protein of the present invention is increasingly expressed in cancer tissues. When expression and/or activity of the Desmocollin-3, TM4SF6 and LY-6K proteins are/is inhibited, or expression of TM4SF13 protein and/or interaction of the protein and integrin are/is controlled, apoptosis in cancer cell is induced, whereby cancer cell growth is inhibited. Therefore, the compounds or their salts that inhibit the expression and/or activity of the Desmocollin-3, TM4SF6 and LY-6K proteins, or the compounds or their salts that controls expression of the TM4SF13 protein and/or interaction of the protein and integrin are useful as agents for promoting apoptosis in cancer cells, agents for inhibiting cancer cell growth, and preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.) (preferably, preventive/remedy agents for breast cancer, lung cancer, colon cancer, prostate cancer, ovary cancer, pancreatic cancer, etc.).

Accordingly, the protein of the present invention (Desmocollin-3, TM4SF6 and LY-6K) is useful as a reagent for screening for the compounds or their salts that inhibit the expression and/or activity of the protein of the present invention.

That is, the present invention provides a method of screening for the compounds or their salts that inhibit the expression and/ or activity of the protein of the present invention, in which the method is characterized in that the protein of the present invention is used.

The method of screening for the compounds or their salts that inhibits the activities of the protein of the present invention is classified broadly into followings;
(a-1) a method of comparing activity of isolated protein of the present invention in the presence and absence of a test compound, and
(a-2) a method of culturing a cell capable of producing the protein of the present invention in the presence of and absence of test compound, and comparing activity of the protein of the present invention under two different conditions.

The protein of the present invention used in the screening method (a-1) above can be isolated and purified by using the above-mentioned method of producing the protein of the present invention or its partial peptide.

The cell capable of producing the protein of the present invention used in the screening method (a-2) above is not particularly limited, as long as it is a human or other warm-blooded animal cell naturally expressing the protein, or a biological specimen (e.g., blood, tissue, organ, etc.) containing thereof. Examples include human breast cancer-cell line MCF7, T47D, etc. For the cells derived from non-human animal blood, tissue, organ, etc., these may be isolated from an organism and then cultured, or a test substance may be administered to an organism and then those biological specimen may be isolated after a given period of time.

As the cells capable of producing the protein of the present invention, various transformants prepared by the genetic engineering mentioned above can be exemplified. Preferably, animal cells such as COS7 cells, CHO cells, HEK293 cells, etc. are used as the host.

Examples of the test compound include proteins, peptides, non-peptide compounds, synthetic compounds, fermentation products, cell extracts, plant extracts, animal tissue extracts, and the like. These may be either a novel substance or well-known substance.

The activity of the protein of the present invention in the screening method of (a-1) above is assayed by, for example, bringing (i) the protein of the present invention or (ii) the protein of the present invention and a test compound, into contact with the warm-blooded animal cell in which the cell growth signal is activated by the protein of the present invention, and then assaying the cell growth or apoptosis induction in cells. The assay for the activity of the protein of the present invention in the screening method of (a-1) above can be carried out by assaying the cell growth or assaying the apoptosis induction in cells capable of producing the protein of the present invention.

When an apoptosis (cell death) is used as an indicator, the induction level of apoptosis (cell death) is assessed after completing the following processes of suspending a cell in a suitable medium culture or buffer solution, adding a test compound (or not be added) thereto, stimulating the cells with oxidation stress (e.g., H₂O₂ addition) or the like, and then incubating the obtained solution normally at about 20 to 40 °C and preferably about 30 to 37 °C for about 6 to 72 hours.

Apoptosis (cell death) can be examined by detecting membrane blebbing, reduction of cell size, chromatin condensation, DNA fragmentation, etc., with the morphological observation of using, for example, optical microscope, phase contrast microscope, fluorescence microscope, etc. For example, cells are incubated in multi-well plates, the abraded cells from the plates or membrane blebbed cells were counted using a microscope, and ratio (%) of a dead cell in a total cell in the visual field, is calculated. The cells were observed in several fields and average number of those observed was obtained as a cell death induction ratio. In addition, the cell death induction ratio can be derived by staining the dead cells using a dye such as trypan blue, erythrosine B, Negrosin, eosine Y, fluorescein diacetate, acridine orange, ethidium bromide, etc. , and then counting the number of cells using a microscope. The cells may also be counted by staining DNA using fluorescent dye of DAPI, Hoechst 33342 etc., and then counting the cells in which chromatin is condensated using a microscope. Alternatively, the apoptosis induction ratio can be assayed by adding dUTP which is labelled with biotin or fluorescent dye with the use of terminal deoxynucleotidyl transferase (TdT), to 3' end of fragmented DNA (TUNEL method), and counting the number of intensely stained cells by using a light microscope, fluorescence microscope, or the like.

The apoptosis induction ratio can be assayed by counting the number of cells reduced in size or fragmented by assessing the cell size distribution with the use of a particle-size measuring apparatus (e.g., Coulter multisizer). The cell death induction ratio can also be assayed by identifying the alive/dead cells by detecting the reduction in cell size/fragmentation into apoptotic body by using flow cytometry (FACS).

Further, apoptosis can be biochemically detected by extracting chromosomal DNA from a cell using a conventional method, and carrying out a gel electrophoresis to measure the level of DNA fragmentation using a densitometer or the like. The cell death induction ratio can be assayed by measuring the absorbance decrease in 570 to 630nm using a microplate reader as 3-(4,5-dimethyl-thiazol-2-yl)-2,5-diphenyl tetrazolium bromide (MTT) is reduced to formazan by alive cells.

For example, in the screening method (a-1) above, when the activity of the protein of the present invention in the presence of test compound is inhibited about 20% or more, preferably 30% or more, and more preferably about 50% or more as compared to the activity of the protein in the absence of a test compound, the test compound can be selected as a substance which inhibits the activity of the protein of the present invention.

As stated above, the substance inhibiting the expression of the protein of the present invention (Desmocollin-3, TM4SF6 and LY-6k) promotes apoptosis in cancer cells and/or inhibits growth of cancer cells, thereby is useful for preventive/remedy for cancer. The substance controlling (promotion or inhibition) the expression of the TM4SF13 protein also promotes apoptosis in cancer cells and/or inhibits growth of cancer cells, thereby is useful for preventive/remedy for cancer.

In other words, the present invention also provides a method of screening for a substance that promotes apoptosis in cancer cells and/or inhibits growth of cancer cells, and a substance for preventive/remedy for cancer, in which the method is characterized in that the expression of a protein in cells capable of producing the protein of the present invention is compared in the presence of and absence of test compound.

The expression level of the protein of the present invention can be assayed at a transcriptional level by detecting mRNA thereof with the use of polynucleotide hybridizable to polynucleotide encoding the protein of the present invention under high stringent conditions (that is, polynucleotide containing a base sequence encoding the protein of the present invention above mentioned or a part of the base sequence (also referred to as 'sense polynucleotide of the present invention') or the antisense polynucleotide of the present invention). Alternatively, the expression level can be assayed at a translational level by detecting the protein of the present invention with the use of an antibody of the present invention mentioned above.

Accordingly, the present invention more specifically provides:
(b) a method of screening for a substance that promotes apoptosis in cancer cells and/or inhibits growth of cancer cells, and a substance for preventive/remedy for cancer, in which the method is characterized in that a cell capable of producing the protein of the present invention is cultured in the presence of and absence of test compound, and the amount of mRNA encoding the protein of the present invention obtained under two different conditions are measured and compared using sense or antisense polynucleotide of the present invention; and,
(c) a method of screening for a substance that promotes apoptosis in cancer cells and/or inhibits growth of cancer cells, and a substance for preventive/remedy for cancer, in which the method is characterized in that a cell capable of producing the protein of the present invention is cultured in the presence of and absence of test compound, and the amount of protein of the present invention obtained under two different conditions are measured and compared using an antibody of the present invention.

In the screening method according to (b) and (c) above, same cells used for the screening method (a-2) above can be preferably used as a cell capable of producing the protein of the present invention.

For example, a quantification of mRNA of the protein of the present invention or the protein can be specifically carried out as the following:
(i) normal or disease model non-human warm-blooded animal (for example, mouse, rat, rabbit, sheep, swine, bovine, feline, canine, simian, bird, etc.) is administered with a medicine (for example, TNF-α, interleukin-1, Fas, anticancer agent) or applied with a physicochemical stress (for example, UV, active oxygen, ischaemia), and after a given period of time, blood, particular organs (for example, a brain, a liver, a kidney), or tissues or cells isolated from organs are obtained.
   mRNA of the protein of the present invention contained in the obtained cell can be for example, quantified by a process comprising extracting mRNA from cells etc. according to normally used method and by means of, for example, RT-PCR, or also can be quantified by carrying out a Northern blot analysis known per se. On the other hand, the protein of the present invention can be quantified by a Western blot analysis or by a various immunoassay method described below in detail.
(ii) A transformant to which polynucleotide encoding the protein of the present invention is introduced is prepared according to above-mentioned method, and the protein of the present invention or mRNA encoding the protein included in the transformant can be quantified and analyzed in the same manner as in the (i) above.

A screening for a substance which modifies the expression amount of the protein of the present invention can be carried out as the following:
(i) amount of mRNA which encodes the protein of the present invention or the amount of protein of the present invention included in the cell isolated from an animal is quantified and analyzed, a given time of period after (30 minutes to 3 days after, preferably 1 hour to 2 days after, more preferably 1 to 24 hours after) of administering a test compound to the normal or disease model non-human warm-blooded animal before (30 minutes to 24 hours before, preferably 30 minutes to 12 hours before, more preferably 1 to 6 hours before) or after (30 minutes to 3 days after, preferably 1 hours to 2 days after, more preferably 1 to 24 hours after) a given time of period for applying a medicine or physiochemical stress, or administering them concurrently with the medicine or physiochemical stress to the animal; or
(ii) amount of mRNA of the protein of the present invention contained or the amount of protein of the present invention in a transformant is quantified and analyzed, after adding a test compound to a medium culture when culturing the transformant according to an ordinary method and incubating for a given period of time (1 to 7 days after, preferably 1 to 3 days after, more preferably 2 to 3 days after).

Examples of the test compound include peptides, proteins, non-peptide compounds, synthetic compounds, fermentation products, etc., and these compounds may be novel compounds or publicly known compounds.

Quantification for the protein of the present invention in the screening method (c) above specifically includes, for example
(i) a method comprising competitively reacting the antibody of the present invention, a sample fluid, and a labeled form of the protein of the present invention, and determining the labeled protein of the present invention that binds to the antibody, thereby to quantify the protein of the present invention in the sample fluid; and
(ii) a method comprising simultaneously or continuously reacting a sample fluid, the immobilized antibody of the present invention on a carrier, and a labeled form of another antibody of the present invention, and measuring the amount (activity) of the label on the immobilizing carrier, thereby to quantify the protein of the present invention in the sample fluid.

In the quantification method of (ii) above, two species of antibodies are desirably the ones that each recognizes the different part in the protein of the present invention. For example, when one antibody recognizes the N-terminal region of the protein of the present invention, another antibody reacting with the C-terminal region of the protein of the present invention is used.

Examples of labeling agents, which are employed for the aforesaid measuring methods using labeling agents, are radioisotopes, enzymes, fluorescent substances, luminescent substances, etc. Examples of radioisotopes are [¹²⁵I], [¹³¹I], [³H], [¹⁴C] , etc. Preferred examples of the enzymes are those that are stable and have a higher specific activity, which include β-galactosidase, β-glucosidase, alkaline phosphatase, peroxidase, malate dehydrogenase, etc. Examples of the fluorescent substances include fluorescamine, fluorescein isothiocyanate, etc. Examples of the luminescent substances are luminol, a luminol derivative, luciferin, lucigenin, etc. Furthermore, a biotin-(strepto)avidin system may be used as well for binding of an antibody or antigen to a labeling agent.

As the sample fluid, when the protein of the present invention is localized in a cell, a cell homogenate obtained by suspending cells in an appropriate buffer, and then breaking cells by ultrasonication, freeze-thaw cycling, etc., is used, and when the protein of the present invention is secreted outside the cell, cell culture supernatant is used. If necessary, the quantification can be carried out after separating and purifying the protein of the present invention from a homogenate or a cell-culture supernatant. In addition, an intact cell can be used as a specimen as long as the label detection is possible.

The quantification method of the protein of the present invention using the antibody of the present invention is not particularly limited. Any quantification method may be used, so long as the amount of an antibody, antigen or antibody-antigen complex corresponding to the amount of antigen in a sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is preferred to use, for example, the sandwich method described later.

In the immobilization of antigens or antibodies, physical adsorption may be used. Alternatively, chemical binding that is conventionally used for immobilization/stabilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

In the sandwich method, the immobilized antibody of the present invention is reacted with a sample fluid (primary reaction), then with a labeled form of another antibody of the present invention (secondary reaction), and the amount or activity of the label on the immobilizing carrier is measured, whereby the amount of the protein of the present invention in the sample fluid can be quantified. The order of the primary and secondary reactions may be reversed, and the reactions may be performed simultaneously or at staggered times. The methods of labeling and immobilization can be performed by the methods described above. In the immunoassay by the sandwich method, the antibody used for immobilized or labeled antibodies is not necessarily one species, but a mixture of two or more species of antibody may be used, for example, to increase the measurement sensitivity.

The antibody of the present invention can also be used in measuring system other than the sandwich method such as in the competitive method, the immunometric method, nephrometry, etc.

In the competitive assay, the protein of the present invention in a sample fluid and a labeled form of the protein of the present invention are reacted competitively against an antibody, an unreacted labeled antigen (F) is separated from an antibody-bound labeled antigen (B) (B/F separation) , and the labeled amount of B or F is determined, thereby to quantify the protein of the present invention in the sample fluid. The present reaction method includes a liquid phase method in which the B/F separation is carried out by using a soluble antibody as an antibody and using polyethylene glycol or a secondary antibody against the antibody (primary antibody) etc.; and a solid phase method in which a solid-phased antibody is used as a primary antibody (direct method) or a soluble antibody is used as a primary antibody and a solid-phased antibody is used as a secondary antibody (indirect method).

In the immunometric assay, the protein of the present invention in a sample fluid and a solid phased protein of the present invention are competitively reacted with a given amount of a labeled form of the antibody followed by separating the solid phase from the liquid phase; or an protein of the present invention in a sample fluid and an excess amount of labeled form of the antibody are reacted, then a solid phased protein of the present invention is added to allow an unreacted labeled form of the antibody to bind to the solid phase, and the solid phase is then separated from the liquid phase. Thereafter, the labeled amount in any of the phases is measured to determine the antigen level in the sample fluid.

In the nephrometry, the amount of insoluble sediment, which is produced as a result of the antigen-antibody reaction in a gel or in a solution, is measured. Even when the amount of a protein of the present invention in a sample fluid is small and only a small amount of the sediment is obtained, a laser nephrometry utilizing laser scattering and the like can be suitably used.

For applying each of these immunological methods to the quantification method of the present invention, any setting of particular conditions or procedures is not required. The system for assaying the protein of the present invention may be established by applying the usual technical concern of those skilled in the art, in addition to the usual conditions and operating method for the respective methods. For the details of these general technical means, reference can be made to the following reviews and texts.

For example, see, Hiroshi Irie, ed. "Radioimmunoassay" (Kodansha, published in 1974), Hiroshi Irie, ed. "Sequel to the Radioimmunoassay" (Kodansha, published in 1979) , Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987) , Methods in ENZYMOLOGY, Vol. 70 (Immunochemical Techniques (Part A)), ibid., Vol. 73 (Immunochemical Techniques (PartB)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.

In the above manner, the production amount of protein of the present invention in a cell can be quantified with a good sensitivity by using the antibody of the present invention.

For example, in the screening method of (b) and (c) , when the expression amount (the amount of mRNA or protein) of the protein of the present invention (Desmocollin-3, TM4SF6 and LY-6K) in the presence of test compound is inhibited by about 20% or more, preferably 30% or more, and more preferably about 50% or more, as compared to the expression amount in the absence of a test compound, the test compound can be selected as a substance inhibiting the expression of the protein of the present invention.

As described above, the substance controlling (promotion or inhibition) the expression of the TM4SF13 protein and/or the interaction of the protein and integrin also promotes apoptosis in cancer cells and/or inhibits growth of cancer cells, thereby is useful for preventive/remedy for cancer.

The screening for substance controlling the expression of the TM4SF13 protein can be carried out in a similar way as the screening for the substance inhibiting the expression of the other proteins of the present invention described above. In the screening method, when the expression amount (amount of mRNA or amount of protein) of the protein of the present invention (TM4SF13) in the presence of test compound is inhibited or increased by about 20% or more, preferably 30% or more, or more preferably about 50% or more, as compared to the expression amount in the absence of the test compound, the test compound can be selected as a substance controlling the expression of the protein of the present invention.

The present invention also provides a method of screening for a substance for promoting the apotosis in cancer cells and/or inhibiting the growth of cancer cells, and a preventive/remedy substance for cancer, the method is characterized by comparing adverse interaction of TM4SF13 protein with integrin in the presence and absence of the test compound.

The interaction of the TM4SF13 protein of the present invention and integrin is detected and measured by a generally used assays of immunoprecipitation, ELISA (Enzyme-Linked Immunosorbent Assay), two-hybrid method, FACS (Fluorescence activated cell sorting), SPR (Surface Plasma Resonance), fluorescent imaging according to FRET (Fluorescence Resonance Energy Transfer), DPI (Dual Polarization Interferometry) and methods based thereon etc., but the method is not limited thereto, and it may be any methods capable of detecting a protein-protein interaction. Also, a method of detecting the interaction on the basis of the effect given to a function of integrin led by the interaction of TM4SF13 protein with integrin, may also be used.

Accordingly, the present invention more specifically provides,
(d) a method of screening for a substance that promotes apoptosis in cancer cells and/or inhibits growth of cancer cells and a preventive/remedy substance for cancer, in which the method is characterized in that a TM4SF13 protein and integrin were kept in the presence and absence of test compound and then the level of interaction of the TM4SF13 protein with integrin under two different conditions is measured and compared by using any of the methods described above.

For example, a measurement of the interaction of the TM4SF13 protein of the present invention and integrin can be carried out as described below specifically.
(i) a test compound is added in a mixture of tagged TM4SF13 and integrin, a product immunoprecipitated with an anti-integrin antibody is signal detected using an labeled anti-tag antibody, and the interaction of the protein with integrin is quantified and analyzed. Hereat, a protein to be tagged may be integrin.
(ii) the antibody for TM4SF13 is adsorbed to a solid-phase, a mixture containing the TM4SF13 protein, integrin, and a test compound is added thereto, and the interaction of TM4SF13 protein with integrin is quantified and analyzed using ELISA assay which detects with an antibody (labeled antibody) for integrin. Hereat, roles of the antibodies for the TM4SF13 and integrin may be reversed to one another.
(iii) in yeast two-hybrid system, in which the TM4SF13 protein and integrin are used either as bait or prey, the interaction of the TM4SF13 protein and integrin is quantified and analyzed in the presence of a test compound.
(vi) in the presence of a test compound, a cell expressing the TM4SF13 or integrin is mixed with respectively labeled integrin or TM4SF13, a labeled cell is detected using FACS, and the interaction of the protein and integrin is quantified and analyzed. Or,
(v) in the presence of a test compound, the TM4SF13 protein or integrinit is adsorbed to a metal surface, and the interaction of the protein and integrin is quantified and analyzed using SPR.

In addition, such measurement can be performed by quantifying and analyzing the effect of the test compound on a function of integrin (e.g., signal transmission, binding to an ECM component).

Examples of the test compound include peptides, proteins, non-peptide compounds, synthetic compounds, fermentation products and the like. These may be either a novel substance or well-known substance.

Examples of labeling agents, which are employed for the aforesaid measuring methods using labeling agents, are radioisotopes, enzymes, fluorescent substances, luminescent substances, etc. Examples of radioisotopes are [¹²⁵I], [¹³¹I] , [³H], [¹⁴C], etc. Preferred examples of the enzymes are those that are stable and have a higher specific activity, which include β-galactosidase, β-glucosidase, alkaline phosphatase, peroxidase, malate dehydrogenase, etc. Examples of the fluorescent substances include fluorescamine, fluorescein isothiocyanate, etc. Examples of the luminescent substances are luminol, a luminol derivative, luciferin, lucigenin, etc. Furthermore, a biotin-(strepto)avidin system may be used as well for binding of an antibody or antigen to a labeling agent.

When the individual methods are applied to the present invention, setting of special conditions, operation, and the like are not required. A measurement system for protein-protein interaction may be established by applying a usual technical concern from those skilled in the art, in addition to usual conditions and operation method for the respective methods. For details about these general technical means, general remarks, books or the like can be used for reference.

As described above, the interaction of the TM4SF13 protein and integrin can be quantified with good sensitivity.

For example, in the (d) screening method, when the interaction of TM4SF13 protein and integrin in the presence of test compound is inhibited or increased by about 20% or more, preferably about 30% or more, and more preferably about 50% or more, as compared to the interaction of TM4SF13 protein and integrin in the absence of test compound, the test compound can be selected as a substance controlling the interaction of TM4SF13 protein and integrin.

The screening kit of the present invention comprises the protein of the present invention or its partial peptide (hereinafter, merely referred to as 'protein of the present invention'). The protein of the present invention may be isolated and purified by any method described above, or may be provided in the form of a cell (warm-blooded animal cell) capable of producing the protein or partial peptide of the invention as above mentioned.

The screening kit of the present invention can further comprise the antibody, or the sense or antisense polynucleotide of the present invention mentioned above, thereby measures the expression amount of the protein in cells capable of producing the protein of the present invention.

The screening kit can optionally comprise reaction buffer, blocking buffer, washing buffer, labeling reagent, labeling detection reagent, and the like, in addition to the above mentioned agents.

The substance (which may be a free from or a salt form) that inhibits the expression and/or activity of the protein of the present invention, which can be obtained by the screening method or the screening kit of the invention, is useful as a safe and low toxic medicament such as agents for promoting apoptosis in cancer cells, agents for inhibiting cancer cell growth or a preventive/remedy agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably as preventive/remedy agents for breast cancer, lung cancer, colon cancer, prostate cancer, ovary cancer, pancreatic cancer, etc.).

Where the compound or its salts obtained by using the screening method or screening kit of the present invention is used as the agents for preventive/remedy described above, the compound and the salts can be converted into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a carrier, diluent, or excipient conventionally used in the field of pharmaceutical preparations. Examples of the carrier or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous, and intramuscular injections, drip infusions, intraarticular injections, etc. These injectable preparations may be prepared by methods publicly known, for example, by dissolving, suspending, or emulsifying the compounds or their salts described above in a sterile aqueous medium or oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the compound or its salt described above with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; and it is preferably 5 to 100 mg especially in the form of injection, and 10 to 250 mg for the other forms.

Each composition described above may further contain other active components unless they cause any adverse interaction with the compound described above due to blending.

Since the pharmaceutical preparation thus obtained is safe and low toxic, it can be administered orally or parenterally to human or a warm-blooded animal (e.g., mouse, rat, rabbit, sheep, swine, bovine, horse, bird, feline, canine, simian, chimpanzee, etc.).

The dose of the compound or its salt may vary depending on its effect, target disease, subject to be administered, route of administration, etc. For example, when the substance that inhibits the expression and/or activity of the protein of the present invention is orally administered for the purpose of treating breast cancer, the compound or its salt is administered to the adult (as 60 kg body weight) generally in a daily dose of about 0.1 to 100 mg, preferably about 1.0 to 50 mg, and more preferably about 1.0 to 20 mg. In parenteral administration, a single dose of the inhibition substance may vary depending on a subject to be administered, target disease, etc. When the substance that inhibits the expression and/or the activity of the protein of the present invention is generally administered in the form of an injectable preparation to the adult (as 60 kg body weight) for the purpose of treating breast cancer, it is advantageous to administer the compound or its salt in a single dose of about 0.01 to about 30 mg, preferably about 0.1 to about 20 mg, and more preferably about 0.1 to about 10 mg a day, by injection to cancer lesion. For other animal species, the corresponding dose as converted per 60 kg weight can be administered.

### (4) Diagnostic agent for cancer

The antibody of the present invention can specifically recognize the protein of the present invention, thus can be used for quantifying the protein of the present invention in a test fluid. Thus, in the method of screening for the substance that inhibits expression of the protein of the present invention in which the antibody of the invention mentioned above is used, an expression level of the protein of the present invention in animal can be examined by carrying out an immunoassay using a biological specimen (e.g., blood, plasma, urine, biopsy, etc.) collected from a test warm-blooded animal instead of using a cell capable of producing the protein of the present invention, thereby the method can be used for diagnosis of cancer. As a result of the immunoassay, when increase in protein of the present invention in the specimen is detected, it can be diagnosed that as to suffer from cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or have a risk to develop them in the future.

Similarly, by using the sense or antisense polynucleotide of the invention mentioned above as a probe or primer, an abnormality (gene abnormality) of the DNA or mRNA encoding the protein of the present invention in human or other warm-blooded animal (e.g., rat, mouse, hamster, rabbit, sheep, goat, swine, bovine, horse, feline, canine, simian, chimpanzee, bird, etc.) can be detected. Therefore, the polynucleotide is useful as a gene diagnostic agent for detecting amplification of DNA or overexpression of mRNA, etc., and particularly as a diagnostic agent for cancer. The polynucleotide encoding the protein of the present invention or corresponding antisense polynucleotide is not particularly limited as long as it has a length required as a probe or primer (for example, about 15 bases or more).

The above-described gene diagnosis in which the sense or antisense polynucleotide of the invention is used can be performed by, for example, the publicly known Northern hybridization, quantitative RT-PCR, PCR-SSCP assay, allele-specific PCR, PCR-SSOP assay, DGGE assay, RNase protection assay, PCR-RFLP assay, etc.

For example, as a result of Northern hybridization or quantitative RT-PCR for RNA fraction extracted from the cells of a warm-blooded animal, when increase expression of protein of the present invention is detected, it can be diagnosed as to suffer from cancer (e. g. , colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or have a risk to develop them in the future.

### (5) Pharmaceutical comprising the protein of the present invention

Since the protein of the present invention is overexpressed in cancer, the protein can be used as a cancer vaccine to activate the immune system in patients with cancer.

For example, the so-called adoptive immunotherapy, which involves culturing potent antigen presenting cells (e.g., dendritic cells) in the presence of protein of the present invention to allow them to engulf the protein and then putting the cells back into the body of a patient, can preferably be used. The dendritic cells, returned back into the body, can induce and activate cytotoxic T cells specific to a cancer antigen whereby to kill cancer cells.

The protein of the present invention can also be administered to a mammal (e. g. human, simian, mouse, rat, rabbit, swine) safely, for example, as a vaccine preparation to prevent or treat a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

The vaccine preparation usually contains the protein of the present invention and a physiologically acceptable carrier. Such carrier includes a liquid carrier such as water, saline (including physiological saline), buffer (e.g., phosphate buffer), alcohol (e.g., ethanol), etc.

The vaccine preparation can be prepared according to a conventional method of manufacturing a vaccine preparation.

In general, the protein of the present invention is dissolved or suspended in a physiologically acceptable carrier. Alternatively, the protein of the present invention and the physiologically acceptable carrier may be separately prepared and then mixed at use.

The vaccine preparation may be further formulated with, for example, an adjuvant (e.g., aluminum hydroxide gel, serum albumin, etc.), a preservative (e.g., thimerosal, etc.), a soothing agent (e.g., glucose, benzyl alcohol, etc.), in addition to the protein of the present invention and the physiologically acceptable carrier. Furthermore, the vaccine preparation may also be formulated with, for example, a cytokine (e.g., an interleukin such as interleukin-2, an interferon such as interferon-γ) to enhance the production of the antibody to the protein of the present invention.

When used as a vaccine preparation, the protein of the present invention may be used in its active form, or may be denatured to enhance the antigenicity. The protein of the present invention may be denatured usually by heating or treating with a protein-denaturing agent (e.g., formalin, guanidine hydrochloride, and urea).

The thus-obtained vaccine preparation is low toxic and may usually be administered in an injectable form, e.g., subcutaneously, intracutaneously, intramuscularly, or topically into or near a mass of cancer cells.

The dose of the protein of the present invention varies depending on a target disease, a subject to be administered, a route for administration, etc. For example, for subcutaneous administration of the protein of the present invention to an adult with cancer (60 kg body weight) in an injectable form, the single dose is normally about 0.1 mg to about 300 mg, preferably about 100 mg to about 300 mg. The administration of the vaccine preparation may be carried out once, or 2 to 4 times in total approximately in about every 2 weeks to 6 months to increase the production of the antibody.

Since the TM4SF13 can act suppressively against certain kind of cancer by interacting with integrin as described above, TM4SF13 protein or its partial peptide can be used as an agent for inducing apoptosis in cancer cells/inhibiting growth of the cancer cells, accordingly can be used as a preventive/remedy agent for cancer.

The TM4SF13 protein or its partial peptide is prepared into pharmaceutical preparations similarly to the antibody of the present invention mentioned above. The dose of TM4SF13 may vary depending upon subject to be administered, target disease, conditions, route of administration, etc. For example, when used for the purpose of treating/preventing, e.g., breast cancer in adult, it is advantageous to administer the protein in a dose of usually about 0.01 to about 20 mg/kg body weight, preferably about 0.1 to about 10 mg/kg body weight, and more preferably about 0.1 to about 5 mg/kg body weight, for about 1 to 5 times/day, preferably about 1 to 3 times/day, by intravenous injection. In other parenteral and oral administration, the agent can be administered in a dose corresponding to the dose given above. When the condition is especially severe, the dose may be increased according to the condition.

### (6) Pharmaceutical containing polynucleotide encoding TM4SF13

Similarly, the polynucleotide encoding the TM4SF13 or its partial peptide can be used also as agents for inducing apoptosis in cancer cells/inhibiting growth of cancer cells, thereby it can be used as a preventive/remedy agent for cancer. The polynucleotide can be administered in the form (for example, expression vector) that is under a control of an appropriate promoter (that is, a promoter having a promoter activity in a cell of subject to be administered) preferably. The polynucleotide can be prepared into pharmaceutical preparations similarly to the antisense polynucleotide of the present invention described above.

A dose of the polynucleotide encoding TM4SF13 or its partial peptide may vary depending on target disease, subject to be administered, route for administration, etc. For example, where the polynucleotide encoding TM4SF13 or its partial peptide is administered for the purpose of treating breast cancer, the polynucleotide encoding TM4SF13 or its partial peptide is generally administered to an adult (60 kg body weight) in a daily dose of about 0.1 to 100 mg.

### (7) DNA transgenic animal

The present invention provides a non-human mammal bearing DNA encoding the exogenous protein of the present invention (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1) , wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase) , by standard means, such as the calcium phosphate method, the electric pulse method, the lipofection method, the agglutination method, the microinjection method, the particle gun method, the DEAE-dextran method, etc. Also, it is possible to transfect the desired exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, canine, feline, guinea pig, hamsters, mice, rats, etc. Above all, preferred are rodents, especially mice (e.g., C57BL/6 strain, DBA2 strain, etc. for a pure line and for a cross line, B6C3F₁ strain, BDF₁ strain, B6D2F₁ strain, BALB/c strain, ICR strain, etc.), rats (Wistar, SD, etc.) or the like, since they are relatively short in ontogeny and life cycle and easy to breed, from a standpoint of creating model animals for disease.

"Mammals" in a recombinant vector that can be expressed in the mammals include the aforesaid non-human mammals, human, etc.

The exogenous DNA of the present invention refers to the DNA of the present invention that is once isolated and extracted from mammals, not the DNA of the present invention inherently possessed by the non-human mammals.

The variant DNA of the present invention includes those resulting from variation (e.g., mutation, etc.) in the base sequence of the original DNA of the present invention, specifically DNAs resulting from base addition, deletion, substitution with other bases, etc. and also includes abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention downstream various promoters which are capable of expressing the DNA derived from various mammals (e. g. , rabbits, canine, feline, guinea pigs, hamsters, rats, mice, etc.) bearing the DNA of the present invention highly homologous to the human DNA, into a fertilized egg of the target non-human mammal (e.g., mouse fertilized egg).

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters of DNA derived from viruses (e.g., simian virus, cytomegalovirus, Moloney leukemia virus, JC virus, mammary tumor virus, poliovirus, etc. ), and (ii) promoters derived from various mammals (human, rabbits, canine, feline, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylation enzyme (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin basic protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide chain elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and preferably SV40 terminator of the simian virus and the like are used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained as the entire genomic DNA or its portion from DNA of liver, kidney, thyroid cell or fibroblast origin from human or various mammals (e.g., rabbits, canine, feline, guinea pigs, hamsters, rats, mice, etc.) and of various commercially available genomic DNA libraries, or obtained by using cDNA prepared by a publicly known method from RNA of liver, kidney, thyroid cell or fibroblast origin as a starting material. Also, an exogenous abnormal DNA can be produced as the mutant translational region through mutation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells of the target mammal. The fact that the exogenous DNA of the present invention is excessively present in the germinal cells of the prepared animal after transfection means that the exogenous DNA of the present invention is excessively present in all of the germinal cells and somatic cells of all offspring of the prepared animal. The offspring of the animal of the species that inherits the exogenous DNA of the present invention have excessively the exogenous DNA of the present invention in all of the germinal cells and somatic cells thereof.

It is possible to obtain homozygous animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits a symptom of the increased protein of the present invention liberated, the animal is usable for screening test of agents for preventing/treating diseases associated with the protein of the present invention, for example, agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the prepared animal after DNA transfection means that all of the offspring of the prepared animal have the abnormal DNA of the present invention in all of the germinal and somatic cells. Such an offspring that inherits the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the pathological mechanism of the function inactive type inadaptability to the protein of the present invention and to investigate how to treat the disease.

More specifically, the animal expressing the abnormal DNA of the present invention at a high level is expected to serve as a model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows a symptom of the increased protein of the present invention liberated, the animal can be also expected to serve for screening test of agents preventing/treating the function inactive type inadaptability of the protein of the present invention, for example, preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

Other potential applications of two kinds of the DNA transgenic animals of the present invention described above further include, for example:
(i) Use as a cell source for tissue culture;
(ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(iii) Research on the function of cells derived from tissues that are usually cultured only with difficulty, using cells in tissues bearing the DNA cultured by a standard tissue culture technique;
(iv) Screening for a drug that enhances the functions of cells using the cells described in (iii) above; and,
(v) Isolation and purification of the variant protein of the present invention and preparation of an antibody thereto.

Furthermore, clinical conditions of a disease associated wit the protein of the present invention, including the function inactive type inadaptability to the protein of the present invention can be determined by using the DNA transgenic animal of the present invention. Also, pathological findings on each organ in a model of a disease associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., and to culture the cells or to establish the line of cultured cells. Furthermore, the DNA transgenic animal of the present invention can serve to identify cells capable of producing the protein of the present invention, and to study the association with apoptosis, differentiation or propagation or the mechanism of signal transduction in these properties to inspect any abnormality thereof. Thus, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (8) Knockout animal

The present invention provides a non-human mammal embryonic stem cell bearing the DNA of the present invention inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from *Escherichia coli*);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e. g. , β-galactosidase gene derived from *Escherichia coli)* therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening for a compound that promotes or inhibits the promoter activity to the DNA of the present invention, or salt thereof, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention carried by the mammal, or in which the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these mutations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, introducing a DNA strand having a DNA sequence constructed by isolating the DNA of the present invention that the desired non-human mammal possesses, inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or constructed by inserting a DNA sequence that terminates gene transcription (e.g., polyA additional signal, etc.), in the intron between exons, thus inhibiting the synthesis of complete messenger RNA and eventually destroying the gene (hereinafter simply referred to as a targeting vector), to a chromosome of the target animal by, e.g., homologous recombination. The thus-obtained ES cells are subjected to the southern hybridization analysis with a DNA sequence on or near the DNA of the present invention as a probe, or to PCR analysis with a DNA sequence on the targeting vector and another DNA sequence near the DNA of the present invention which is not used in preparing the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman. For example, in the case of mouse ES cells, currently it is common practice to use ES cells of the 129 strain. However, since their immunological background is obscure, for example, those established from the C57BL/6 mouse or the BDF₁ mouse (F₁ between C57BL/ 6 and DBA/2 ) wherein the low ovum availability of the C57BL/6 mouse has been improved by crossing with DBA/2, may be preferably used, for the purpose of obtaining a pure line of ES cells with the clear immunological genetic background instead of above strain and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocysts at 3.5 days after fertilization are commonly used. Besides, embryos are preferably collected at the 8-cell stage and cultured until the blastocyst stage, and then the embryos can be used to efficiently obtain a large number of early stage embryos.

Although the ES cells used may be of either sex, male ES cells are generally more convenient for generation of a germ cell line chimera. It is also desirable that sexes are identified as soon as possible to save painstaking culture procedure.

Methods for sex identification of the ES cell include the method in which a gene in the sex-determining region on the Y-chromosome is amplified by the PCR process and detected, as an example. When this method is used, about one colony of ES cells (about 50 cells) is sufficient for sex-determination analysis, while conventional karyotype analysis requires about 10⁶ cells; therefore, the first selection of ES cells at the early stage of culture can be based on sex identification, and male cells can be selected early, which saves a significant amount of labor at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained usually shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that when cells are observed at the passage and found to be morphologically abnormal in culture, the cell culture is abandoned.

Where ES cells are allowed to reach a high density in mono-layers or to form cell aggregates in suspension under appropriate conditions, it is possible to differentiate the ES cells to various cell types, for example, parietal and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology and Experimental Morphology, 87, 27, 1985]. The cells deficient in expression of the DNA of the present invention, which are obtained by differentiating ES cells of the present invention, are useful for cytologically studying the protein of the present invention in vitro.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse egg cells, and conducting homologous recombination in which a targeting vector DNA sequence, wherein the DNA of the present invention is inactivated by the transfection, is replaced with the DNA of the present invention on a chromosome of a mouse embryonic stem cell or mouse egg cell.

The knockout cells with the disrupted DNA of the present invention can be identified by the southern hybridization analysis using as a probe a DNA sequence on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting cells are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a group of individuals obtained by crossing between such a chimeric individual and a normal individual, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an egg cell is used, a DNA solution may be injected, e.g. , into the nucleus of the egg cell by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout can be passaged and reared under ordinary rearing conditions, after the DNA of the individuals obtained by their crossing have also proven to have been knockout.

Furthermore, the germ line may be obtained and retained by conventional methods. That is, by crossing male and female animals each having the inactivated DNA, homozygous animals having the inactivated DNA in both homologous chromosomes can be obtained. The homozygous animals thus obtained may be reared so that one normal animal and plural homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal deficient in expression of the DNA of the present invention lacks various biological activities derived from the protein of the present invention, such an animal can be a model of a disease due to inactivated biological activities of the protein of the present invention and thus, is useful to investigate the causes for and therapy for these diseases.

### (9a) Method of screening for the compound having a therapeutic/prophylactic effect on diseases caused by deficiency, damages, etc. of the DNA of the present invention

The non-human mammal deficient in expression of the DNA of the present invention can be employed for screening for the compound having a therapeutic/prophylactic effect on diseases caused by deficiency, damages, etc. of the DNA of the present invention.

That is, the present invention provides a method of screening for the compound having a therapeutic/prophylactic effect on diseases, e.g., cancer, caused by deficiency, damages, etc. of the DNA of the present invention, or salt thereof, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control, and a change in each organ, tissue, disease conditions, etc. of the animal is used as an indicator to assess the therapeutic/prophylactic effects of the test compound.

For treating a test animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the way of administration, nature of the test compound, etc.

For screening for the compound having a therapeutic/prophylactic effect on a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), a test compound is administered to the non-human mammal deficient in expression of the DNA of the present invention. Differences in incidence of cancer or differences in degree of healing of cancer from the group administered with no test compound are observed in the tissues described above over time.

In the screening method, when a test compound is administered to a test animal, the test compound can be selected as a compound having therapeutic/prophylactic effects on the above-mentioned diseases, if the symptoms described above in the animal are relieved by about 10% or more, preferably about 30% or more, and more preferably about 50% or more.

Since a compound obtained using the screening method is a compound selected from the above test compounds, and have therapeutic/prophylactic effects on diseases caused by deficiency, damage, etc. of the protein of the present invention, it can be used as a safe and low toxic medicament such as a prophylactic/therapeutic agent for the disease. Further, a compound derived from the compound that is obtained from the above-mentioned screening can also be used as well.

The compound obtained by the screening method above may form salts, and may be used in the form of salts with physiologically acceptable acids (e.g., inorganic acids, organic acids, etc.) or bases (e.g., alkali metals, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or a mammal (e.g., rat, mouse, guinea pig, rabbit, sheep, swine, bovine, horse, feline, canine, simian, etc.).

The dose of the compound or its salt may vary depending upon target disease, subject to be administered, route of administration, etc. For example, when the compound is orally administered, the compound is administered to the adult patient with breast cancer (as 60 kg body weight) generally in a daily dose of about 0.1 to 100 mg, preferably about 1.0 to 50 mg and more preferably about 1.0 to 20 mg. In parenteral administration, a single dose of the compound may vary depending upon subject to be administered, target disease, etc. For example, when the compound is administered to the general adult patient with breast cancer (as 60 kg body weight) in the form of an injectable preparation, it is advantageous to administer the compound in a dose of about 0.01 to about 30 mg, preferably about 0.1 to about 20 mg and more preferably about 0.1 to about 10 mg a day, by intravenous injection. For other animal species, the corresponding dose as converted per 60 kg weight can be administered.

### (9b) Method of screening for a compound that promotes or inhibits the activity of a promoter to the DNA of the present invention

The present invention provides a method of screening for a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, among the aforesaid non-human mammals deficient in expression of the DNA of the present invention.

The same examples of the test compound apply to specific compounds described above.

As the reporter gene, the same specific examples apply to this screening method. Preferably, there are used β-galactosidase gene (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the sample is reacted with a staining solution containing X-gal at room temperature or about 37 °C for approximately 30 minutes to an hour. After the β-galactosidase reaction is terminated by washing the tissue preparation with 1 mM EDTA/PBS solution, the color development is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts that inhibit the promoter activity to the DNA of the present invention can inhibit the expression of the protein of the present invention to inhibit the functions of the protein. Thus, the compound or its salts are useful as preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

In addition, compounds derived from the compound obtained by the screening described above may be used as well.

A pharmaceutical comprising the compound obtained by the above screening method or salts thereof can be manufactured in a manner similar to the method for preparing the pharmaceutical comprising the protein of the present invention described above or salt thereof.

Since the pharmaceutical preparation thus obtained is safe and low toxic, it can be administered to human or a mammal (e.g., rat, mouse, guinea pig, rabbit, sheep, swine, bovine, horse, feline, canine, simian, etc.).

A dose of the compound or salts thereof may vary depending on target disease, subject to be administered, route for administration, etc.; when the compound that inhibits the promoter activity to the DNA of the present invention is orally administered, the compound is administered to the adult patient (as 60 kg body weight) with breast cancer normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to the general adult patient (as 60 kg body weight) with breast cancer in the form of injectable preparation, it is advantageous to administer the compound intravenously by injection to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening for the compound or its salt that promotes or inhibits the promoter activity to the DNA of the present invention and, can greatly contribute to elucidation of causes for various diseases due to deficiency in expression of the DNA of the present invention or for the development of prophylactic/therapeutic agents for these diseases.

In addition, a so-called transgenic animal (gene transferred animal) can be prepared by using DNA containing the promoter region of the protein of the present invention, ligating genes encoding various proteins at downstream of the promoter and injecting the resultant into egg cell of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification, where bases, amino acids, etc. are denoted by their codes, they are expressed based on conventional codes in accordance with the IUPAC-IUB Commission on Biochemical Nomenclature or by the common codes in the art, examples of which are shown below. For amino acids that may have the optical isomer, L form is presented unless otherwise indicated.
DNA : deoxyribonucleic acid
cDNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
Gly : glycine
Ala : alanine
Val : valine
Leu : leucine
Ile : isoleucine
Ser : serine
Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gln : glutamine
pGlu : pyroglutamic acid
Sec : selenocysteine

Substituents, protecting groups and reagents frequently used in this specification are presented by the codes described below.
Me : methyl group
Et : ethyl group
Bu : butyl group
Ph : phenyl group
TC : thiazolidine-4(R)-carboxamido group
Tos : p-toluenesulfonyl
CHO : formyl
Bzl : benzyl
Cl₂-Bzl : 2,6-dichlorobenzyl
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl
DNP : dinitrophenyl
Trt : trityl
Bum : t-butoxymethyl
Fmoc : N-9-fluorenyl methoxycarbonyl
HOBt : 1-hydroxybenztriazole
HOOBt : 3,4-dihydro-3-hydroxy-4-oxo-1,2,3-benzotriazine
HONB : 1-hydroxy-5-norbornene-2,3-dicarboxyimide
DCC : N,N'-dicyclohexylcarbodiimide

The sequence identification numbers in the sequence listing of the specification indicate the following sequences.
[SEQ ID NO: 1]
   This shows the base sequence of DNA (CDS) encoding Desmocollin-3a.
[SEQ ID NO: 2]
   This shows the amino acid sequence of Desmocollin-3a.
[SEQ ID NO: 3]
   This shows the base sequence of DNA (CDS) encoding Desmocollin-3b.
[SEQ ID NO: 4]
   This shows the amino acid sequence of Desmocollin-3b.
[SEQ ID NO: 5]
   This shows the base sequence of DNA (CDS) encoding TM4SF13.
[SEQ ID NO: 6]
   This shows the amino acid sequence of TM4SF13.
[SEQ ID NO: 7]
   This shows the base sequence of DNA (CDS) encoding TM4SF6.
[SEQ ID NO: 8]
   This shows the amino acid sequence of TM4SF6.
[SEQ ID NO: 9]
   This shows the base sequence of DNA (CDS) encoding LY-6K.
[SEQ ID NO: 10]
   This shows the amino acid sequence of LY-6K.
[SEQ ID NO: 11]
   This shows the RNA sequence forming siRNA1a with SEQ ID NO: 12.
[SEQ ID NO: 12]
   This shows the RNA sequence forming siRNA1a with SEQ ID NO: 11.
[SEQ ID NO: 13]
   This shows the RNA sequence forming siRNA1b with SEQ ID NO: 14.
[SEQ ID NO: 14]
   This shows the RNA sequence forming siRNA1b with SEQ ID NO: 13.
[SEQ ID NO: 15]
   This shows the RNA sequence forming siRNA1c with SEQ ID NO: 16.
[SEQ ID NO: 16]
   This shows the RNA sequence forming siRNA1c with SEQ ID NO: 15.
[SEQ ID NO: 17]
   This shows the RNA sequence forming siRNA1d with SEQ ID NO: 18.
[SEQ ID NO: 18]
   This shows the RNA sequence forming siRNA1d with SEQ ID NO: 17.
[SEQ ID NO: 19]
   This shows the RNA sequence forming non-silensing dsRNA1 with SEQ ID NO: 20.
[SEQ ID NO: 20]
   This shows the RNA sequence forming non-silensing dsRNA1 with SEQ ID NO: 19.
[SEQ ID NO: 21]
   This shows the RNA sequence forming siRNA2a with SEQ ID NO: 22.
[SEQ ID NO: 22]
   This shows the RNA sequence forming siRNA2a with SEQ ID NO: 21.
[SEQ ID NO: 23]
   This shows the RNA sequence forming siRNA2b with SEQ ID NO: 24.
[SEQ ID NO: 24]
   This shows the RNA sequence forming siRNA2b with SEQ ID NO: 23.
[SEQ ID NO: 25]
   This shows the RNA sequence forming siRNA2c with SEQ ID NO: 26.
[SEQ ID NO: 26]
   This shows the RNA sequence forming siRNA2c with SEQ ID NO: 25.
[SEQ ID NO: 27]
   This shows the RNA sequence forming siRNA2d with SEQ ID NO: 28.
[SEQ ID NO: 28]
   This shows the RNA sequence forming siRNA2d with SEQ ID NO: 27.
[SEQ ID NO: 29]
   This shows the base sequence of the primer X.
[SEQ ID NO: 30]
   This shows the base sequence of the primer Y.
[SEQ ID NO: 31]
   This shows the base sequence of the primer Z.

### Examples

Hereinafter, the present invention will be described in more detail with reference to Examples and Reference Examples, but is not limited to the examples and reference examples.

### Example 1

### Overexpression of Desmocollin-3 in cancer

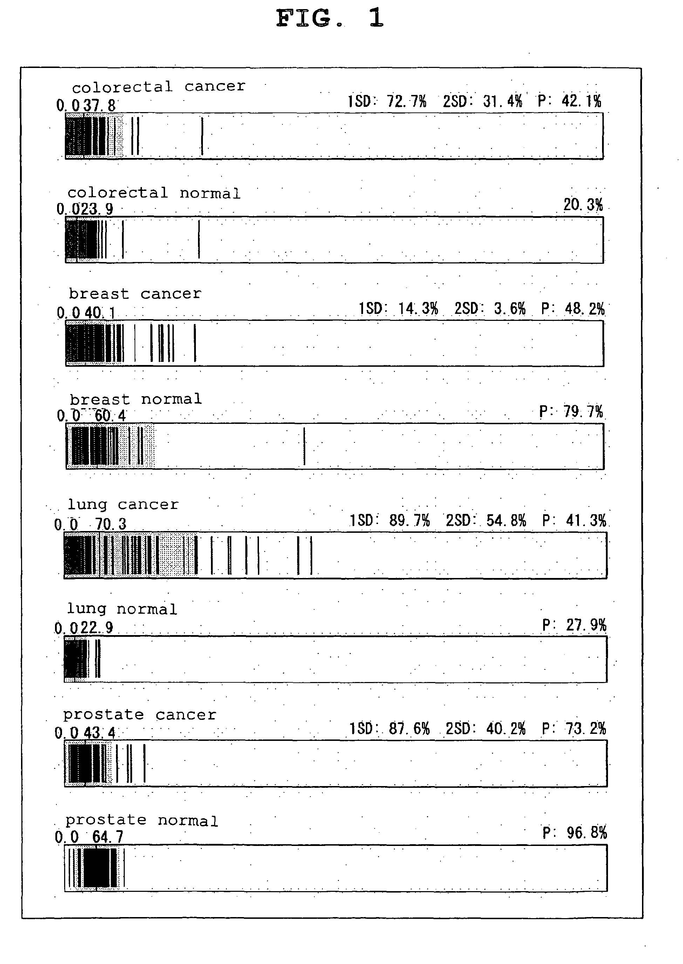

By carrying out the hybridization method using mRNA derived from cancer tissue and mRNA derived from peripheral normal tissue, which are all extracted from a cancer patient, a gene expression profile was prepared. As a result of analysis of the gene expression profile, it was found that the corresponding gene is expressed highly in the cancer tissue. Particularly, in lung cancer, remarkable overexpression was observed in comparison with the peripheral normal tissue (Fig. 1).

### Example 2

### Inhibition of cell growth in human lung cancer cell line by administering siRNA against Desmocollin-3 gene

Human lung cancer cell line NCI-H226 purchased from American Type Culture Collection (ATCC) was suspended in an RPMI1640 medium (Invitrogen) containing 10% fetal bovine serum (JRH), was seeded onto a 6-well flat-bottomed tissue culture plate (BD Falcon) with a cell density of 1×10⁵ cells/well, and was cultured in 5% CO₂ gas stream at 37°C overnight. Thereafter, siRNA was transfected.

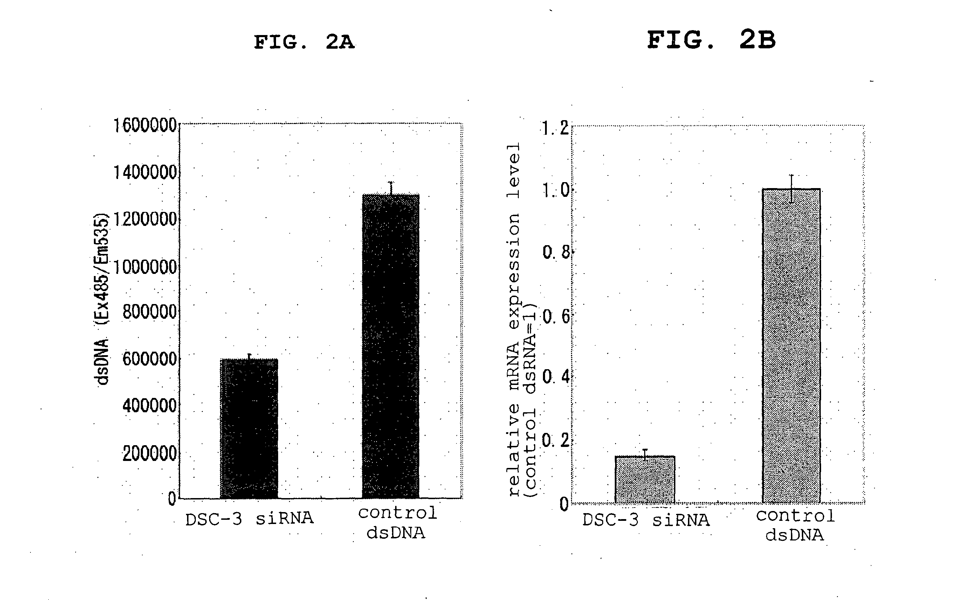

Specifically, the siRNA having an activity of cleaving mRNA of a Desmocollin-3a gene or a Desmocollin-3b gene was manufactured by hybridizing two kinds of RNA fragments (SEQ ID NO: 11 and SEQ ID NO: 12 (siRNA1a)). In the same way, three kinds of siRNAs were additionally manufactured by hybridizing two kinds of RNA fragments (SEQ ID NO: 13 and SEQ ID NO: 14 (siRNA1b), or SEQ ID NO: 15 and SEQ ID NO: 16 (siRNAlc), or SEQ ID NO: 17 and SEQ ID NO: 18 (siRNA1d)). Total four kinds of siRNAs were mixed by equal quantity and provided to transfection (hereinafter, abbreviated as siRNA1). As a control, two kinds of RNA fragments (SEQ ID NO: 19 and SEQ ID NO: 20) having no activity of cleaving mRNA in animal cells were hybridized in the same way for use (hereinafter abbreviated as non-silencing dsRNA1). 80 pmol of siRNA1 or 80 pmol of non-silencing dsRNA1 was added to 50 µL of Opti-MEM I (Invitrogen), was mixed with 50 µL of Opti-MEM I (Invitrogen) to which 4 µL of Lipofectamine 2000 (Invitrogen) was added, and then was allowed to stand at the room temperature for 20 minutes. The mixed solution was all added to a cell culture of NCI-H226 and the cells were further cultured for 24 hours, and then cells were collected. The collected cells were seeded onto 96-well flat-bottomed tissue culture plate with a cell density of 3,000 cells/well and were additionally cultured in the RPMI1640 medium (Invitrogen) containing 10% fetal bovine serum (JRH) in 5% CO₂ gas stream at 37°C for two days. After the medium was removed, the plate was left at rest at -80°C for 5 minutes and then was left at the room temperature for 5 minutes. An aqueous solution containing 1% PicoGreen (Molecular Probes) and 1% IGEPAL-CA630 (ICN Biomedicals) was added thereto by 100 µL/well and then the mixture was left for 20 minutes. Thereafter, by measuring fluorescence intensity thereof at an excitation wavelength of 485 nm and at an emission wavelength of 535 nm, the DNA content in cells was assayed. As a result, the cells administered with siRNA1 had the fluorescence intensity lowered by about 59% than that of the cells administered with non-silencing dsRNA1 and showed a statistically significant difference (P<0.001) (Fig. 2A).

### Example 3

### Decrease in expression level of mRNA of Desmocollin-3 gene by administering siRNA against Desmocollin-3 gene

The human lung cancer cell line NCI-H226 used in Example 2 was suspended in the RPMI1640 medium and was seeded onto a 24-well flat-bottomed tissue culture plate (BD Falcon) with a cell density of 1×10⁵ cells/well. The cells were cultured in 5% CO₂ gas stream at 37°C overnight and transfected with an siRNA in accordance with the method used in Example 2. After the transfection, the cells were continuously cultured for 24 hours and then total RNAs were extracted therefrom by the use of RNeasy Mini Total RNA Kit (QIAGEN). Using as a template about 100 ng of the total RNA, reverse transcription reaction was carried out with TaqMan Reverse Transcription Reagents (Applied Biosystems Inc.) in accordance with the protocol attached thereto. Using as a template cDNA in an amount corresponding to 10 ng when converted into the total RNA, the expression level of the Desmocollin-3 genes was assayed using a quantitative PCR method by adding 5 µL of TaqMan Universal PCR master Mix (Applied Biosystems Inc.) and 5 µL of TaqMan Reagents (Applied Biosystems Inc.) containing a primer set amplifying a base sequence derived from a part of a first exon and a part of a second exon of the Desmocollin-3 gene to prepare a 10 µL of reaction solution. In the PCR reaction, a cycle of 15 seconds at 95°C and 1 minute at 60°C was repeated 40 times after 2 minutes at 50°C and 10 minutes at 95°C. On the other hand, the expression level of a β-actin gene contained in the same amount of template cDNA was assayed with TaqMan β-actin Control Reagents (Applied Biosystems Inc.), which was used as an internal standard.

In the group administered with the siRNA, the expression level of mRNA of the Desmocollin-3 genes was decreased by 85% in comparison with the group administered with the non-silencing dsRNA1 which was used as a negative control and showed a statistically significant difference (P<0.001) (Fig. 2B). These results revealed that the inhibition of cell growth of the human lung cancer cell line NCI-H226 was induced by the decrease in expression level of the Desmocollin-3a and Desmocollin-3b genes.

### Example 4

### Decrease in expression level of Desmocollin-3 protein by administering siRNA against Desmocollin-3 gene

The human lung cancer cell line NCI-H226 used in Example 2 was suspended in the RPMI1640 medium and was seeded onto a 24-well flat-bottomed tissue culture plate (BD Falcon) with a cell density of 1×10⁵ cells/well. The cells were cultured in 5% CO₂ gas stream at 37°C overnight and transfected with an siRNA in accordance with the method used in Example 2. 72 hours after the transfection, the cells were washed with PBS and lyzed with RIPA buffer [50 mM Tris-hydrochloride buffer, pH 7.5, 150 mM sodium chloride, 1% Triton X-100, 0.1% SDS, 1% deoxycholic acid, Complete Protease Inhibitor (Roche Diagnostics) and Phosphatase Inhibitor Cocktail-2 (Sigma)]. In order to unify the number of cells per unit amount of the lysate, the amounts of RIPA buffer added to the wells were determined in accordance with the fluorescence intensities in Example 2. 20 µL of cell lysate was subjected to SDS-PAGE in polyacrylamide gel with a concentration gradient of 4% to 20%. The electrophoresed and separated proteins were transferred to a PVDF membrane (BIORAD) by a conventional method and then were left in a blocking solution (Tris-buffered saline, 0.1% Tween 20, 3% skimmed milk) at the room temperature for 1 hour. Anti-Desmocollin-3 antibody (Progen) was added to Can Get Signal (Toyobo Co., Ltd.) solution 1 to have a concentration diluted to 1000 folds, was incubated at the room temperature for 90 minutes, and then was left in a secondary antibody solution, which was obtained by diluting HRP-labeled anti-mouse IgG antibody (ImmunoResearch Laboratories) in Can Get Signal (Toyobo Co., Ltd.) solution 2, at the room temperature for 1 hour. As an inner standard control, after blocking, anti-β-actin antibody (SIGMA) was added so as to have a concentration diluted to 1000 folds with the blocking solution, incubated at the room temperature for 90 minutes, and left in a secondary antibody solution, which was obtained by diluting HRP-labeled anti-mouse IgG antibody (ImmunoResearch Laboratories) with the blocking buffer to 50,000 folds, at the room temperature for 1 hour. The detection was performed using Supersignal West Chemiluminescent (Pierce) in accordance with the protocol attached thereto.

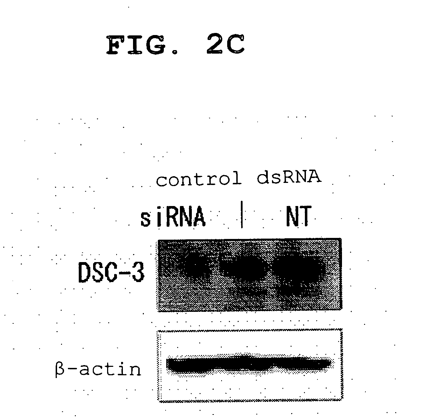

In the cells administered with the non-silencing dsRNA1, specific bands derived from the Desmocollin-3a protein and the Desmocollin-3b protein was noted at the positions near 109 kDa and 100 kDa in molecular weight by means of the antibody against the Desmocollin-3, while the specific bands were not detected in the cells administered with the siRNA. The same degree of intensity was detected in the cells administered with the non-silencing dsRNA1 and the cells administered with the siRNA by the antibody against the β-actin (Fig. 2C).

### Example 5

### Immunostaining of micro array of lung cancer tissue using anti-Desmocollin-3 antibody

Immunohistostaining of a micro array of human lung cancer tissue was carried out using the anti-Desmocolling-3 antibody (Progen). VECTASTAIN Elite ABC KIT (VECTOR LABORATORIES) was used for the immunostaining. Specifically, a slide with a sample attached thereto was immersed in xylene for 5 minutes three times, in 100% ethanol for 5 minutes twice, and in 90%, 80%, and 70% ethanol for 5 minutes once, respectively, immersed in a citrate buffer with pH 6.0 and then autoclaved at 121°C for 20 minutes. After depressurization, the slide was left in the citrate buffer at the room temperature for 20 minutes, the buffer was washed out by water-washing, and then the slide was immersed in purified water for 5 minutes. 3% hydrogen peroxide was dropped onto the slide, the slide was left at rest in an incubator at the room temperature for 7. 5 minutes, the reagent on the section was washed out with purified water and was immersed in the PBS for 5 minutes, and then normal bovine serum attached to the kit was dropped on the section and then was allowed to react in the incubator for 20 minutes. After removing the normal bovine serum, the anti-Desmocollin-3 antibody (Progen) diluted with an antibody diluting buffer (containing Tween 20) was dropped on the section and then was left at rest at 4°C overnight. The reagent on the section was washed out with the PBS, the section was immersed in the PBS for 5 minutes three times, a biotin-labeled anti-mouse IgG solution attached to the kit was dropped on the section, and then the section was left at rest for 30 minutes. The reagent on the section was washed out with the PBS, the section was immersed in the PBS for 5 minutes, a CECTASTAIN Elite ABS Reagent attached to the kit was dropped on the section, and then the section was left at rest for 30 minutes. The reagent on the section was washed out with the PBS, the section was immersed in the PBS for 5 minutes, a DAB substrate attached to the kit was dropped on the section, and then the section was allowed to react for 5 to 15 minutes. The reagent on the section was washed out with purified water, the section was immersed in the purified water, the section was immersed in a haematoxylin solution for 1 minute, and then the section was washed with tap water. The slide was immersed in 70%, 80%, and 90% ethanol for 5 minutes once, respectively, was immersed in 100% ethanol for 5 minutes twice, was immersed in xylene for 5 minutes three times, and then was sealed by a mounting agent. As a result of observation of the stained image with a microscope, a strong stain was observed in the squamous cell cancer section of the lung (Table 1).

**[Table 1]**

| Summary of micro array of squamous cell cancer tissue | | | | | | | |
|---|---|---|---|---|---|---|---|
| Grade | Array spot | Positive | Staining score | | | | |
| | Total | Positi ve | ratio % | negative | + | ++ | +++ |
| Normal | 60 | 0 | 0 | 60 | | | |
| LCCa | 65 | 5 | 8 | 58 | | | |
| I | 3 | 3 | 100 | 1 | 1 | 1 | 1 |
| II | 38 | 32 | 84.2 | 6 | 5 | 13 | 14 |
| III | 17 | 11 | 64.7 | 6 | 1 | 7 | 3 |
| Sum of I-III | 58 | 46 | 79.3 | 12 | 7 | 21 | 18 |

| | | | | | | | |
|---|---|---|---|---|---|---|---|
| LCCa: Large cell cancer I, II, and III: Grades of squamous cell cancer I, II, and III | | | | | | | |

### Example 6

### Overexpression of TM4SF13 in cancer

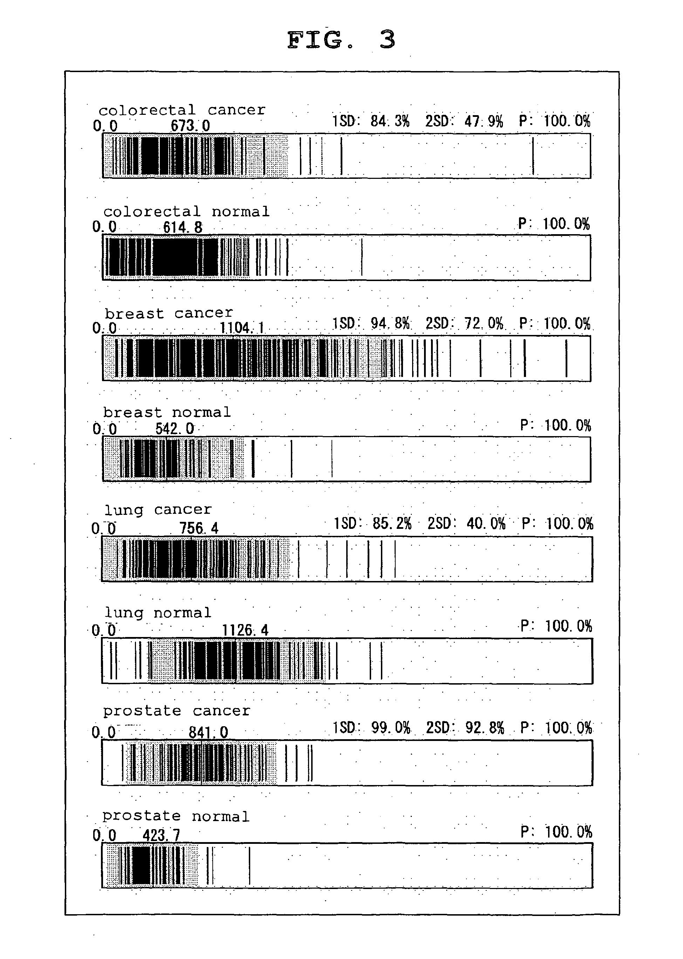

By carrying out the hybridization method using mRNA derived from cancer tissue and mRNA derived from peripheral normal tissue, which are all extracted from a cancer patient, a gene expression profile was prepared. As a result of analysis of the gene expression profile, it was found that the corresponding gene is expressed highly in the cancer tissue. Particularly, in breast cancer and prostate cancer, remarkable overexpression was observed in comparison with the peripheral normal tissue (Fig. 3).

### Example 7

### Inhibition of cell growth in human breast cancer cell line by administering siRNA against TM4SF13 gene

Human breast cancer cell line T-47D purchased from American Type Culture Collection (ATCC) was suspended in an RPMI1640 medium (Invitrogen) containing 10% fetal bovine serum (JRH), was seeded onto a 6-well flat-bottomed tissue culture plate (BD Falcon) with a cell density of 100,000 cells/well, and was cultured in 5% CO₂ gas stream at 37°C overnight. Thereafter, siRNA was transfected.

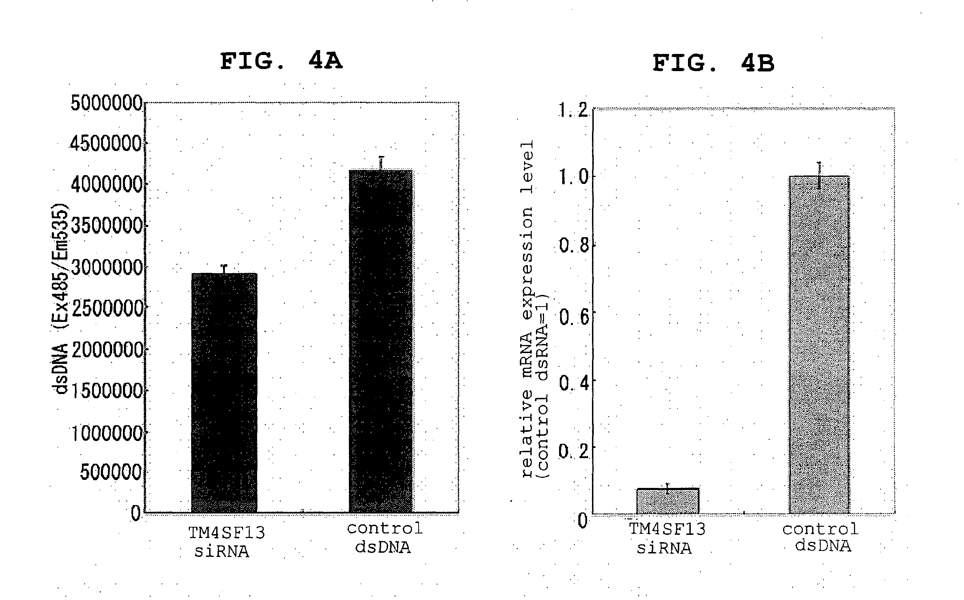

Specifically, the siRNA having an activity of cleaving mRNA of a TM4SF13 gene was manufactured by hybridizing two kinds of RNA fragments (SEQ ID NO: 21 and SEQ ID NO: 22 (siRNA2a)). In the same way, three kinds of siRNAs were additionally manufactured by hybridizing two kinds of RNA fragments (SEQ ID NO: 23 and SEQ ID NO: 24 (siRNA2b), or SEQ ID NO: 25 and SEQ ID NO: 26 (siRNA2c), or SEQ ID NO: 27 and SEQ ID NO: 28 (siRNA2d)). Total four kinds of siRNAs were mixed by equal quantity and were provided to transfection (hereinafter, abbreviated as siRNA2). As a control, two kinds of RNA fragments (SEQ ID NO: 29 and SEQ ID NO: 30) having no activity of cleaving mRNA in animal cells were hybridized in the same way for use (hereinafter, abbreviated as non-silencing dsRNA1). 40 pmol of siRNA2 or 40 pmol of non-silencing dsRNA1 was added to 50 µL of Opti-MEM I (Invitrogen), was mixed with 50 µL of Opti-MEM I (Invitrogen) to which 2 µL of Lipofectamine 2000 (Invitrogen) was added, and then was left at the room temperature for 20 minutes. The mixed solution was all added to a cell culture of T-47D and the cells were further cultured for 24 hours, and then cells were collected. The collected cells were seeded onto 96-well flat-bottomed tissue culture plate with a cell density of 3,000 cells/well and were additionally cultured in the RPMI1640 medium (Invitrogen) containing 10% fetal bovine serum (JRH) in 5% CO₂ gas stream at 37°C for two days. After the medium was removed, the plate was left at rest at -80°C for 5 minutes and then was left at the room temperature for 5 minutes. An aqueous solution containing 1% PicoGreen (Molecular Probes) and 1% IGEPAL-CA630 (ICN Biomedicals) was added thereto by 100 µL/well and then the mixture was left for 20 minutes. Thereafter, by measuring fluorescence intensity thereof at an excitation wavelength of 485 nm and at an emission wavelength of 535 nm, the DNA content in cells was assayed. As a result, the cell administered with siRNA2 had the fluorescence intensity lowered by about 30% than that of the cell administered with non-silencing dsRNA1 and showed a statistically significant difference (P<0.001) (Fig. 4A).

### Example 8

### Decrease in expression level of mRNA of TM4SF13 gene by administering siRNA against TM4SF13 gene

The human breast cancer cell line T-47D used in Example 7 was suspended in the RPMI1640 medium and seeded onto a 24-well flat-bottomed tissue culture plate (BD Falcon) with a cell density of 100,000 cells/well. The cells were cultured in 5% CO₂ gas stream at 37°C overnight and transfected with an siRNA in accordance with the method used in Example 7. After the transfection, the cells were continuously cultured for 24 hours and then total RNAs were extracted therefrom by the use of RNeasy Mini Total RNA Kit (QIAGEN) . Using as a template about 100 ng of the total RNA, reverse transcription reaction was carried out on TaqMan Reverse Transcription Reagents (Applied Biosystems Inc.) in accordance with the protocol attached thereto. Using as a template cDNA in an amount corresponding to 10 ng when converted into the total RNA, the expression level of the TM4SF6 gene was assayed using a quantitative PCR method by adding 5 µL of TaqMan Universal PCR master Mix (Applied Biosystems Inc.) and 5 µL of TaqMan Reagents (Applied Biosystems Inc.) containing a primer set amplifying a base sequence derived from a part of a fourth exon and a part of a fifth exon of the TM4SF13 gene to prepare a 10 µL reaction solution. In the PCR reaction, a cycle of 15 seconds at 95°C and 1 minute at 60°C was repeated 40 times after 2 minutes at 50°C and 10 minutes at 95°C. On the other hand, the expression level of a β-actin gene contained in the same amount of template cDNA was assayed with TaqMan β-actin Control Reagents (Applied Biosystems Inc.), which was used as an internal standard.

In the group administered with the siRNA2, the expression level of mRNA of the TM4SF13 gene was decreased by 97% in comparison with the group administered with the non-silencing dsRNA1 which was used as a negative control and showed a statistically significant difference (P<0.001) (Fig. 4B). These results revealed that the inhibition of cell growth of the human breast cancer cell line T-47D was induced by the decrease in expression level of the TM4SF13 gene.

### Example 9

### Localization of TM4SF13 protein in a cell membrane

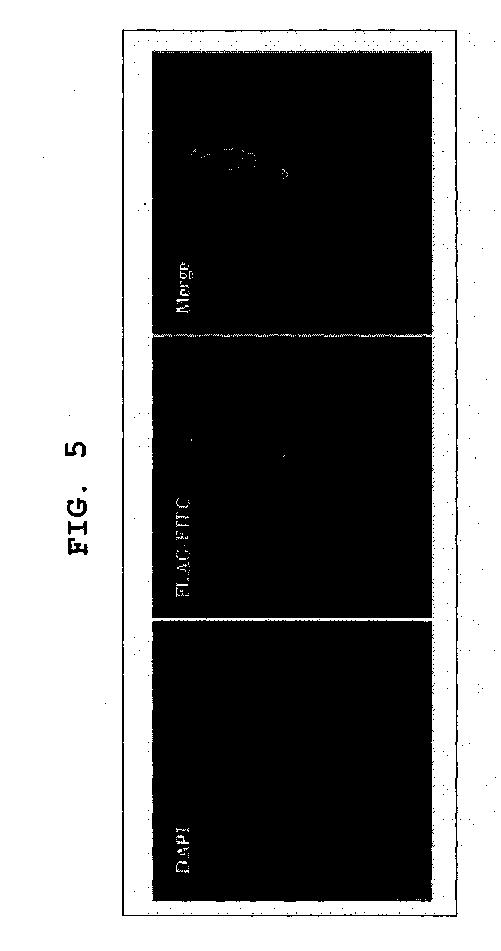

Human embryo kidney cells HEK293 purchased from American Type Culture Collection (ATCC) were suspended in an Eagle' s MEM medium (Invitrogen) containing 10% fetal bovine serum (JRH), were seeded onto a 6-well flat-bottomed tissue culture plate (BD Falcon), and were cultured in 5% CO₂ gas stream at 37°C overnight. Thereafter, an animal cell expression vector p3xFLAG-CMV-14-TM4SF13 having a cDNA sequence (SEQ OD NO: 5) encoding TM4SF13 protein was transfected. Specifically, about 4 µL of p3xFLAG-CMV-14-TM4SF13 was added to 250 µL of Opti-MEM I (Invitrogen) and the mixture was mixed with 250 µL of Opti-MEM I (Invitrogen) to which 10 µL of Lipofectamine 2000 (Invitrogen) was added and then was left at the room temperature for 15 minutes. The mixed solution was all added to a cell culture of HEK293 and the cells were cultured continuously, and then cells were collected. The collected cells were suspended in a solution for cell culture and 5,000 cells were seeded onto a 4-well flat-bottomed tissue culture plate coated with 2% gelatin and were additionally cultured for 24 hours. The cells were immunostained using a conventional method with anti-FLAG M2 antibody (Sigma) and FITC-labeled anti-mouse IgG antibody (WAKO) and the stained images were observed with a fluorescent microscope. It was observed that the TM4SF13-3xFLAG fusion protein was localized in a cell membrane (Fig. 5).

### Example 10

### Interaction between TM4SF13 protein and integrin-α3

1 mL of an aqueous solution containing 1 mM CaCl₂ and MgCl₂, 10 mM Tris-HCl (pH 7.5), 150 mM NaCl, 1% Brij98, and complete protease inhibitor (Roche) by one particle per 7 mL was added to MDA-MB231 cell clone strongly expressing TM4SF13-3xFLAG fusion protein and the mixture was left at rest on ice for 20 minutes, thereby lysing the cells. As a negative control, MDA-MB231 cell clone obtained by transfecting p3xFLAG-CMV-14 (Sigma) was lysed in the same way. After collection of the cell lysate, the collected cell lysate was subjected to a centrifugal separation at 15,000 rpm for 30 minutes, thereby collecting the supernatant. 50 µL of protein G agarose slurry was added to the collected solution and then the mixture was rotationally agitated at 4°C overnight. The supernatant was collected by performing a centrifugal separation to the agitated solution by the use of a small-sized centrifuge and then was immunoprecipitated by adding 4 µg of anti-integrin-α3 antibody (Chemicon) and 12.5 µL of protein G agarose slurry and rotationally agitating the mixture at 4°C for 4 hours. As a negative control, 4 µg of nonimmunized mouse IgG₁ (R&D Systems) was added, 12. 5 µL of protein G agarose slurry was then added, and the mixture was rotationally agitated at 4°C for 4 hours. The supernatant was removed by performing a centrifugal separation to the agitated solution with a small-sized centrifuge and then the precipitated protein G agarose was washed with 250 µL of the solution used for the cell lysis four times. 65 µL of the aqueous solution used for the cell lysis and 75 µL of Laemmli' s sample buffer (BIORAD) were added to the washed protein G agarose, the mixture was processed at 95°C for 5 minutes, and then a liquid fraction was collected.

10 µL of the cell-lysate was subjected to SDS-PAGE in 7.5% polyacrylamide gel. The electrophoresed and separated proteins were transferred to a PVDF membrane (BIORAD) by a conventional method and then left in a blocking solution (Tris-buffered saline, 0.1% Tween 20, 3% skimmed milk) at the room temperature for 1 hour. Anti-FLAG-M2 antibody (Sigma) was added to Can Get Signal (Toyobo Co. , Ltd.) solution 1 so as to have a concentration diluted to 1000 folds, was incubated at the room temperature for 90 minutes, and then was left in a secondary antibody solution, which was obtained by diluting HRP-labeled anti-mouse IgG antibody (ImmunoResearch Laboratories) in Can Get Signal (Toyobo Co., Ltd.) solution 2, at the room temperature for 1 hour. As a negative control, after blocking, the nonimmunized mouse IgG₁ (R&D Systems) was added so as to have a concentration diluted to 1000 folds with the blocking solution, incubated at the room temperature for 90 minutes, and left in a secondary antibody solution, which was obtained by diluting HRP-labeled anti-mouse IgG antibody (ImmunoResearch Laboratories) with the blocking buffer to 50,000 folds, at the room temperature for 1 hour. The detection was performed using Supersignal West Chemiluminescent (Pierce) in accordance with the protocol attached thereto. A signal derived from the TM4SF13-Flag fusion protein by the anti-flag-M2 antibody was detected from the immunoprecipitate by the anti-integrin-α3, while a signal derived from the anti-Flag-M2 antibody was not detected from the product of the immunoprecipitated negative control. No signal was detected from the immunoprecipitate by the anti-integrin-α3 and the product of the immunoprecipitated negative control according to nonimmunized mouse IgG1.

### Example 11

### Interaction between TM4SF13 protein and integrin-α5

1 mL of an aqueous solution containing 1 mM CaCl₂ and MgCl₂, 10 mM Tris-HCl (pH 7.5), 150 mM NaCl, 1% CHAPS, and complete protease inhibitor (Roche) by one particle per 7 mL was added to MDA-MB231 cell clone strongly expressing TM4SF13-3xFLAG fusion protein and the mixture was left at rest on ice for 20 minutes, thereby lysing the cells. As a negative control, MDA-MB231 cell clone obtained by transfecting p3xFLAG-CMV-14 (Sigma) was lysed in the same way. After collection of the cell lysate, the collected cell lysate was subjected to a centrifugal separation at 15, 000 rpm for 30 minutes, thereby collecting the supernatant. 50 µL of protein G agarose slurry was added to the collected solution and then the mixture was rotationally agitated at 4°C overnight. The supernatant was collected by performing a centrifugal separation to the agitated solution by the use of a small-sized centrifuge and then was immunoprecipitated by adding 4 µg of anti-integrin-α5 antibody (Sigma) and 12.5 µL of protein G agarose slurry and rotationally agitating the mixture at 4°C for 4 hours. As a negative control, 4 µg of nonimmunized mouse IgG₁ (R&D Systems) was added, 12.5 µL of protein G agarose slurry was then added, and the mixture was rotationally agitated at 4°C for 4 hours. The supernatant was removed by performing a centrifugal separation to the agitated solution with a small-sized centrifuge and then the precipitated protein G agarose was washed with 250 µL of the solution used for the cell lysis four times. 65 µL of the aqueous solution used for the cell lysis and 75 µL of Laemmli' s sample buffer (BIORAD) were added to the washed protein G agarose, the mixture was processed at 95°C for 5 minutes, and then a liquid fraction was collected.

10 µL of the cell-lysate was subjected to SDS-PAGE in 7.5% polyacrylamide gel. The electrophoresed and separated proteins were transferred to a PVDF membrane (BIORAD) by a conventional method and then left in a blocking solution (Tris-buffered saline, 0.1% Tween 20, 3% skimmed milk) at the room temperature for 1 hour. Anti-FLAG-M2 antibody (Sigma) was added to Can Get Signal (Toyobo Co., Ltd.) solution so as to have a concentration diluted to 1000 folds, was incubated at the room temperature for 90 minutes, and then was left in a secondary antibody solution, which was obtained by diluting HRP-labeled anti-mouse IgG antibody (ImmunoResearch Laboratories) in Can Get Signal (Toyobo Co., Ltd.) solution 2, at the room temperature for 1 hour. As a negative control, after blocking, the nonimmunized mouse IgG₁ (R&D Systems) was added so as to have a concentration diluted to 1000 folds with the blocking solution, incubated at the room temperature for 90 minutes, and left in a secondary antibody solution, which was obtained by diluting HRP-labeled anti-mouse IgG antibody (ImmunoResearch Laboratories) with the blocking buffer to 50,000 folds, at the room temperature for 1 hour. The detection was performed using Supersignal West Chemiluminescent (Pierce) in accordance with the protocol attached thereto. A signal derived from the TM4SF13-Flag fusion protein by the anti-flag-M2 antibody was detected from the immunoprecipitate by the anti-integrin-α5, while a signal derived from the anti-Flag-M2 antibody was not detected from the product of the immunoprecipitated negative control. No signal was detected from the immunoprecipitate by the anti-integrin-α5 and the product of the immunoprecipitated negative control according to nonimmunized mouse IgG1.

### Example 12

### Variation in cell form on extracellular matrix coated plate due to expression of TM4SF13 protein

TM4SF13 protein was transiently expressed in human breast cancer cell line MDA-MB231 and the phenotype on an extracellular matrix coat was observed. Specifically, an animal cell expression vector having a cDNA sequence (SEQ ID NO: 5) encoding TM4SF13 protein (SEQ ID NO: 6) was introduced into the cells, the human breast cancer cell line MDA-MB231 transiently expressing the TM4SF13 protein was seeded onto a 96-well flat-bottomed tissue culture plate (BD Falcon) coated with laminin which is a ligand for integrin-α3 or a 96-well flat-bottomed tissue culture plate (BD Falcon) coated with fibronectin which is a ligand for integrain-α5, and then the phenotype was observed when the seeded cell lines were cultured in a Leibovitz L-15 medium (Invitrogen) without 10% fetal bovine serum. As a negative control, an animal cell expression vector expressing LacZ protein was introduced into the cells in the same way.

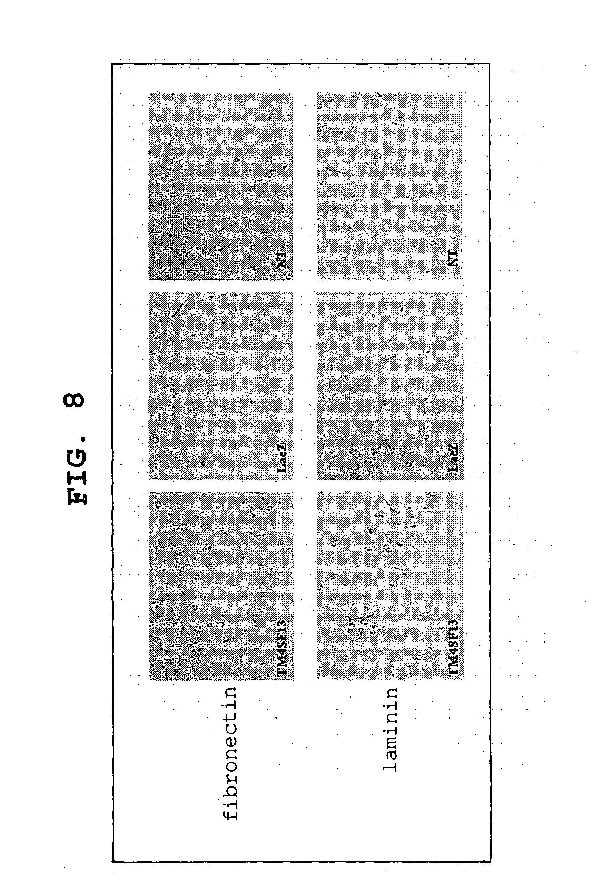

In the group expressing TM4SF13, it was observed that on the laminin coated plate and the fibronectin coated plate, the extension of cells were suppressed and the length of cells were reduced in comparison with the LacZ expressed group and the foreign gene non-introduced group (Fig. 8).

### Reference Example 1

### Cloning and determination of base sequence of cDNA encoding TM4SF13

Using as a template cDNA derived from human breast cancer cell line HCC70, the PCR was performed with primer X (SEQ ID NO: 29) and primer Y (SEQ ID NO: 30). In the composition of a reaction solution for the reaction, using as a template 10 ng of the cDNA, 50 µL of the reaction solution was prepared by adding 1 µL of KOD-plus DNA Polymerase (Toyobo), 300 nM primer X (SEQ ID NO: 29) and 300 nM primer Y (SEQ ID NO: 30) , 1 mM MgSO₄, 200 µM dNTPs, and 5 µL of 10xKOD-plus Buffer (Toyobo). In the PCR reaction, a cycle of 15 seconds at 94°C, 30 seconds at 58°C, and 1 minute at 72°C was repeated 35 times after 2 minutes at 94°C. After migrating by agarose gel electrophoresis, the DNA fragment corresponding to about 745b was confirmed. By diluting the PCR product to 1000 folds and using 1 µL thereof as a template, the PCR was performed with primer X (SEQ ID NO: 29) and primer Z (SEQ ID NO: 31). In the composition of a reaction solution for the reaction, 50 µL of the reaction solution was prepared by adding 1 µL of the solution obtained by diluting the PCR product to 1000 folds, 1µL of KOD-plus DNA Polymerase (Toyobo), 300 nM primer X (SEQ ID NO: 29) and 300 nM primer Z (SEQ ID NO: 31), 1 mM MgSO₄, 200 µM dNTPs, and 5 µL of 10×KOD-plus Buffer (Toyobo). 5 µL of the reaction solution was migrated and the DNA fragment corresponding to about 646b was confirmed using agarose gel electrophoresis. The remaining 45 µL was purified using MinElute PCR purification kit (Qiagen) and then was treated with restriction enzymes KpnI and XbaI. The p3xFLAG-CMV-14 (Sigma) was also treated with restriction enzymes KpnI and XbaI. Each of the DNA fragments was purified using PCR purification kit (Qiagen), a ligation reaction was performed using DNA Ligation Kit ver. 2 (TaKaRa Bio) , the products were introduced into E. coli DH5α (TaKaRa Bio), followed by the selection on LB agar medium containing ampicillin. As a result of analysis of the clone sequences, an animal cell expression vector p3xFLAG-CMV-14-TM4SF13 having a cDNA sequence (SEQ ID NO: 5) encoding TM4SF13 protein (SEQ ID NO: 6) was obtained.

### Reference Example 2

### Establishment of clone of human breast cancer cell line MDA-MB231 expressing TM4SF13-3xFLAG fusion protein

Human breast cancer line cell MDA-MB231 purchased from American Type Culture Collection (ATCC) was suspended in an Dulbecco' s MEM medium (Invitrogen) containing 10% fetal bovine serum (JRH), was seeded onto a 12-well flat-bottomed tissue culture plate (BD Falcon), and cultured in 5% CO₂ gas stream at 37°C overnight. Thereafter, an animal cell expression vector p3xFLAG-CMV-14-TM4SF13 having a cDNA sequence (SEQ ID NO: 5) encoding TM4SF13 protein was transfected. Specifically, about 4 µg of p3xFLAG-CMV-14-TM4SF13 was added to 100 µL of Opti-MEM I (Invitrogen), and the mixture was mixed with 100 µL of Opti-MEM I (Invitrogen) to which 4 µL of Lipofectamine 2000 (Invitrogen) was added and then was left at the room temperature for 15 minutes. The mixed solution was all added to a cell culture of MDA-MB231 and the cells were further cultured for 18 hours. As a negative control, p3xFLAG-CMV-14-TM4SF13 was transfected in the same way as being described above. After collecting the cells, the collected cells were suspended in the Dulbecco' s MEM medium (Invitrogen) containing 10% fetal bovine serum (JRH) and 500 µg/ml G418 (Invitrogen) and were cultured in 5% CO₂ gas stream at 37°C for 6 days. The grown cells were collected, were diluted to 5 folds, were suspended in the Dulbecco' s MEM medium (Invitrogen) containing 10% fetal bovine serum (JRH) and 1000 µg/ml G418 (Invitrogen), and were cultured in 5% CO₂ gas stream at 37°C for 6 days. The cells resistant to G418 were collected, a limiting dilution was performed with the Dulbecco's MEM medium (Invitrogen) containing 10% fetal bovine serum (JRH) and 1000 µg/ml G418 (Invitrogen) so as to be 0.5 cells/well, and the diluted cells were seeded onto a 96-well flat-bottomed tissue culture plate (BD Falcon). The grown cells were suspended in the Dulbecco's MEM medium (Invitrogen) containing 10% fetal bovine serum (JRH) and 1000 µg/ml G418 (Invitrogen), were seeded onto a 24-well flat-bottomed tissue culture plate (BD Falcon), were allowed to grow again, and then were seeded onto a 24-well flat-bottomed tissue culture plate (BD Falcon) in the same way. The expression level of TM4SF13-3xFLAG fusion protein in the collected clones was assayed by fluorescent flow cytometry by the use of anti-FLAG-M2 antibody (Sigma). Specifically, the cells were fixed with PBS containing 4% paraformaldehyde, were washed with the PBS, were suspended in 200 µL of a reaction solution (PBS containing 1% bovine serum albumin, 0.1% sodium azide, and 0.1% saponin) containing 0.2 µg of anti-FLAG-M2 antibody (Sigma) or nonimmunized mouse IgG₁ (R&D) as a negative control, and were left on ice for 30 minutes. After washing the cells with the reaction solution, the cells were suspended in a secondary antibody solution obtained by diluting FITC-labeled anti-mouse IgG antibody (WAKO) to 500 folds by the use of the reaction solution, and were left on ice for 30 minutes. By washing the cells with the reaction solution, suspending the cells with the reaction solution, and then assaying the fluorescence intensity with FACS, clones strongly expressing the TM4-SF13-3xFLAG fusion protein were selected.

### Example 13

### Inhibition of cell growth in human lung cancer cell line by administering siRNA against TM4SF6 gene

Human lung cancer cell line NCI-H522 purchased from American Type Culture Collection (ATCC) was suspended in an RPMI1640 medium (Invitrogen) containing 10% fetal bovine serum (JRH), was seeded onto a 6-well flat-bottomed tissue culture plate (BD Falcon) with a cell density of 1×10⁵ cells/well, and was cultured in 5% CO₂ gas stream at 37°C overnight. Thereafter, an siRNA was transfected.

Specifically, four kinds of siRNAs having an activity of cleaving mRNA of TM4SF6 gene were mixed and provided to transfection. As a control, non-silencing dsRNA was used. 40 pmol of siRNA against TM4SF6 gene or 40 pmol of non-silencing dsRNA was added to 50 µL of Opti-MEM I (Invitrogen), was mixed with 50 µL of Opti-MEM I (Invitrogen) to which 2 µL of Lipofectamine 2000 (Invitrogen) was added, and then was left at the room temperature for 20 minutes. The mixed solution was all added to a cell culture of NCI-H522 and the cells were further cultured for 24 hours, and then cells were collected. The collected cells were seeded onto 96-well flat-bottomed tissue culture plate with a cell density of 3,000 cells/well and were additionally cultured in the RPMI1640 medium (Invitrogen) containing 10% fetal bovine serum (JRH) in 5% CO₂ gas stream at 37°C for two days. After the medium was removed, the plate was left at rest at -80°C for 5 minutes and then was left at the room temperature for 5 minutes. An aqueous solution containing 1% PicoGreen (Molecular Probes) and 1% IGEPAL-CA630 (ICN Biomedicals) was added thereto by 100 µL/well and then the mixture was left for 20 minutes. Thereafter, by measuring fluorescence intensity thereof at an excitation wavelength of 485 nm and at an emission wavelength of 535 nm, the DNA content in cells was assayed. As a result, the cells administered with siRNA against TM4SF6 gene had the fluorescence intensity lowered by about 59% than that of the cell administered with non-silencing dsRNA1 and showed a statistically significant difference (P<0.001).

### Example 14

### Decrease in expression level of mRNA of TM4SF6 gene by administering siRNA against TM4SF6 gene

The human lung cancer cell line NCI-H522 used in Example 13 was suspended in the RPMI1640 medium and seeded onto a 24-well flat-bottomed tissue culture plate (BD Falcon) with a cell density of 1×10⁵ cells/well. The cells were cultured in 5% CO₂ gas stream at 37°C overnight and transfected with an siRNA in accordance with the method used in Example 13. After the transfection, the cells were continuously cultured for 24 hours and then total RNAs were extracted therefrom by the use of RNeasy Mini Total RNA Kit (QIAGEN). Using as a template about 100 ng of the total RNA, reverse transcription reaction was carried out on TaqMan Reverse Transcription Reagents (Applied Biosystems Inc.) in accordance with the protocol attached thereto. Using as a template cDNA in an amount corresponding to 10 ng when converted into the total RNA, the expression level of the TM4SF6 gene was assayed using a quantitative PCR method by adding 5 µL of TaqMan Universal PCR master Mix (Applied Biosystems Inc.) and 5 µL of TaqMan Reagents (Applied Biosystems Inc.) containing a primer set amplifying a base sequence derived from a part of a first exon and a part of a second exon of the TM4SF6 gene to prepare a 10 µL of reaction solution. In the PCR reaction, a cycle of 15 seconds at 95°C and 1 minute at 60°C was repeated 40 times after 2 minutes at 50°C and 10 minutes at 95°C. On the other hand, the expression level of a β-actin gene contained in the same amount of template cDNA was assayed with TaqMan β-actin Control Reagents (Applied Biosystems Inc.), which was used as an internal standard.

In the group administered with the siRNA against TM4SF6 gene, the expression level of mRNA of the TM4SF6 gene was decreased by 93% in comparison with the group administered with the non-silencing dsRNA which was used as a negative control and showed a statistically significant difference (P<0.001). These results revealed that the inhibition of cell growth of the human lung cancer cell line NCI-H522 was induced by the decrease in expression level of the TM4SF6 gene.

### Example 15

### Inhibition of cell growth in human lung cancer cell line by administering siRNA against LY-6K gene

Human lung cancer cell line NCI-H23 purchased from American Type Culture Collection (ATCC) was suspended in an RPMI1640 medium (Invitrogen) containing 10% fetal bovine serum (JRH), seeded onto a 6-well flat-bottomed tissue culture plate (BD Falcon) with a cell density of 1×10⁵ cells/well, and cultured in 5% CO₂ gas stream at 37°C overnight. Thereafter, an siRNA was transfected.

Specifically, four kinds of siRNAs having an activity of cleaving mRNA of LY-6K gene were mixed by equal quantity and provided to transfection. As a control, non-silencing dsRNA was used. 80 pmol of siRNA for LY-6K gene or 80 pmol of non-silencing dsRNA was added to 50 µL of Opti-MEM I (Invitrogen), was mixed with 50 µL of Opti-MEM I (Invitrogen) to which 4 µL of Lipofectamine 2000 (Invitrogen) was added, and then was left at the room temperature for 20 minutes. The mixed solution was all added to a cell culture of NCI-H23 and the cells were further cultured for 24 hours, and then cells were collected. The collected cells were seeded onto 96-well flat-bottomed tissue culture plate with a cell density of 3,000 cells/well and were additionally cultured in the RPMI1640 medium (Invitrogen) containing 10% fetal bovine serum (JRH) in 5% CO₂ gas stream at 37°C for two days. After the medium was removed, the plate was left at rest at -80°C for 5 minutes and then was left at the room temperature for 5 minutes. The DNA content in cells was assayed using CyQuant cell proliferation assay kit (Molecular Probes) in accordance with the protocol attached thereto. As a result, the cell administered with siRNA against LY-6K gene had the fluorescence intensity lowered by about 17% than that of the cell administered with non-silencing dsRNA1 and showed a statistically significant difference (P<0.001).

### Example 16

### Decrease in expression level of mRNA of LY-6K gene by

### administering siRNA against LY-6K gene

The human lung cancer cell line NCI-H23 used in Example 15 was suspended in the RPMI1640 medium and seeded onto a 24-well flat-bottomed tissue culture plate (BD Falcon) with a cell density of 1×10⁵ cells/well. The cells were cultured in 5% CO₂ gas stream at 37°C overnight and transfected with an siRNA in accordance with the method used in Example 15. After the transfection, the cells were continuously cultured for 24 hours and then total RNAs were extracted therefrom by the use of RNeasy Mini Total RNA Kit (QIAGEN). Using as a template about 100 ng of the total RNA, reverse transcription reaction was carried out on TaqMan Reverse Transcription Reagents (Applied Biosystems Inc.) in accordance with the protocol attached thereto. Using as a template cDNA in an amount corresponding to 10 ng when converted into the total RNA, the expression level of the LY-6K gene was assayed using a quantitative PCR method by adding 5 µL of TaqMan Universal PCR master Mix (Applied Biosystems Inc.) and 5 µL of TaqMan Reagents (Applied Biosystems Inc.) containing a primer set amplifying a base sequence derived from a part of a second exon and a part of a third exon of the LY-6K gene to prepare a 10 µL of reaction solution. In the PCR reaction, a cycle of 15 seconds at 95°C and 1 minute at 60°C was repeated 40 times after 2 minutes at 50°C and 10 minutes at 95°C. On the other hand, the expression level of a β-actin gene contained in the same amount of template cDNA was assayed with TaqMan β-actin Control Reagents (Applied Biosystems Inc.), which was used as an internal standard.

In the group administered with the siRNA against LY-6K gene, the expression level of mRNA of the LY-6K gene was decreased by 84% in comparison with the group administered with the non-silencing dsRNA which was used as a negative control and showed a statistically significant difference (P<0.001). These results revealed that the inhibition of cell growth of the human lung cancer cell line NCI-H23 was induced by the decrease in expression level of the LY-6K gene.

### INDUSTRIAL APPLICABILITY

The proteins used in the present invention or a polynucleotide encoding the proteins are specifically expressed in cancer tissue, thus can be a diagnosis marker for cancer. Also, by suppressing the activity and/or expression of the proteins, promoting effect on apoptosis in cancer cells and inhibiting effect on the growth of cancer cells are obtained, thus antibodies against the proteins, antisense polynucleotides against the polynucleotides, compounds that inhibit activities of the proteins or salt thereof, and compounds that inhibit expression of genes of the proteins or salt thereof, can be safely used as preventive/remedy agents for cancer, agents for promoting apoptosis in cancer cells, and agents for inhibiting the growth of cancer cells. Further, the proteins, the polynucleotides, the antibodies, and the like, are useful in screening for a preventive/remedy substance for cancer, a substance that promotes apoptosis in cancer cells, and/or a substance that inhibits the growth of cancer cells.

This application is based on a patent application No. 2005-059277 filed in Japan (filing date: March 3, 2005), the contents of which are incorporated in full herein by this reference.

## Claims

1. An agent for promoting the apoptosis in cancer cells and/or inhibiting the growth of cancer cells, which comprises an antibody against a protein comprising the same or substantially the same amino acid sequence as the amino acid sequence represented by SEQ ID NO: 2 or SEQ ID NO: 4, or against partial peptide thereof.

2. The agent according to Claim 1, which is for preventive/remedy for cancer.

3. An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 2 or SEQ ID NO: 4.

4. The agent according to Claim 3, which is for preventive/remedy for cancer.

5. An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 2 or SEQ ID NO: 4.

6. The agent according to Claim 5, which is for preventive/remedy for cancer.

7. A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 2 or SEQ ID NO: 4.

8. The method according to Claim 7, which is for preventive/remedy for cancer.

9. Use of a substance that inhibits the expression and/or activity of a protein comprising the same or substantially the same amino acid sequence as the amino acid sequence represented by SEQ ID NO: 2 or SEQ ID NO: 4, to manufacture an agent for promoting the apoptosis in cancer cells and/or inhibiting the growth of cancer cells.

10. The use according to Claim 9, wherein the agent for promoting the apoptosis in cancer cells and/or inhibiting the growth of cancer cells is for preventive/remedy for cancer.

11. A method of screening for a substance that promotes the apoptosis in cancer cells and/or inhibits the growth of cancer cells, the method includes using a protein comprising the same or substantially the same amino acid sequence as the amino acid sequence represented by SEQ ID NO: 2 or SEQ ID NO: 4, or partial peptide thereof.

12. The method according to Claim 11, wherein the protein comprising the same or substantially the same amino acid sequence as the amino acid sequence represented by SEQ ID NO: 2 or SEQ ID NO: 4, or partial peptide thereof, is provided in the form of a cell capable of producing the protein or the partial peptide.

13. The method according to Claim 12, comprises further using any one selected from a group consisting of antibodies against a protein comprising the same or substantially the same amino acid sequence as the amino acid sequence represented by SEQ ID NO: 2 or SEQ ID NO: 4, or against partial peptide thereof, and polynucleotide encoding the protein or polynucleotide including a part of the base sequence.

14. The method according to Claim 11, which is for the screening for a preventive/remedy substance for cancer.

15. An agent for promoting the apoptosis in cancer cells and/or inhibiting the growth of cancer cells, which comprises a substance that controls the expression of a protein comprising the same or substantially the same amino acid sequence as the amino acid sequence represented by SEQ ID NO: 6 and/or the interaction between the protein and integrin.

16. The agent according to Claim 15, wherein the substance that controls the interaction between the protein comprising the same or substantially the same amino acid sequence as the amino acid sequence represented by SEQ ID NO: 6 and integrin is a neutralizing antibody against the protein.

17. The agent according to Claim 15, wherein the substance that controls the expression of the protein comprising the same or substantially the same amino acid sequence as the amino acid sequence represented by SEQ ID NO: 6 is an antisense polynucleotide including a base sequence or a part of the base sequence, the base sequence being complementary or substantially complementary to a base sequence of polynucleotide encoding the protein.

18. The agent according to Claim 15, wherein the substance that controls the interaction between the protein comprising the same or substantially the same amino acid sequence as the amino acid sequence represented by SEQ ID NO: 6 and integrin is a non-neutralizing antibody against the protein.

19. The agent according to Claim 18, wherein the antibody is an agonist antibody.

20. The agent according to Claim 15, wherein the substance that controls the expression of the protein comprising the same or substantially the same amino acid sequence as the amino acid sequence represented by SEQ ID NO: 6 is the protein or partial peptide thereof, or a polynucleotide that encodes the protein or the partial peptide.

21. The agent according to Claim 15, which is for preventive/remedy for cancer.

22. A method of promoting the apoptosis in cancer cells and/or inhibiting the growth of cancer cells, the method comprises controlling the expression of a protein comprising the same or substantially the same amino acid sequence as the amino acid sequence represented by SEQ ID NO: 6 and/or the interaction between the protein and integrin.

23. The method according to Claim 22, which is for preventive/remedy for cancer.

24. Use of a substance that controls the expression of a protein comprising the same or substantially the same amino acid sequence as the amino acid sequence represented by SEQ ID NO: 6 and/or the interaction between the protein and integrin, to manufacture an agent for promoting the apoptosis in cancer cells and/or inhibiting the growth of cancer cells.

25. The use according to Claim 24, wherein the agent for promoting the apoptosis in cancer cells and/or inhibiting the growth of cancer cells is for preventive/remedy for cancer.

26. A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 6, or partial peptide thereof.

27. A method according to Claim 26, wherein the protein comprising the same or substantially the same amino acid sequence as the amino acid sequence represented by SEQ ID NO: 6, or partial peptide thereof, is provided in the form of a cell capable of producing the protein or the partial peptide.

28. The method according to Claim 27, comprises further using any one selected from a group consisting of antibodies against a protein comprising the same or substantially the same amino acid sequence as the amino acid sequence represented by SEQ ID NO: 6, or against partial peptide thereof, and polynucleotide encoding the protein or polynucleotide including a part of the base sequence thereof.

29. The method according to Claim 26, which is for the screening for a preventive/remedy substance for cancer.

30. A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 6 or partial peptide thereof, and integrin.

31. The method according to Claim 30, wherein the protein comprising the same or substantially the same amino acid sequence as the amino acid sequence represented by SEQ ID NO: 6 or partial peptide thereof, and/or integrin is provided in the form of a cell capable of producing the protein or the partial peptide and/or integrin.

32. The method according to Claim 30, which is for the screening for a preventive/remedy substance for cancer.

33. An agent for promoting the apoptosis in cancer cells and/or inhibiting the growth of cancer cells, which comprises an antibody against a protein comprising the same or substantially the same amino acid sequence as the amino acid sequence represented by SEQ ID NO: 8 or SEQ ID NO: 10, or against partial peptide thereof.

34. The agent according to Claim 33, which is for preventive/remedy for cancer.

35. An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 8 or SEQ ID NO: 10.

36. The agent according to Claim 35, which is for preventive/remedy for cancer.

37. An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 8 or SEQ ID NO: 10.

38. The agent according to Claim 37, which is for preventive/remedy for cancer.

39. A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 8 or SEQ ID NO: 10.

40. The method according to Claim 39, which is for preventive/remedy for cancer.

41. Use of a substance that inhibits the expression and/or activity of a protein comprising the same or substantially the same amino acid sequence as the amino acid sequence represented by SEQ ID NO: 8 or SEQ ID NO: 10, to manufacture an agent for promoting the apoptosis in cancer cells and/or inhibiting the growth of cancer cells.

42. The use according to Claim 41, wherein the agent for promoting the apoptosis in cancer cells and/or inhibiting the growth of cancer cells is for a preventive/remedy for cancer.

43. A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 8 or SEQ ID NO: 10, or partial peptide thereof.

44. The method according to Claim 43, wherein the protein comprising the same or substantially the same amino acid sequence as the amino acid sequence represented by SEQ ID NO: 8 or SEQ ID NO: 10, or partial peptide thereof, is provided in the form of a cell capable of producing the protein or the partial peptide.

45. The method according to Claim 44, comprises further using any one selected from a group consisting of antibodies against a protein comprising the same or substantially the same amino acid sequence as the amino acid sequence represented by SEQ ID NO: 8 or SEQ ID NO: 10, or against partial peptide thereof, and polynucleotide encoding the protein or polynucleotide including a part of the base sequence.

46. The method according to Claim 43, which is for the screening for a preventive/remedy substance for cancer.


SEQUENCE LISTING

<110>  Takeda Pharmaceutical Company Limited

<120>  Preventive/Therapeutic Agent for Cancer

<130>  09890

<150>  JP 2005-059277
<151>  2005-03-03

<160>  31

<170>  PatentIn version 3.3

<210>  1
<211>  2688
<212>  DNA
<213>  Homo sapiens

<220>
<221>  CDS
<222>  (1)..(2688)

<400>  1
atg gcc gcc gct ggg ccc cgg cgc tcc gtg cgc gga gcc gtc tgc ctg        48
Met Ala Ala Ala Gly Pro Arg Arg Ser Val Arg Gly Ala Val Cys Leu
1               5                   10                  15

cat ctg ctg ctg acc ctc gtg atc ttc agt cgt gat ggt gaa gcc tgc        96
His Leu Leu Leu Thr Leu Val Ile Phe Ser Arg Asp Gly Glu Ala Cys
            20                  25                  30

aaa aag gtg ata ctt aat gta cct tct aaa cta gag gca gac aaa ata       144
Lys Lys Val Ile Leu Asn Val Pro Ser Lys Leu Glu Ala Asp Lys Ile
        35                  40                  45

att ggc aga gtt aat ttg gaa gag tgc ttc agg tct gca gac ctc atc       192
Ile Gly Arg Val Asn Leu Glu Glu Cys Phe Arg Ser Ala Asp Leu Ile

    50                  55                  60

cgg tca agt gat cct gat ttc aga gtt cta aat gat ggg tca gtg tac 240
Arg Ser Ser Asp Pro Asp Phe Arg Val Leu Asn Asp Gly Ser Val Tyr
65                  70                  75                  80

aca gcc agg gct gtt gcg ctg tct gat aag aaa aga tca ttt acc ata 288
Thr Ala Arg Ala Val Ala Leu Ser Asp Lys Lys Arg Ser Phe Thr Ile
                85                  90                  95

tgg ctt tct gac aaa agg aaa cag aca cag aaa gag gtt act gtg ctg 336
Trp Leu Ser Asp Lys Arg Lys Gln Thr Gln Lys Glu Val Thr Val Leu
            100                 105                 110

cta gaa cat cag aag aag gta tcg aag aca aga cac act aga gaa act 384
Leu Glu His Gln Lys Lys Val Ser Lys Thr Arg His Thr Arg Glu Thr
        115                 120                 125

gtt ctc agg cgt gcc aag agg aga tgg gca cct att cct tgc tct atg 432
Val Leu Arg Arg Ala Lys Arg Arg Trp Ala Pro Ile Pro Cys Ser Met
    130                 135                 140

caa gag aat tcc ttg ggc cct ttc cca ttg ttt ctt caa caa gtt gaa 480
Gln Glu Asn Ser Leu Gly Pro Phe Pro Leu Phe Leu Gln Gln Val Glu
145                 150                 155                 160

tct gat gca gca cag aac tat act gtc ttc tac tca ata agt gga cgt 528
Ser Asp Ala Ala Gln Asn Tyr Thr Val Phe Tyr Ser Ile Ser Gly Arg
                165                 170                 175

gga gtt gat aaa gaa cct tta aat ttg ttt tat ata gaa aga gac act 576
Gly Val Asp Lys Glu Pro Leu Asn Leu Phe Tyr Ile Glu Arg Asp Thr
            180                 185                 190

gga aat cta ttt tgc act cgg cct gtg gat cgt gaa gaa tat gat gtt 624
Gly Asn Leu Phe Cys Thr Arg Pro Val Asp Arg Glu Glu Tyr Asp Val
        195                 200                 205

ttt gat ttg att gct tat gcg tca act gca gat gga tat tca gca gat 672
Phe Asp Leu Ile Ala Tyr Ala Ser Thr Ala Asp Gly Tyr Ser Ala Asp

210                 215                 220

ctg ccc ctc cca cta ccc atc agg gta gag gat gaa aat gac aac cac        720
Leu Pro Leu Pro Leu Pro Ile Arg Val Glu Asp Glu Asn Asp Asn His
225                 230                 235                 240

cct gtt ttc aca gaa gca att tat aat ttt gaa gtt ttg gaa agt agt        768
Pro Val Phe Thr Glu Ala Ile Tyr Asn Phe Glu Val Leu Glu Ser Ser
                245                 250                 255

aga cct ggt act aca gtg ggg gtg gtt tgt gcc aca gac aga gat gaa        816
Arg Pro Gly Thr Thr Val Gly Val Val Cys Ala Thr Asp Arg Asp Glu
            260                 265                 270

ccg gac aca atg cat acg cgc ctg aaa tac agc att ttg cag cag aca        864
Pro Asp Thr Met His Thr Arg Leu Lys Tyr Ser Ile Leu Gln Gln Thr
        275                 280                 285

cca agg tca cct ggg ctc ttt tct gtg cat ccc agc aca ggc gta atc        912
Pro Arg Ser Pro Gly Leu Phe Ser Val His Pro Ser Thr Gly Val Ile
    290                 295                 300

acc aca gtc tct cat tat ttg gac aga gag gtt gta gac aag tac tca        960
Thr Thr Val Ser His Tyr Leu Asp Arg Glu Val Val Asp Lys Tyr Ser
305                 310                 315                 320

ttg ata atg aaa gta caa gac atg gat ggc cag ttt ttt gga ttg ata        1008
Leu Ile Met Lys Val Gln Asp Met Asp Gly Gln Phe Phe Gly Leu Ile
                325                 330                 335

ggc aca tca act tgt atc ata aca gta aca gat tca aat gat aat gca        1056
Gly Thr Ser Thr Cys Ile Ile Thr Val Thr Asp Ser Asn Asp Asn Ala
            340                 345                 350

ccc act ttc aga caa aat gct tat gaa gca ttt gta gag gaa aat gca        1104
Pro Thr Phe Arg Gln Asn Ala Tyr Glu Ala Phe Val Glu Glu Asn Ala
        355                 360                 365

ttc aat gtg gaa atc tta cga ata cct ata gaa gat aag gat tta att        1152
Phe Asn Val Glu Ile Leu Arg Ile Pro Ile Glu Asp Lys Asp Leu Ile

370                 375                 380

aac act gcc aat tgg aga gtc aat ttt acc att tta aag gga aat gaa     1200
Asn Thr Ala Asn Trp Arg Val Asn Phe Thr Ile Leu Lys Gly Asn Glu
385                 390                 395                 400

aat gga cat ttc aaa atc agc aca gac aaa gaa act aat gaa ggt gtt     1248
Asn Gly His Phe Lys Ile Ser Thr Asp Lys Glu Thr Asn Glu Gly Val
                405                 410                 415

ctt tct gtt gta aag cca ctg aat tat gaa gaa aac cgt caa gtg aac     1296
Leu Ser Val Val Lys Pro Leu Asn Tyr Glu Glu Asn Arg Gln Val Asn
            420                 425                 430

ctg gaa att gga gta aac aat gaa gcg cca ttt gct aga gat att ccc     1344
Leu Glu Ile Gly Val Asn Asn Glu Ala Pro Phe Ala Arg Asp Ile Pro
        435                 440                 445

aga gtg aca gcc ttg aac aga gcc ttg gtt aca gtt cat gtg agg gat     1392
Arg Val Thr Ala Leu Asn Arg Ala Leu Val Thr Val His Val Arg Asp
    450                 455                 460

ctg gat gag ggg cct gaa tgc act cct gca gcc caa tat gtg cgg att     1440
Leu Asp Glu Gly Pro Glu Cys Thr Pro Ala Ala Gln Tyr Val Arg Ile
465                 470                 475                 480

aaa gaa aac tta gca gtg ggg tca aag atc aac ggc tat aag gca tat     1488
Lys Glu Asn Leu Ala Val Gly Ser Lys Ile Asn Gly Tyr Lys Ala Tyr
                485                 490                 495

gac ccc gaa aat aga aat ggc aat ggt tta agg tac aaa aaa ttg cat     1536
Asp Pro Glu Asn Arg Asn Gly Asn Gly Leu Arg Tyr Lys Lys Leu His
            500                 505                 510

gat cct aaa ggt tgg atc acc att gat gaa att tca ggg tca atc ata     1584
Asp Pro Lys Gly Trp Ile Thr Ile Asp Glu Ile Ser Gly Ser Ile Ile
        515                 520                 525

act tcc aaa atc ctg gat agg gag gtt gaa act ccc aaa aat gag ttg     1632
Thr Ser Lys Ile Leu Asp Arg Glu Val Glu Thr Pro Lys Asn Glu Leu

530                 535                 540

tat aat att aca gtc ctg gca ata gac aaa gat gat aga tca tgt act        1680
Tyr Asn Ile Thr Val Leu Ala Ile Asp Lys Asp Asp Arg Ser Cys Thr
545                 550                 555                 560

gga aca ctt gct gtg aac att gaa gat gta aat gat aat cca cca gaa        1728
Gly Thr Leu Ala Val Asn Ile Glu Asp Val Asn Asp Asn Pro Pro Glu
                565                 570                 575

ata ctt caa gaa tat gta gtc att tgc aaa cca aaa atg ggg tat acc        1776
Ile Leu Gln Glu Tyr Val Val Ile Cys Lys Pro Lys Met Gly Tyr Thr
            580                 585                 590

gac att tta gct gtt gat cct gat gaa cct gtc cat gga gct cca ttt        1824
Asp Ile Leu Ala Val Asp Pro Asp Glu Pro Val His Gly Ala Pro Phe
        595                 600                 605

tat ttc agt ttg ccc aat act tct cca gaa atc agt aga ctg tgg agc        1872
Tyr Phe Ser Leu Pro Asn Thr Ser Pro Glu Ile Ser Arg Leu Trp Ser
    610                 615                 620

ctc acc aaa gtt aat gat aca gct gcc cgt ctt tca tat cag aaa aat        1920
Leu Thr Lys Val Asn Asp Thr Ala Ala Arg Leu Ser Tyr Gln Lys Asn
625                 630                 635                 640

gct gga ttt caa gaa tat acc att cct att act gta aaa gac agg gcc        1968
Ala Gly Phe Gln Glu Tyr Thr Ile Pro Ile Thr Val Lys Asp Arg Ala
                645                 650                 655

ggc caa gct gca aca aaa tta ttg aga gtt aat ctg tgt gaa tgt act        2016
Gly Gln Ala Ala Thr Lys Leu Leu Arg Val Asn Leu Cys Glu Cys Thr
            660                 665                 670

cat cca act cag tgt cgt gcg act tca agg agt aca gga gta ata ctt        2064
His Pro Thr Gln Cys Arg Ala Thr Ser Arg Ser Thr Gly Val Ile Leu
        675                 680                 685

gga aaa tgg gca atc ctt gca ata tta ctg ggt ata gca ctg ctc ttt        2112
Gly Lys Trp Ala Ile Leu Ala Ile Leu Leu Gly Ile Ala Leu Leu Phe

690                 695                 700

tct gta ttg cta act tta gta tgt gga gtt ttt ggt gca act aaa ggg     2160
Ser Val Leu Leu Thr Leu Val Cys Gly Val Phe Gly Ala Thr Lys Gly
705                 710                 715                 720

aaa cgt ttt cct gaa gat tta gca cag caa aac tta att ata tca aac     2208
Lys Arg Phe Pro Glu Asp Leu Ala Gln Gln Asn Leu Ile Ile Ser Asn
                725                 730                 735

aca gaa gca cct gga gac gat aga gtg tgc tct gcc aat gga ttt atg     2256
Thr Glu Ala Pro Gly Asp Asp Arg Val Cys Ser Ala Asn Gly Phe Met
            740                 745                 750

acc caa act acc aac aac tct agc caa ggt ttt tgt ggt act atg gga     2304
Thr Gln Thr Thr Asn Asn Ser Ser Gln Gly Phe Cys Gly Thr Met Gly
        755                 760                 765

tca gga atg aaa aat gga ggg cag gaa acc att gaa atg atg aaa gga     2352
Ser Gly Met Lys Asn Gly Gly Gln Glu Thr Ile Glu Met Met Lys Gly
    770                 775                 780

gga aac cag acc ttg gaa tcc tgc cgg ggg gct ggg cat cat cat acc     2400
Gly Asn Gln Thr Leu Glu Ser Cys Arg Gly Ala Gly His His His Thr
785                 790                 795                 800

ctg gac tcc tgc agg gga gga cac acg gag gtg gac aac tgc aga tac     2448
Leu Asp Ser Cys Arg Gly Gly His Thr Glu Val Asp Asn Cys Arg Tyr
                805                 810                 815

act tac tcg gag tgg cac agt ttt act caa ccc cgt ctc ggt gaa aaa     2496
Thr Tyr Ser Glu Trp His Ser Phe Thr Gln Pro Arg Leu Gly Glu Lys
            820                 825                 830

ttg cat cga tgt aat cag aat gaa gac cgc atg cca tcc caa gat tat     2544
Leu His Arg Cys Asn Gln Asn Glu Asp Arg Met Pro Ser Gln Asp Tyr
        835                 840                 845

gtc ctc act tat aac tat gag gga aga gga tct cca gct ggt tct gtg     2592
Val Leu Thr Tyr Asn Tyr Glu Gly Arg Gly Ser Pro Ala Gly Ser Val

850                 855                 860

ggc tgc tgc agt gaa aag cag gaa gaa gat ggc ctt gac ttt tta aat        2640
Gly Cys Cys Ser Glu Lys Gln Glu Glu Asp Gly Leu Asp Phe Leu Asn
865                 870                 875                 880

aat ttg gaa ccc aaa ttt att aca tta gca gaa gca tgc aca aag aga        2688
Asn Leu Glu Pro Lys Phe Ile Thr Leu Ala Glu Ala Cys Thr Lys Arg
                885                 890                 895

<210>  2
<211>  896
<212>  PRT
<213>  Homo sapiens

<400>  2

Met Ala Ala Ala Gly Pro Arg Arg Ser Val Arg Gly Ala Val Cys Leu
1               5                   10                  15

His Leu Leu Leu Thr Leu Val Ile Phe Ser Arg Asp Gly Glu Ala Cys
            20                  25                  30

Lys Lys Val Ile Leu Asn Val Pro Ser Lys Leu Glu Ala Asp Lys Ile
        35                  40                  45

Ile Gly Arg Val Asn Leu Glu Glu Cys Phe Arg Ser Ala Asp Leu Ile
    50                  55                  60

Arg Ser Ser Asp Pro Asp Phe Arg Val Leu Asn Asp Gly Ser Val Tyr
65                  70                  75                  80

Thr Ala Arg Ala Val Ala Leu Ser Asp Lys Lys Arg Ser Phe Thr Ile
                85                  90                  95

Trp Leu Ser Asp Lys Arg Lys Gln Thr Gln Lys Glu Val Thr Val Leu
            100                 105                 110

Leu Glu His Gln Lys Lys Val Ser Lys Thr Arg His Thr Arg Glu Thr
        115                 120                 125

Val Leu Arg Arg Ala Lys Arg Arg Trp Ala Pro Ile Pro Cys Ser Met
    130                 135                 140

Gln Glu Asn Ser Leu Gly Pro Phe Pro Leu Phe Leu Gln Gln Val Glu
145                 150                 155                 160

Ser Asp Ala Ala Gln Asn Tyr Thr Val Phe Tyr Ser Ile Ser Gly Arg
                165                 170                 175

Gly Val Asp Lys Glu Pro Leu Asn Leu Phe Tyr Ile Glu Arg Asp Thr
            180                 185                 190

Gly Asn Leu Phe Cys Thr Arg Pro Val Asp Arg Glu Glu Tyr Asp Val
        195                 200                 205

Phe Asp Leu Ile Ala Tyr Ala Ser Thr Ala Asp Gly Tyr Ser Ala Asp
    210                 215                 220

Leu Pro Leu Pro Leu Pro Ile Arg Val Glu Asp Glu Asn Asp Asn His
225                 230                 235                 240

Pro Val Phe Thr Glu Ala Ile Tyr Asn Phe Glu Val Leu Glu Ser Ser
                245                 250                 255

Arg Pro Gly Thr Thr Val Gly Val Val Cys Ala Thr Asp Arg Asp Glu
            260                 265                 270

Pro Asp Thr Met His Thr Arg Leu Lys Tyr Ser Ile Leu Gln Gln Thr
        275                 280                 285

Pro Arg Ser Pro Gly Leu Phe Ser Val His Pro Ser Thr Gly Val Ile
    290                 295                 300

Thr Thr Val Ser His Tyr Leu Asp Arg Glu Val Val Asp Lys Tyr Ser
305                 310                 315                 320

Leu Ile Met Lys Val Gln Asp Met Asp Gly Gln Phe Phe Gly Leu Ile
                325                 330                 335

Gly Thr Ser Thr Cys Ile Ile Thr Val Thr Asp Ser Asn Asp Asn Ala
            340                 345                 350

Pro Thr Phe Arg Gln Asn Ala Tyr Glu Ala Phe Val Glu Glu Asn Ala
        355                 360                 365

Phe Asn Val Glu Ile Leu Arg Ile Pro Ile Glu Asp Lys Asp Leu Ile
    370                 375                 380

Asn Thr Ala Asn Trp Arg Val Asn Phe Thr Ile Leu Lys Gly Asn Glu
385                 390                 395                 400

Asn Gly His Phe Lys Ile Ser Thr Asp Lys Glu Thr Asn Glu Gly Val
                405                 410                 415

Leu Ser Val Val Lys Pro Leu Asn Tyr Glu Glu Asn Arg Gln Val Asn
            420                 425                 430

Leu Glu Ile Gly Val Asn Asn Glu Ala Pro Phe Ala Arg Asp Ile Pro
        435                 440                 445

Arg Val Thr Ala Leu Asn Arg Ala Leu Val Thr Val His Val Arg Asp
    450                 455                 460

Leu Asp Glu Gly Pro Glu Cys Thr Pro Ala Ala Gln Tyr Val Arg Ile
465                 470                 475                 480

Lys Glu Asn Leu Ala Val Gly Ser Lys Ile Asn Gly Tyr Lys Ala Tyr
                485                 490                 495

Asp Pro Glu Asn Arg Asn Gly Asn Gly Leu Arg Tyr Lys Lys Leu His
            500                 505                 510

Asp Pro Lys Gly Trp Ile Thr Ile Asp Glu Ile Ser Gly Ser Ile Ile
        515                 520                 525

Thr Ser Lys Ile Leu Asp Arg Glu Val Glu Thr Pro Lys Asn Glu Leu
    530                 535                 540

Tyr Asn Ile Thr Val Leu Ala Ile Asp Lys Asp Asp Arg Ser Cys Thr
545                 550                 555                 560

Gly Thr Leu Ala Val Asn Ile Glu Asp Val Asn Asp Asn Pro Pro Glu
                565                 570                 575

Ile Leu Gln Glu Tyr Val Val Ile Cys Lys Pro Lys Met Gly Tyr Thr
            580                 585                 590

Asp Ile Leu Ala Val Asp Pro Asp Glu Pro Val His Gly Ala Pro Phe
        595                 600                 605

Tyr Phe Ser Leu Pro Asn Thr Ser Pro Glu Ile Ser Arg Leu Trp Ser
    610                 615                 620

Leu Thr Lys Val Asn Asp Thr Ala Ala Arg Leu Ser Tyr Gln Lys Asn
625                 630                 635                 640

Ala Gly Phe Gln Glu Tyr Thr Ile Pro Ile Thr Val Lys Asp Arg Ala
                645                 650                 655

Gly Gln Ala Ala Thr Lys Leu Leu Arg Val Asn Leu Cys Glu Cys Thr
            660                 665                 670

His Pro Thr Gln Cys Arg Ala Thr Ser Arg Ser Thr Gly Val Ile Leu
        675                 680                 685

Gly Lys Trp Ala Ile Leu Ala Ile Leu Leu Gly Ile Ala Leu Leu Phe
    690                 695                 700

Ser Val Leu Leu Thr Leu Val Cys Gly Val Phe Gly Ala Thr Lys Gly
705                 710                 715                 720

Lys Arg Phe Pro Glu Asp Leu Ala Gln Gln Asn Leu Ile Ile Ser Asn
                725                 730                 735

Thr Glu Ala Pro Gly Asp Asp Arg Val Cys Ser Ala Asn Gly Phe Met
        740                 745                 750

Thr Gln Thr Thr Asn Asn Ser Ser Gln Gly Phe Cys Gly Thr Met Gly
    755                 760                 765

Ser Gly Met Lys Asn Gly Gly Gln Glu Thr Ile Glu Met Met Lys Gly
    770                 775                 780

Gly Asn Gln Thr Leu Glu Ser Cys Arg Gly Ala Gly His His His Thr
785                 790                 795                 800

Leu Asp Ser Cys Arg Gly Gly His Thr Glu Val Asp Asn Cys Arg Tyr
                805                 810                 815

Thr Tyr Ser Glu Trp His Ser Phe Thr Gln Pro Arg Leu Gly Glu Lys
            820                 825                 830

Leu His Arg Cys Asn Gln Asn Glu Asp Arg Met Pro Ser Gln Asp Tyr
        835                 840                 845

Val Leu Thr Tyr Asn Tyr Glu Gly Arg Gly Ser Pro Ala Gly Ser Val
    850                 855                 860

Gly Cys Cys Ser Glu Lys Gln Glu Glu Asp Gly Leu Asp Phe Leu Asn
865                 870                 875                 880

Asn Leu Glu Pro Lys Phe Ile Thr Leu Ala Glu Ala Cys Thr Lys Arg
                885                 890                 895

<210> 3
<211> 2517
<212> DNA
<213> Homo sapiens

<220>
<221> CDS
<222> (1)..(2517)

<400> 3
atg gcc gcc gct ggg ccc cgg cgc tcc gtg cgc gga gcc gtc tgc ctg 48
Met Ala Ala Ala Gly Pro Arg Arg Ser Val Arg Gly Ala Val Cys Leu
1               5                   10                  15

cat ctg ctg ctg acc ctc gtg atc ttc agt cgt gat ggt gaa gcc tgc 96
His Leu Leu Leu Thr Leu Val Ile Phe Ser Arg Asp Gly Glu Ala Cys
            20                  25                  30

aaa aag gtg ata ctt aat gta cct tct aaa cta gag gca gac aaa ata 144
Lys Lys Val Ile Leu Asn Val Pro Ser Lys Leu Glu Ala Asp Lys Ile
        35                  40                  45

att ggc aga gtt aat ttg gaa gag tgc ttc agg tct gca gac ctc atc 192
Ile Gly Arg Val Asn Leu Glu Glu Cys Phe Arg Ser Ala Asp Leu Ile
    50                  55                  60

cgg tca agt gat cct gat ttc aga gtt cta aat gat ggg tca gtg tac 240
Arg Ser Ser Asp Pro Asp Phe Arg Val Leu Asn Asp Gly Ser Val Tyr
65                  70                  75                  80

aca gcc agg gct gtt gcg ctg tct gat aag aaa aga tca ttt acc ata 288
Thr Ala Arg Ala Val Ala Leu Ser Asp Lys Lys Arg Ser Phe Thr Ile
                85                  90                  95

tgg ctt tct gac aaa agg aaa cag aca cag aaa gag gtt act gtg ctg 336
Trp Leu Ser Asp Lys Arg Lys Gln Thr Gln Lys Glu Val Thr Val Leu
            100                 105                 110

cta gaa cat cag aag aag gta tcg aag aca aga cac act aga gaa act 384
Leu Glu His Gln Lys Lys Val Ser Lys Thr Arg His Thr Arg Glu Thr
        115                 120                 125

gtt ctc agg cgt gcc aag agg aga tgg gca cct att cct tgc tct atg 432
Val Leu Arg Arg Ala Lys Arg Arg Trp Ala Pro Ile Pro Cys Ser Met
    130                 135                 140

caa gag aat tcc ttg ggc cct ttc cca ttg ttt ctt caa caa gtt gaa 480
Gln Glu Asn Ser Leu Gly Pro Phe Pro Leu Phe Leu Gln Gln Val Glu
145                 150                 155                 160

tct gat gca gca cag aac tat act gtc ttc tac tca ata agt gga cgt 528
Ser Asp Ala Ala Gln Asn Tyr Thr Val Phe Tyr Ser Ile Ser Gly Arg
                165                 170                 175

gga gtt gat aaa gaa cct tta aat ttg ttt tat ata gaa aga gac act 576
Gly Val Asp Lys Glu Pro Leu Asn Leu Phe Tyr Ile Glu Arg Asp Thr
            180                 185                 190

gga aat cta ttt tgc act cgg cct gtg gat cgt gaa gaa tat gat gtt 624
Gly Asn Leu Phe Cys Thr Arg Pro Val Asp Arg Glu Glu Tyr Asp Val
        195                 200                 205

ttt gat ttg att gct tat gcg tca act gca gat gga tat tca gca gat 672
Phe Asp Leu Ile Ala Tyr Ala Ser Thr Ala Asp Gly Tyr Ser Ala Asp
    210                 215                 220

ctg ccc ctc cca cta ccc atc agg gta gag gat gaa aat gac aac cac 720
Leu Pro Leu Pro Leu Pro Ile Arg Val Glu Asp Glu Asn Asp Asn His
225                 230                 235                 240

cct gtt ttc aca gaa gca att tat aat ttt gaa gtt ttg gaa agt agt 768
Pro Val Phe Thr Glu Ala Ile Tyr Asn Phe Glu Val Leu Glu Ser Ser
                245                 250                 255

aga cct ggt act aca gtg ggg gtg gtt tgt gcc aca gac aga gat gaa 816
Arg Pro Gly Thr Thr Val Gly Val Val Cys Ala Thr Asp Arg Asp Glu
            260                 265                 270

ccg gac aca atg cat acg cgc ctg aaa tac agc att ttg cag cag aca 864
Pro Asp Thr Met His Thr Arg Leu Lys Tyr Ser Ile Leu Gln Gln Thr
        275                 280                 285

cca agg tca cct ggg ctc ttt tct gtg cat ccc agc aca ggc gta atc 912
Pro Arg Ser Pro Gly Leu Phe Ser Val His Pro Ser Thr Gly Val Ile
    290                 295                 300

acc aca gtc tct cat tat ttg gac aga gag gtt gta gac aag tac tca 960
Thr Thr Val Ser His Tyr Leu Asp Arg Glu Val Val Asp Lys Tyr Ser
305                 310                 315                 320

ttg ata atg aaa gta caa gac atg gat ggc cag ttt ttt gga ttg ata 1008
Leu Ile Met Lys Val Gln Asp Met Asp Gly Gln Phe Phe Gly Leu Ile
                325                 330                 335

ggc aca tca act tgt atc ata aca gta aca gat tca aat gat aat gca 1056
Gly Thr Ser Thr Cys Ile Ile Thr Val Thr Asp Ser Asn Asp Asn Ala
            340                 345                 350

ccc act ttc aga caa aat gct tat gaa gca ttt gta gag gaa aat gca 1104
Pro Thr Phe Arg Gln Asn Ala Tyr Glu Ala Phe Val Glu Glu Asn Ala
        355                 360                 365

ttc aat gtg gaa atc tta cga ata cct ata gaa gat aag gat tta att 1152
Phe Asn Val Glu Ile Leu Arg Ile Pro Ile Glu Asp Lys Asp Leu Ile
    370                 375                 380

aac act gcc aat tgg aga gtc aat ttt acc att tta aag gga aat gaa 1200
Asn Thr Ala Asn Trp Arg Val Asn Phe Thr Ile Leu Lys Gly Asn Glu
385                 390                 395                 400

aat gga cat ttc aaa atc agc aca gac aaa gaa act aat gaa ggt gtt 1248
Asn Gly His Phe Lys Ile Ser Thr Asp Lys Glu Thr Asn Glu Gly Val
                405                 410                 415

ctt tct gtt gta aag cca ctg aat tat gaa gaa aac cgt caa gtg aac 1296
Leu Ser Val Val Lys Pro Leu Asn Tyr Glu Glu Asn Arg Gln Val Asn
            420                 425                 430

ctg gaa att gga gta aac aat gaa gcg cca ttt gct aga gat att ccc 1344
Leu Glu Ile Gly Val Asn Asn Glu Ala Pro Phe Ala Arg Asp Ile Pro
        435                 440                 445

aga gtg aca gcc ttg aac aga gcc ttg gtt aca gtt cat gtg agg gat 1392
Arg Val Thr Ala Leu Asn Arg Ala Leu Val Thr Val His Val Arg Asp
    450                 455                 460

ctg gat gag ggg cct gaa tgc act cct gca gcc caa tat gtg cgg att 1440
Leu Asp Glu Gly Pro Glu Cys Thr Pro Ala Ala Gln Tyr Val Arg Ile
465                 470                 475                 480

aaa gaa aac tta gca gtg ggg tca aag atc aac ggc tat aag gca tat 1488
Lys Glu Asn Leu Ala Val Gly Ser Lys Ile Asn Gly Tyr Lys Ala Tyr
                485                 490                 495

gac ccc gaa aat aga aat ggc aat ggt tta agg tac aaa aaa ttg cat 1536
Asp Pro Glu Asn Arg Asn Gly Asn Gly Leu Arg Tyr Lys Lys Leu His
            500                 505                 510

gat cct aaa ggt tgg atc acc att gat gaa att tca ggg tca atc ata 1584
Asp Pro Lys Gly Trp Ile Thr Ile Asp Glu Ile Ser Gly Ser Ile Ile
        515                 520                 525

act tcc aaa atc ctg gat agg gag gtt gaa act ccc aaa aat gag ttg 1632
Thr Ser Lys Ile Leu Asp Arg Glu Val Glu Thr Pro Lys Asn Glu Leu
    530                 535                 540

tat aat att aca gtc ctg gca ata gac aaa gat gat aga tca tgt act 1680
Tyr Asn Ile Thr Val Leu Ala Ile Asp Lys Asp Asp Arg Ser Cys Thr
545                 550                 555                 560

gga aca ctt gct gtg aac att gaa gat gta aat gat aat cca cca gaa 1728
Gly Thr Leu Ala Val Asn Ile Glu Asp Val Asn Asp Asn Pro Pro Glu
                565                 570                 575

ata ctt caa gaa tat gta gtc att tgc aaa cca aaa atg ggg tat acc 1776
Ile Leu Gln Glu Tyr Val Val Ile Cys Lys Pro Lys Met Gly Tyr Thr
            580                 585                 590

gac att tta gct gtt gat cct gat gaa cct gtc cat gga gct cca ttt 1824
Asp Ile Leu Ala Val Asp Pro Asp Glu Pro Val His Gly Ala Pro Phe
        595                 600                 605

tat ttc agt ttg ccc aat act tct cca gaa atc agt aga ctg tgg agc 1872
Tyr Phe Ser Leu Pro Asn Thr Ser Pro Glu Ile Ser Arg Leu Trp Ser
    610                 615                 620

ctc acc aaa gtt aat gat aca gct gcc cgt ctt tca tat cag aaa aat 1920
Leu Thr Lys Val Asn Asp Thr Ala Ala Arg Leu Ser Tyr Gln Lys Asn
625                 630                 635                 640

gct gga ttt caa gaa tat acc att cct att act gta aaa gac agg gcc 1968
Ala Gly Phe Gln Glu Tyr Thr Ile Pro Ile Thr Val Lys Asp Arg Ala
                645                 650                 655

ggc caa gct gca aca aaa tta ttg aga gtt aat ctg tgt gaa tgt act 2016
Gly Gln Ala Ala Thr Lys Leu Leu Arg Val Asn Leu Cys Glu Cys Thr
            660                 665                 670

cat cca act cag tgt cgt gcg act tca agg agt aca gga gta ata ctt 2064
His Pro Thr Gln Cys Arg Ala Thr Ser Arg Ser Thr Gly Val Ile Leu
        675                 680                 685

gga aaa tgg gca atc ctt gca ata tta ctg ggt ata gca ctg ctc ttt 2112
Gly Lys Trp Ala Ile Leu Ala Ile Leu Leu Gly Ile Ala Leu Leu Phe
    690                 695                 700

tct gta ttg cta act tta gta tgt gga gtt ttt ggt gca act aaa ggg 2160
Ser Val Leu Leu Thr Leu Val Cys Gly Val Phe Gly Ala Thr Lys Gly
705                 710                 715                 720

aaa cgt ttt cct gaa gat tta gca cag caa aac tta att ata tca aac 2208
Lys Arg Phe Pro Glu Asp Leu Ala Gln Gln Asn Leu Ile Ile Ser Asn
                725                 730                 735

aca gaa gca cct gga gac gat aga gtg tgc tct gcc aat gga ttt atg 2256
Thr Glu Ala Pro Gly Asp Asp Arg Val Cys Ser Ala Asn Gly Phe Met
            740                 745                 750

acc caa act acc aac aac tct agc caa ggt ttt tgt ggt act atg gga     2304
Thr Gln Thr Thr Asn Asn Ser Ser Gln Gly Phe Cys Gly Thr Met Gly
        755                 760                 765

tca gga atg aaa aat gga ggg cag gaa acc att gaa atg atg aaa gga     2352
Ser Gly Met Lys Asn Gly Gly Gln Glu Thr Ile Glu Met Met Lys Gly
    770                 775                 780

gga aac cag acc ttg gaa tcc tgc cgg ggg gct ggg cat cat cat acc     2400
Gly Asn Gln Thr Leu Glu Ser Cys Arg Gly Ala Gly His His His Thr
785                 790                 795                 800

ctg gac tcc tgc agg gga gga cac acg gag gtg gac aac tgc aga tac     2448
Leu Asp Ser Cys Arg Gly Gly His Thr Glu Val Asp Asn Cys Arg Tyr
                805                 810                 815

act tac tcg gag tgg cac agt ttt act caa ccc cgt ctc ggt gaa gaa     2496
Thr Tyr Ser Glu Trp His Ser Phe Thr Gln Pro Arg Leu Gly Glu Glu
            820                 825                 830

tcc att aga gga cac act ggt                                         2517
Ser Ile Arg Gly His Thr Gly
        835

<210>  4
<211>  839
<212>  PRT
<213>  Homo sapiens

<400>  4

Met Ala Ala Ala Gly Pro Arg Arg Ser Val Arg Gly Ala Val Cys Leu
1               5                   10                  15

His Leu Leu Leu Thr Leu Val Ile Phe Ser Arg Asp Gly Glu Ala Cys
            20                  25                  30

Lys Lys Val Ile Leu Asn Val Pro Ser Lys Leu Glu Ala Asp Lys Ile
        35                  40                  45

Ile Gly Arg Val Asn Leu Glu Glu Cys Phe Arg Ser Ala Asp Leu Ile
    50                  55                  60

Arg Ser Ser Asp Pro Asp Phe Arg Val Leu Asn Asp Gly Ser Val Tyr
65                  70                  75                  80

Thr Ala Arg Ala Val Ala Leu Ser Asp Lys Lys Arg Ser Phe Thr Ile
                85                  90                  95

Trp Leu Ser Asp Lys Arg Lys Gln Thr Gln Lys Glu Val Thr Val Leu
            100                 105                 110

Leu Glu His Gln Lys Lys Val Ser Lys Thr Arg His Thr Arg Glu Thr
        115                 120                 125

Val Leu Arg Arg Ala Lys Arg Arg Trp Ala Pro Ile Pro Cys Ser Met
    130                 135                 140

Gln Glu Asn Ser Leu Gly Pro Phe Pro Leu Phe Leu Gln Gln Val Glu
145                 150                 155                 160

Ser Asp Ala Ala Gln Asn Tyr Thr Val Phe Tyr Ser Ile Ser Gly Arg
                165                 170                 175

Gly Val Asp Lys Glu Pro Leu Asn Leu Phe Tyr Ile Glu Arg Asp Thr
            180                 185                 190

Gly Asn Leu Phe Cys Thr Arg Pro Val Asp Arg Glu Glu Tyr Asp Val
        195                 200                 205

Phe Asp Leu Ile Ala Tyr Ala Ser Thr Ala Asp Gly Tyr Ser Ala Asp
    210                 215                 220

Leu Pro Leu Pro Leu Pro Ile Arg Val Glu Asp Glu Asn Asp Asn His
225                 230                 235                 240

Pro Val Phe Thr Glu Ala Ile Tyr Asn Phe Glu Val Leu Glu Ser Ser
                245                 250                 255

Arg Pro Gly Thr Thr Val Gly Val Val Cys Ala Thr Asp Arg Asp Glu
            260                 265                 270

Pro Asp Thr Met His Thr Arg Leu Lys Tyr Ser Ile Leu Gln Gln Thr
        275                 280                 285

Pro Arg Ser Pro Gly Leu Phe Ser Val His Pro Ser Thr Gly Val Ile
    290                 295                 300

Thr Thr Val Ser His Tyr Leu Asp Arg Glu Val Val Asp Lys Tyr Ser
305                 310                 315                 320

Leu Ile Met Lys Val Gln Asp Met Asp Gly Gln Phe Phe Gly Leu Ile
                325                 330                 335

Gly Thr Ser Thr Cys Ile Ile Thr Val Thr Asp Ser Asn Asp Asn Ala
            340                 345                 350

Pro Thr Phe Arg Gln Asn Ala Tyr Glu Ala Phe Val Glu Glu Asn Ala
        355                 360                 365

Phe Asn Val Glu Ile Leu Arg Ile Pro Ile Glu Asp Lys Asp Leu Ile
    370                 375                 380

Asn Thr Ala Asn Trp Arg Val Asn Phe Thr Ile Leu Lys Gly Asn Glu
385                 390                 395                 400

Asn Gly His Phe Lys Ile Ser Thr Asp Lys Glu Thr Asn Glu Gly Val
                405                 410                 415

Leu Ser Val Val Lys Pro Leu Asn Tyr Glu Glu Asn Arg Gln Val Asn
            420                 425                 430

Leu Glu Ile Gly Val Asn Asn Glu Ala Pro Phe Ala Arg Asp Ile Pro
        435                 440                 445

Arg Val Thr Ala Leu Asn Arg Ala Leu Val Thr Val His Val Arg Asp
    450                 455                 460

Leu Asp Glu Gly Pro Glu Cys Thr Pro Ala Ala Gln Tyr Val Arg Ile
465                 470                 475                 480

Lys Glu Asn Leu Ala Val Gly Ser Lys Ile Asn Gly Tyr Lys Ala Tyr
                485                 490                 495

Asp Pro Glu Asn Arg Asn Gly Asn Gly Leu Arg Tyr Lys Lys Leu His
            500                 505                 510

Asp Pro Lys Gly Trp Ile Thr Ile Asp Glu Ile Ser Gly Ser Ile Ile
        515                 520                 525

Thr Ser Lys Ile Leu Asp Arg Glu Val Glu Thr Pro Lys Asn Glu Leu
    530                 535                 540

Tyr Asn Ile Thr Val Leu Ala Ile Asp Lys Asp Asp Arg Ser Cys Thr
545                 550                 555                 560

Gly Thr Leu Ala Val Asn Ile Glu Asp Val Asn Asp Asn Pro Pro Glu
                565                 570                 575

Ile Leu Gln Glu Tyr Val Val Ile Cys Lys Pro Lys Met Gly Tyr Thr
            580                 585                 590

Asp Ile Leu Ala Val Asp Pro Asp Glu Pro Val His Gly Ala Pro Phe
        595                 600                 605

Tyr Phe Ser Leu Pro Asn Thr Ser Pro Glu Ile Ser Arg Leu Trp Ser
    610                 615                 620

Leu Thr Lys Val Asn Asp Thr Ala Ala Arg Leu Ser Tyr Gln Lys Asn
625                 630                 635                 640

Ala Gly Phe Gln Glu Tyr Thr Ile Pro Ile Thr Val Lys Asp Arg Ala
                645                 650                 655

Gly Gln Ala Ala Thr Lys Leu Leu Arg Val Asn Leu Cys Glu Cys Thr
            660                 665                 670

His Pro Thr Gln Cys Arg Ala Thr Ser Arg Ser Thr Gly Val Ile Leu
        675                 680                 685

Gly Lys Trp Ala Ile Leu Ala Ile Leu Leu Gly Ile Ala Leu Leu Phe
    690                 695                 700

Ser Val Leu Leu Thr Leu Val Cys Gly Val Phe Gly Ala Thr Lys Gly
705                 710                 715                 720

Lys Arg Phe Pro Glu Asp Leu Ala Gln Gln Asn Leu Ile Ile Ser Asn
                725                 730                 735

Thr Glu Ala Pro Gly Asp Asp Arg Val Cys Ser Ala Asn Gly Phe Met
            740                 745                 750

Thr Gln Thr Thr Asn Asn Ser Ser Gln Gly Phe Cys Gly Thr Met Gly
        755                 760                 765

Ser Gly Met Lys Asn Gly Gly Gln Glu Thr Ile Glu Met Met Lys Gly
    770                 775                 780

Gly Asn Gln Thr Leu Glu Ser Cys Arg Gly Ala Gly His His His Thr
785                 790                 795                 800

Leu Asp Ser Cys Arg Gly Gly His Thr Glu Val Asp Asn Cys Arg Tyr
                805                 810                 815

Thr Tyr Ser Glu Trp His Ser Phe Thr Gln Pro Arg Leu Gly Glu Glu
            820                 825                 830

Ser Ile Arg Gly His Thr Gly
        835

<210>  5
<211>  612
<212>  DNA
<213>  Homo sapiens

<220>
<221>  CDS
<222>  (1)..(612)

<400>  5
atg gtt tgc ggg ggc ttc gcg tgt tcc aag aac tgc ctg tgc gcc ctc        48
Met Val Cys Gly Gly Phe Ala Cys Ser Lys Asn Cys Leu Cys Ala Leu
1               5                   10                  15

aac ctg ctt tac acc ttg gtt agt ctg ctg cta att gga att gct gcg        96
Asn Leu Leu Tyr Thr Leu Val Ser Leu Leu Leu Ile Gly Ile Ala Ala
            20                  25                  30

tgg ggc att ggc ttc ggg ctg att tcc agt ctc cga gtg gtc ggc gtg        144
Trp Gly Ile Gly Phe Gly Leu Ile Ser Ser Leu Arg Val Val Gly Val
        35                  40                  45

gtc att gca gtg ggc atc ttc ttg ttc ctg att gct tta gtg ggt ctg        192
Val Ile Ala Val Gly Ile Phe Leu Phe Leu Ile Ala Leu Val Gly Leu
    50                  55                  60

att gga gct gta aaa cat cat cag gtg ttg cta ttt ttt tat atg att        240
Ile Gly Ala Val Lys His His Gln Val Leu Leu Phe Phe Tyr Met Ile
65                  70                  75                  80

att ctg tta ctt gta ttt att gtt cag ttt tct gta tct tgc gct tgt        288
Ile Leu Leu Leu Val Phe Ile Val Gln Phe Ser Val Ser Cys Ala Cys
                85                  90                  95

tta gcc ctg aac cag gag caa cag ggt cag ctt ctg gag gtt ggt tgg 336
Leu Ala Leu Asn Gln Glu Gln Gln Gly Gln Leu Leu Glu Val Gly Trp
            100                 105                 110

aac aat acg gca agt gct cga aat gac atc cag aga aat cta aac tgc 384
Asn Asn Thr Ala Ser Ala Arg Asn Asp Ile Gln Arg Asn Leu Asn Cys
        115                 120                 125

tgt ggg ttc cga agt gtt aac cca aat gac acc tgt ctg gct agc tgt 432
Cys Gly Phe Arg Ser Val Asn Pro Asn Asp Thr Cys Leu Ala Ser Cys
    130                 135                 140

gtt aaa agt gac cac tcg tgc tcg cca tgt gct cca atc ata gga gaa 480
Val Lys Ser Asp His Ser Cys Ser Pro Cys Ala Pro Ile Ile Gly Glu
145                 150                 155                 160

tat gct gga gag gtt ttg aga ttt gtt ggt ggc att ggc ctg ttc ttc 528
Tyr Ala Gly Glu Val Leu Arg Phe Val Gly Gly Ile Gly Leu Phe Phe
                165                 170                 175

agt ttt aca gag atc ctg ggt gtt tgg ctg acc tac aga tac agg aac 576
Ser Phe Thr Glu Ile Leu Gly Val Trp Leu Thr Tyr Arg Tyr Arg Asn
            180                 185                 190

cag aaa gac ccc cgc gcg aat cct agt gca ttc ctt 612
Gln Lys Asp Pro Arg Ala Asn Pro Ser Ala Phe Leu
        195                 200

<210> 6
<211> 204
<212> PRT
<213> Homo sapiens

<400> 6

Met Val Cys Gly Gly Phe Ala Cys Ser Lys Asn Cys Leu Cys Ala Leu
1               5                   10                  15

Asn Leu Leu Tyr Thr Leu Val Ser Leu Leu Leu Ile Gly Ile Ala Ala
            20                  25                  30

Trp Gly Ile Gly Phe Gly Leu Ile Ser Ser Leu Arg Val Val Gly Val
        35                  40                  45

Val Ile Ala Val Gly Ile Phe Leu Phe Leu Ile Ala Leu Val Gly Leu
    50                  55                  60

Ile Gly Ala Val Lys His His Gln Val Leu Leu Phe Phe Tyr Met Ile
65                  70                  75                  80

Ile Leu Leu Leu Val Phe Ile Val Gln Phe Ser Val Ser Cys Ala Cys
                85                  90                  95

Leu Ala Leu Asn Gln Glu Gln Gln Gly Gln Leu Leu Glu Val Gly Trp
            100                 105                 110

Asn Asn Thr Ala Ser Ala Arg Asn Asp Ile Gln Arg Asn Leu Asn Cys
        115                 120                 125

Cys Gly Phe Arg Ser Val Asn Pro Asn Asp Thr Cys Leu Ala Ser Cys
    130                 135                 140

Val Lys Ser Asp His Ser Cys Ser Pro Cys Ala Pro Ile Ile Gly Glu
145                 150                 155                 160

Tyr Ala Gly Glu Val Leu Arg Phe Val Gly Gly Ile Gly Leu Phe Phe
                165                 170                 175

Ser Phe Thr Glu Ile Leu Gly Val Trp Leu Thr Tyr Arg Tyr Arg Asn
            180                 185                 190

Gln Lys Asp Pro Arg Ala Asn Pro Ser Ala Phe Leu
        195                 200

<210>  7
<211>  1095
<212>  DNA
<213>  Homo sapiens

<220>
<221>  CDS
<222>  (1)..(735)

<400>  7
atg gcg tcc ccg tct cgg aga ctg cag act aaa cca gtc att act tgt        48
Met Ala Ser Pro Ser Arg Arg Leu Gln Thr Lys Pro Val Ile Thr Cys
1               5                   10                  15

ttc aag agc gtt ctg cta atc tac act ttt att ttc tgg atc act ggc        96
Phe Lys Ser Val Leu Leu Ile Tyr Thr Phe Ile Phe Trp Ile Thr Gly
            20                  25                  30

gtt atc ctt ctt gca gtt ggc att tgg ggc aag gtg agc ctg gag aat       144
Val Ile Leu Leu Ala Val Gly Ile Trp Gly Lys Val Ser Leu Glu Asn
        35                  40                  45

tac ttt tct ctt tta aat gag aag gcc acc aat gtc ccc ttc gtg ctc       192
Tyr Phe Ser Leu Leu Asn Glu Lys Ala Thr Asn Val Pro Phe Val Leu
    50                  55                  60

att gct act ggt acc gtc att att ctt ttg ggc acc ttt ggt tgt ttt       240
Ile Ala Thr Gly Thr Val Ile Ile Leu Leu Gly Thr Phe Gly Cys Phe
65                  70                  75                  80

gct acc tgc cga gct tct gca tgg atg cta aaa ctg tat gca atg ttt       288

Ala Thr Cys Arg Ala Ser Ala Trp Met Leu Lys Leu Tyr Ala Met Phe
                85                  90                  95

ctg act ctc gtt ttt ttg gtc gaa ctg gtc gct gcc atc gta gga ttt      336
Leu Thr Leu Val Phe Leu Val Glu Leu Val Ala Ala Ile Val Gly Phe
            100                 105                 110

gtt ttc aga cat gag att aag aac atg gcg tcc ccg tct cgg aga ctg      384
Val Phe Arg His Glu Ile Lys Asn Met Ala Ser Pro Ser Arg Arg Leu
        115                 120                 125

cag act aaa cca gtc att act tgt ttc aag agc gtt ctg cta atc tac      432
Gln Thr Lys Pro Val Ile Thr Cys Phe Lys Ser Val Leu Leu Ile Tyr
    130                 135                 140

act ttt att ttc tgg atc act ggc gtt atc ctt ctt gca gtt ggc att      480
Thr Phe Ile Phe Trp Ile Thr Gly Val Ile Leu Leu Ala Val Gly Ile
145                 150                 155                 160

tgg ggc aag gtg agc ctg gag aat tac ttt tct ctt tta aat gag aag      528
Trp Gly Lys Val Ser Leu Glu Asn Tyr Phe Ser Leu Leu Asn Glu Lys
                165                 170                 175

gcc acc aat gtc ccc ttc gtg ctc att gct act ggt acc gtc att att      576
Ala Thr Asn Val Pro Phe Val Leu Ile Ala Thr Gly Thr Val Ile Ile
            180                 185                 190

ctt ttg ggc acc ttt ggt tgt ttt gct acc tgc cga gct tct gca tgg      624
Leu Leu Gly Thr Phe Gly Cys Phe Ala Thr Cys Arg Ala Ser Ala Trp
        195                 200                 205

atg cta aaa ctg tat gca atg ttt ctg act ctc gtt ttt ttg gtc gaa      672
Met Leu Lys Leu Tyr Ala Met Phe Leu Thr Leu Val Phe Leu Val Glu
    210                 215                 220

ctg gtc gct gcc atc gta gga ttt gtt ttc aga cat gag att aag aac      720
Leu Val Ala Ala Ile Val Gly Phe Val Phe Arg His Glu Ile Lys Asn
225                 230                 235                 240

agc ttt aag aat aat tatgagaagg ctttgaagca gtataactct acaggagatt      775

Ser Phe Lys Asn Asn
                245

atagaagcca tgcagtagac aagatccaaa atacgttgca ttgttgtggt gtcaccgatt     835

atagagattg gacagatact aattattact cagaaaaagg atttcctaag agttgctgta     895

aacttgaaga ttgtactcca cagagagatg cagacaaagt aaacaatgaa ggttgtttta     955

taaaggtgat gaccattata gagtcagaaa tgggagtcgt tgcaggaatt tcctttggag    1015

ttgcttgctt ccaactgatt ggaatctttc tcgcctactg cctctctcgt gccataacaa    1075

ataaccagta tgagatagtg                                                1095

<210>  8
<211>  245
<212>  PRT
<213>  Homo sapiens

<400>  8

Met Ala Ser Pro Ser Arg Arg Leu Gln Thr Lys Pro Val Ile Thr Cys
1               5                   10                  15

Phe Lys Ser Val Leu Leu Ile Tyr Thr Phe Ile Phe Trp Ile Thr Gly
            20                  25                  30

Val Ile Leu Leu Ala Val Gly Ile Trp Gly Lys Val Ser Leu Glu Asn
        35                  40                  45

Tyr Phe Ser Leu Leu Asn Glu Lys Ala Thr Asn Val Pro Phe Val Leu
    50                  55                  60

Ile Ala Thr Gly Thr Val Ile Ile Leu Leu Gly Thr Phe Gly Cys Phe

65                  70                  75                  80

Ala Thr Cys Arg Ala Ser Ala Trp Met Leu Lys Leu Tyr Ala Met Phe
                85                  90                  95

Leu Thr Leu Val Phe Leu Val Glu Leu Val Ala Ala Ile Val Gly Phe
            100                 105                 110

Val Phe Arg His Glu Ile Lys Asn Met Ala Ser Pro Ser Arg Arg Leu
        115                 120                 125

Gln Thr Lys Pro Val Ile Thr Cys Phe Lys Ser Val Leu Leu Ile Tyr
    130                 135                 140

Thr Phe Ile Phe Trp Ile Thr Gly Val Ile Leu Leu Ala Val Gly Ile
145                 150                 155                 160

Trp Gly Lys Val Ser Leu Glu Asn Tyr Phe Ser Leu Leu Asn Glu Lys
                165                 170                 175

Ala Thr Asn Val Pro Phe Val Leu Ile Ala Thr Gly Thr Val Ile Ile
            180                 185                 190

Leu Leu Gly Thr Phe Gly Cys Phe Ala Thr Cys Arg Ala Ser Ala Trp
        195                 200                 205

Met Leu Lys Leu Tyr Ala Met Phe Leu Thr Leu Val Phe Leu Val Glu
    210                 215                 220

Leu Val Ala Ala Ile Val Gly Phe Val Phe Arg His Glu Ile Lys Asn

225                 230                 235                 240

Ser Phe Lys Asn Asn
                245

<210>  9
<211>  669
<212>  DNA
<213>  Homo sapiens

<220>
<221>  CDS
<222>  (1)..(669)

<400>  9
atg agg ctc caa aga ccc cga cag gcc ccg gcg ggt ggg agg cgc gcg        48
Met Arg Leu Gln Arg Pro Arg Gln Ala Pro Ala Gly Gly Arg Arg Ala
1               5                   10                  15

ccc cgg ggc ggg cgg ggc tcc ccc tac cgg cca gac ccg ggg aga ggc        96
Pro Arg Gly Gly Arg Gly Ser Pro Tyr Arg Pro Asp Pro Gly Arg Gly
            20                  25                  30

gcg cgg agg ctg cga agg ttc cag aag ggc ggg gag ggg gcg ccg cgc       144
Ala Arg Arg Leu Arg Arg Phe Gln Lys Gly Gly Glu Gly Ala Pro Arg
        35                  40                  45

gct gac cct ccc tgg gca ccg ctg ggg acg atg gcg ctg ctc gcc ttg       192
Ala Asp Pro Pro Trp Ala Pro Leu Gly Thr Met Ala Leu Leu Ala Leu
    50                  55                  60

ctg ctg gtc gtg gcc cta ccg cgg gtg tgg aca gac gcc aac ctg act       240
Leu Leu Val Val Ala Leu Pro Arg Val Trp Thr Asp Ala Asn Leu Thr
65                  70                  75                  80

gcg aga caa cga gat cca gag gac tcc cag cga acg gac gag ggt gac       288
Ala Arg Gln Arg Asp Pro Glu Asp Ser Gln Arg Thr Asp Glu Gly Asp

                85                  90                  95

aat aga gtg tgg tgt cat gtt tgt gag aga gaa aac act ttc gag tgc 336
Asn Arg Val Trp Cys His Val Cys Glu Arg Glu Asn Thr Phe Glu Cys
            100                 105                 110

cag aac cca agg agg tgc aaa tgg aca gag cca tac tgc gtt ata gcg 384
Gln Asn Pro Arg Arg Cys Lys Trp Thr Glu Pro Tyr Cys Val Ile Ala
        115                 120                 125

gcc gtg aaa ata ttt cca cgt ttt ttc atg gtt gcg aag cag tgc tcc 432
Ala Val Lys Ile Phe Pro Arg Phe Phe Met Val Ala Lys Gln Cys Ser
    130                 135                 140

gct ggt tgt gca gcg atg gag aga ccc aag cca gag gag aag cgg ttt 480
Ala Gly Cys Ala Ala Met Glu Arg Pro Lys Pro Glu Glu Lys Arg Phe
145                 150                 155                 160

ctc ctg gaa gag ccc atg ccc ttc ttt tac ctc aag tgt tgt aaa att 528
Leu Leu Glu Glu Pro Met Pro Phe Phe Tyr Leu Lys Cys Cys Lys Ile
                165                 170                 175

cgc tac tgc aat tta gag ggg cca cct atc aac tca tca gtg ttc aaa 576
Arg Tyr Cys Asn Leu Glu Gly Pro Pro Ile Asn Ser Ser Val Phe Lys
            180                 185                 190

gaa tat gct ggg agc atg ggt gag agc tgt ggt ggg ctg tgg ctg gcc 624
Glu Tyr Ala Gly Ser Met Gly Glu Ser Cys Gly Gly Leu Trp Leu Ala
        195                 200                 205

atc ctc ctg ctg ctg gcc tcc att gca gcc ggc ctc agc ctg tct 669
Ile Leu Leu Leu Leu Ala Ser Ile Ala Ala Gly Leu Ser Leu Ser
    210                 215                 220

<210> 10
<211> 223
<212> PRT
<213> Homo sapiens

<400> 10

Met Arg Leu Gln Arg Pro Arg Gln Ala Pro Ala Gly Gly Arg Arg Ala
1               5                   10                  15

Pro Arg Gly Gly Arg Gly Ser Pro Tyr Arg Pro Asp Pro Gly Arg Gly
            20                  25                  30

Ala Arg Arg Leu Arg Arg Phe Gln Lys Gly Gly Glu Gly Ala Pro Arg
        35                  40                  45

Ala Asp Pro Pro Trp Ala Pro Leu Gly Thr Met Ala Leu Leu Ala Leu
    50                  55                  60

Leu Leu Val Val Ala Leu Pro Arg Val Trp Thr Asp Ala Asn Leu Thr
65                  70                  75                  80

Ala Arg Gln Arg Asp Pro Glu Asp Ser Gln Arg Thr Asp Glu Gly Asp
                85                  90                  95

Asn Arg Val Trp Cys His Val Cys Glu Arg Glu Asn Thr Phe Glu Cys
            100                 105                 110

Gln Asn Pro Arg Arg Cys Lys Trp Thr Glu Pro Tyr Cys Val Ile Ala
        115                 120                 125

Ala Val Lys Ile Phe Pro Arg Phe Phe Met Val Ala Lys Gln Cys Ser
    130                 135                 140

Ala Gly Cys Ala Ala Met Glu Arg Pro Lys Pro Glu Glu Lys Arg Phe
145                 150                 155                 160

Leu Leu Glu Glu Pro Met Pro Phe Phe Tyr Leu Lys Cys Cys Lys Ile
                165                 170                 175

Arg Tyr Cys Asn Leu Glu Gly Pro Pro Ile Asn Ser Ser Val Phe Lys
            180                 185                 190

Glu Tyr Ala Gly Ser Met Gly Glu Ser Cys Gly Gly Leu Trp Leu Ala
        195                 200                 205

Ile Leu Leu Leu Leu Ala Ser Ile Ala Ala Gly Leu Ser Leu Ser
    210                 215                 220

<210>  11
<211>  21
<212>  RNA
<213>  Artificial Sequence

<220>
<223>  sense oligonucleotide for siRNA1a

<400>  11
uaaagccacu gaauuaugau u                                                21

<210>  12
<211>  21
<212>  RNA
<213>  Artificial Sequence

<220>
<223>  antisense oligonucleotide for siRNA1a

<400>  12
ucauaauuca guggcuuuau u                                                21

<210>  13
<211>  21
<212>  RNA
<213>  Artificial Sequence

<220>
<223>  sense oligonucleotide for siRNA1b

<400>  13
gaaaguagua gaccugguau u                                                21


<210>  14
<211>  21
<212>  RNA
<213>  Artificial Sequence

<220>
<223>  antisense oligonucleotide for siRNA1b

<400>  14
uaccaggucu acuacuuucu u                                                21


<210>  15
<211>  21
<212>  RNA
<213>  Artificial Sequence

<220>
<223>  sense oligonucleotide for siRNA1c

<400>  15
gaacuauacu gucuucuacu u                                                21


<210>  16
<211>  21

<212>  RNA
<213>  Artificial Sequence

<220>
<223>  antisense oligonucleotide for siRNA1c

<400>  16
guagaagaca guauaguucu u                                                21


<210>  17
<211>  21
<212>  RNA
<213>  Artificial Sequence

<220>
<223>  sense oligonucleotide for siRNA1d

<400>  17
agacauggau ggccaguuuu u                                                21


<210>  18
<211>  21
<212>  RNA
<213>  Artificial Sequence

<220>
<223>  antisense oligonucleotide for siRNA1d

<400>  18
aaacuggcca uccaugucuu u                                                21


<210>  19
<211>  21
<212>  RNA
<213>  Artificial Sequence

<220>

<223>  sense oligonucleotide for non-silensing dsRNA1

<400>  19
auucuaucac uagcgugacu u                                                21


<210>  20
<211>  21
<212>  RNA
<213>  Artificial Sequence

<220>
<223>  antisense oligonucleotide for non-silensing dsRNA1

<400>  20
gucacgcuag ugauagaauu u                                                21


<210>  21
<211>  21
<212>  RNA
<213>  Artificial Sequence

<220>
<223>  sense oligonucleotide for siRNA2a

<400>  21
gagguugguu ggaacaauau u                                                21


<210>  22
<211>  21
<212>  RNA
<213>  Artificial Sequence

<220>
<223>  antisense oligonucleotide for siRNA2a

<400>  22
uauuguucca accaaccucu u                                                21

<210>  23
<211>  21
<212>  RNA
<213>  Artificial Sequence

<220>
<223>  sense oligonucleotide for siRNA2b

<400>  23
gccaugugcu ccaaucauau u                                                21


<210>  24
<211>  21
<212>  RNA
<213>  Artificial Sequence

<220>
<223>  antisense oligonucleotide for siRNA2b

<400>  24
uaugauugga gcacauggcu u                                                21


<210>  25
<211>  21
<212>  RNA
<213>  Artificial Sequence

<220>
<223>  sense oligonucleotide for siRNA2c

<400>  25
gcagugggca ucuucuuguu u                                                21


<210>  26
<211>  21

<212>  RNA
<213>  Artificial Sequence

<220>
<223>  antisense oligonucleotide for siRNA2c

<400>  26
acaagaagau gcccacugcu u                                                21


<210>  27
<211>  21
<212>  RNA
<213>  Artificial Sequence

<220>
<223>  sense oligonucleotide for siRNA2d

<400>  27
gauacaggaa ccagaaagau u                                                21


<210>  28
<211>  21
<212>  RNA
<213>  Artificial Sequence

<220>
<223>  antisense oligonucleotide for siRNA2d

<400>  28
ucuuucuggu uccuguaucu u                                                21


<210>  29
<211>  22
<212>  DNA
<213>  Artificial Sequence

<220>

<223>  primer X

<400>  29
gcaaccacag gtaccaagat gg                                                22

<210>  30
<211>  24
<212>  DNA
<213>  Artificial Sequence

<220>
<223>  primer Y

<400>  30
ggtacttttc tagatagtgt ttcc                                              24

<210>  31
<211>  27
<212>  DNA
<213>  Artificial Sequence

<220>
<223>  primer Z

<400>  31
ccttgttttc tctagaaagg aatgcac                                           27